(19) Europäisches Patentamt / European Patent Office / Office européen des brevets

(11) **EP 3 995 586 B1**

(12) **EUROPEAN PATENT SPECIFICATION**

(45) Date of publication and mention of the grant of the patent:
**05.11.2025 Bulletin 2025/45**

(21) Application number: **20834669.2**

(22) Date of filing: **30.06.2020**

(51) International Patent Classification (IPC):
**C12Q 1/37** (2006.01)     **C12M 1/34** (2006.01)
**C12Q 1/26** (2006.01)     **G01N 33/68** (2006.01)

(52) Cooperative Patent Classification (CPC):
**C12Q 1/37; G01N 33/68;** G01N 2440/38

(86) International application number:
**PCT/JP2020/025738**

(87) International publication number:
**WO 2021/002371 (07.01.2021 Gazette 2021/01)**

(54) **GLYCOSYLATED PROTEIN ASSAY REAGENT CONTAINING PROTEASE STABILIZER INCREASING REDOX POTENTIAL OF FERROCYANIDE, METHOD FOR ASSAYING GLYCOSYLATED PROTEIN, METHOD FOR PRESERVING GLYCOSYLATED PROTEIN ASSAY REAGENT, AND METHOD FOR STABILIZING GLYCOSYLATED PROTEIN ASSAY REAGENT**

GLYKOSYLIERTES PROTEINTESTREAGENS MIT PROTEASESTABILISATOR ZUR ERHÖHUNG DES REDOXPOTENZIALS VON FERROCYANID, VERFAHREN ZUR ANALYSE VON GLYCOSYLIERTEM PROTEIN, VERFAHREN ZUR KONSERVIERUNG EINES GLYCOSYLIERTEN PROTEINTESTREAGENS UND VERFAHREN ZUR STABILISIERUNG EINES GLYCOSYLIERTEM PROTEINTESTREAGENS

RÉACTIF DE DOSAGE DE PROTÉINE GLYCOSYLÉE CONTENANT UN STABILISANT DE PROTÉASE AUGMENTANT LE POTENTIEL REDOX DE FERROCYANURE, PROCÉDÉ DE DOSAGE DE PROTÉINE GLYCOSYLÉE, PROCÉDÉ DE CONSERVATION DE RÉACTIF DE DOSAGE DE PROTÉINE GLYCOSYLÉE, ET PROCÉDÉ DE STABILISATION DE RÉACTIF DE DOSAGE DE PROTÉINE GLYCOSYLÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.07.2019 JP 2019122997**
        **01.07.2019 JP 2019122875**

(43) Date of publication of application:
**11.05.2022 Bulletin 2022/19**

(73) Proprietor: **Nagase Diagnostics Co., Ltd.**
**Tokyo 103-0024 (JP)**

(72) Inventors:
• **HONJO, Akiko**
  **Tokyo 100-0006 (JP)**
• **UEDA, Yuki**
  **Tokyo 100-0006 (JP)**
• **KONNO, Shota**
  **Tokyo 100-0006 (JP)**

(74) Representative: **Forstmeyer, Dietmar et al**
**Boeters & Lieck**
**Oberanger 32**
**80331 München (DE)**

(56) References cited:
EP-A1- 1 362 925          WO-A1-2006/013921
WO-A1-2006/013921      WO-A1-96/34977
CN-A- 104 152 431        CN-A- 109 239 059
CN-A- 109 239 059        JP-A- 2006 010 711
JP-A- H11 504 808

**Description**

Technical Field

The present invention relates to an in-vitro diagnostic reagent and relates to a glycated protein assay reagent, a method for assaying a glycated protein, a method for preserving a glycated protein assay reagent, and a method for stabilizing a glycated protein assay reagent.

Background Art

The assay of glycated proteins is important for diagnosing and managing diabetes mellitus. For example, glycated hemoglobin (HbA1c, etc.) which reflects a mean blood glucose level of the past 1 to 2 months or so, glycated albumin (GA) which reflects a mean blood glucose level of the past 2 weeks or so, and fructosamine (FRA) which is a generic name for glycated proteins in serum are assayed on a daily basis.

Enzymatic methods are exemplified as methods for assaying glycated proteins highly accurately, inexpensively, and conveniently include . For example, Patent Literatures 1, 2 and 3 state that glycated proteins in serum can be assayed by allowing protease to act on the glycated proteins, and generating hydrogen peroxide using ketoamine oxidase which acts on the resulting glycated amino acids or glycated peptides, followed by colorimetric determination using peroxidase, a Trinder reagent, and 4-aminoantipyrine. Patent Literature 4 states that glycated proteins in serum can be assayed by allowing protease to act on the glycated proteins, and measuring, by chromogenic reaction or an oxygen electrode, the amount of consumed oxygen using ketoamine oxidase which acts on the resulting glycated amino acids or glycated peptides. Patent Literatures 5 and 6 state that glycated hemoglobin or glycated albumin can be assayed by allowing protease to act on the glycated hemoglobin or the glycated albumin, and generating hydrogen peroxide using ketoamine oxidase which acts on the resulting glycated amino acids or glycated peptides, followed by colorimetric determination using peroxidase, a Trinder reagent, and 4-aminoantipyrine.

These glycated protein assay reagents contain a protease stabilizer in order to improve the stability of the reagent. For example, Patent Literature 6 discloses dimethyl sulfoxide, an alcohol, water-soluble calcium salt, common salt, quaternary ammonium salt, or a quaternary ammonium salt-type cationic surfactant as a protease stabilizer. Patent Literature 7, though having no mention about assay reagents, states that the storage stability of protease in liquid detergents can be improved by allowing the liquid detergents to contain boric acid, boronic acid, or phenylboronic acid as a protease inhibitor. Patent Literature 8 states that polyol is effective for stabilizing protease as an enzyme in water-in-oil emulsions. Patent Literatures 9 and 10 state that peptide aldehyde, calcium ions, boron compositions, polyol, and benzamidine hydrochloride in liquid detergent compositions are used as stabilizers of protease.

In reagents for colorimetric determination of hydrogen peroxide using dyes such as a Trinder reagent and 4-aminoantipyrine, the Trinder reagent and the 4-aminoantipyrine may be nonspecifically condensed and develop color due to components other than assay components in the reagents or in specimens. This is called as reagent blank. If this reagent blank is strong, the ratio of the reagent blank (noise) to an assay component (signal) in a specimen is large and causes assay errors. Therefore, a lower reagent blank is more preferred. For example, Patent Literature 11 describes a method for suppressing the reagent blank, containing allowing catalase to coexist with a protease-containing reagent in a glycated protein assay reagent so that peroxide in the reagent is eliminated. Patent Literature 12 states that a reagent blank can be suppressed by blending an amphoteric surfactant into a reagent containing ferrocyanide and a hydrogen doner such as a Trinder reagent. Patent Literature 13 discloses a method for the determination of a glycated protein in a sample. Patent Literature 14 discloses a kit for determining glycosylated serum protein.

Citation List

Patent Literature

Patent Literature 1: Japanese Patent Laid-Open No. 6-46846
Patent Literature 2: Japanese Patent Laid-Open No. 5-192193
Patent Literature 3: International Publication No. WO 96/34977
Patent Literature 4: Japanese Patent Laid-Open No. 2-195900
Patent Literature 5: International Publication No. WO 1997/13872
Patent Literature 6: International Publication No. WO 2002/061119
Patent Literature 7: International Publication No. WO 1996/041859
Patent Literature 8: International Publication No. WO 1995/028092

Patent Literature 9: International Publication No. WO 98/13462
Patent Literature 10: International Publication No. WO 98/13459
Patent Literature 11: Japanese Patent Laid-Open No. 2004-49063
Patent Literature 12: Japanese Patent Laid-Open No. 2006-10711
Patent Literature 13: International Publication No. WO 96/34977
Patent Literature 14: Chinese Publication No. CN 109 239 059 A

Summary of Invention

Technical Problem

As described above, various methods for suppressing the reagent blank have been proposed. Nonetheless, no methods for sufficiently suppressing the reagent blank in a glycated protein assay reagent containing a Trinder reagent, 4-aminoantipyrine, protease, a stabilizer of the protease, and ferrocyanide have yet been disclosed. Accordingly, an object of the present invention is to provide a glycated protein assay reagent which suppresses the reagent blank, a method for assaying a glycated protein, a method for preserving a glycated protein assay reagent, and a method for stabilizing a glycated protein assay reagent.

Solution to Problem

According to the findings of the present inventors, if ferrocyanide is contained in a glycated protein assay reagent for the purpose of preventing assay errors ascribable to reducing substances such as bilirubin, the ferrocyanide in the assay reagent may be oxidized into ferricyanide, during preservation, which causes reagent blanks. Regarding this problem, the present inventor has conducted diligent studies and consequently found that in a reagent containing a Trinder reagent, 4-aminoantipyrine, protease, a stabilizer of the protease, and ferrocyanide, use of a stabilizer of the protease that increases the oxidation-reduction potential of the ferrocyanide above 0.058 V when the stabilizer of the protease and the ferrocyanide are mixed can markedly suppress the reagent blank, reaching the completion of the present invention.

[1] A glycated protein assay reagent containing at least a Trinder reagent, 4-aminoantipyrine, protease, a stabilizer of the protease, and ferrocyanide, wherein

at least the Trinder reagent is contained in a Trinder reagent-containing partial composition,
at least the 4-aminoantipyrine is contained in a 4-aminoantipyrine-containing partial composition, and
the stabilizer of the protease is selected from the group consisting of propylene glycol, trimethylene glycol, ethylene glycol and carboxyphenylboronic acid.

[2] The glycated protein assay reagent according to [1], wherein at least one of the Trinder reagent-containing partial composition and the 4-aminoantipyrine-containing partial composition further contains the protease and the stabilizer of the protease.

[3] The glycated protein assay reagent according to [2], wherein the Trinder reagent-containing partial composition further contains the protease and the stabilizer of the protease.

[4] The glycated protein assay reagent according to any one of [1] to [3], wherein the 4-aminoantipyrine-containing partial composition further contains fructosyl amino acid oxidase.

[5] The glycated protein assay reagent according to any one of [1] to [4], wherein at least one of the Trinder reagent-containing partial composition and the 4-aminoantipyrine-containing partial composition contains the ferrocyanide.

[6] The glycated protein assay reagent according to [2], wherein the 4-aminoantipyrine-containing partial composition further contains the protease and the stabilizer of the protease.

[7] The glycated protein assay reagent according to any one of [1] to [3] and [6], wherein the Trinder reagent-containing partial composition further contains fructosyl amino acid oxidase.

[8] The glycated protein assay reagent according to [6] or [7], wherein at least one of the Trinder reagent-containing partial composition and the 4-aminoantipyrine-containing partial composition contains the ferrocyanide.

[9] The glycated protein assay reagent according to [1], wherein one of the Trinder reagent-containing partial composition and the 4-aminoantipyrine-containing partial composition further contains the protease, the stabilizer of the protease and the ferrocyanide.

[10] The glycated protein assay reagent according to [1], wherein the 4-aminoantipyrine-containing partial composition further contains the protease, the stabilizer of the protease, and the ferrocyanide.

[11] The glycated protein assay reagent according to any one of [1] to [10], wherein the concentration of the stabilizer of the protease is a concentration that increases the oxidation-reduction potential of the ferrocyanide above 0.058 V when the stabilizer of the protease and the ferrocyanide are mixed.

[12] The glycated protein assay reagent according to any one of [1] to [5] and [9], wherein the Trinder reagent-containing partial composition contains the stabilizer of the protease and the ferrocyanide, and the concentration of the stabilizer of the protease in the Trinder reagent-containing partial composition is a concentration that increases the oxidation-reduction potential of the ferrocyanide above 0.058 V.

[13] The glycated protein assay reagent according to any one of [1], [2], and [6] to [10], wherein the 4-aminoantipyrine-containing partial composition contains the stabilizer of the protease and the ferrocyanide, and the concentration of the stabilizer of the protease in the 4-aminoantipyrine-containing partial composition is a concentration that increases the oxidation-reduction potential of the ferrocyanide above 0.058 V.

[14] The glycated protein assay reagent according to any one of [1] to [13], wherein the 4-aminoantipyrine-containing partial composition further contains a chelating agent.

[15] The glycated protein assay reagent according to [14], wherein the chelating agent is selected from the group consisting of ethylenediaminetetraacetic acid, glycol ether diaminetetraacetic acid, N-(2-hydroxyethyl)iminodiacetic acid, nitrilotris(methylphosphonic acid), nitrilotriacetic acid, trans-1,2-diaminocyclohexane-N,N,N,N-tetraacetic acid, iminodiacetic acid, diethylenetriamine-N,N,N',N",N"-pentaacetic acid, 1,3-diamino-2-propanol-N,N,N',N'-tetraacetic acid, gluconic acid, malic acid, succinic acid, citric acid, salicylic acid, tartaric acid, N-[tris(hydroxymethyl)methyl] glycine, N,N-bis(2-hydroxyethyl)glycine, and salts thereof.

[16] The glycated protein assay reagent according to [14], wherein the chelating agent is citric acid or a salt thereof.

[17] The glycated protein assay reagent according to any one of [14] to [16], wherein the concentration of the chelating agent is 5 μmol/L or higher and 1000 mmol/L or lower.

[18] The glycated protein assay reagent according to [17], wherein the concentration of the chelating agent is 10 μmol/L or higher.

[19] The glycated protein assay reagent according to [17], wherein the concentration of the chelating agent is 50 μmol/L or higher.

[20] The glycated protein assay reagent according to [17], wherein the concentration of the chelating agent is 100 mmol/L or lower.

[21] The glycated protein assay reagent according to [17], wherein the concentration of the chelating agent is 25 mmol/L or lower.

[22] The glycated protein assay reagent according to any one of [1] to [21], wherein the reaction system containing the stabilizer of the protease and the ferrocyanide and not containing glycated protein further contains N-tris(hydroxymethyl)methyl-2-aminoethanesulfonic acid and sodium chloride.

[23] The glycated protein assay reagent according to any one of [1] to [22], wherein the oxidation-reduction potential is an oxidation-reduction potential measured using a sliver/silver chloride electrode as a reference electrode.

[24] The glycated protein assay reagent according to any one of [1] to [23], wherein the oxidation-reduction potential is higher than 0.058 V and less than or equal to 0.400 V.

[25] The glycated protein assay reagent according to [24], wherein the oxidation-reduction potential is 0.070 V or higher.

[26] The glycated protein assay reagent according to [24], wherein the oxidation-reduction potential is 0.084 V or higher.

[27] The glycated protein assay reagent according to [24], wherein the oxidation-reduction potential is 0.112 V or higher.

[28] The glycated protein assay reagent according to [24], wherein the oxidation-reduction potential is 0.300 V or lower.

[29] The glycated protein assay reagent according to [24], wherein the oxidation-reduction potential is 0.250 V or lower.

[30] The glycated protein assay reagent according to [24], wherein the oxidation-reduction potential is 0.235 V or lower.

[31] The glycated protein assay reagent according to any one of [1] to [30], wherein the stabilizer of the protease is propylene glycol.

[32] The glycated protein assay reagent according to [30], wherein the carboxyphenylboronic acid is 2-carboxyphenylboronic acid.

[33] The glycated protein assay reagent according to any one of [1] to [31], wherein the 4-aminoantipyrine-containing partial composition or the Trinder reagent-containing partial composition contains the stabilizer of the protease, the stabilizer of the protease is propylene glycol, and the concentration of the propylene glycol in the 4-aminoantipyrine-containing partial composition or the Trinder reagent-containing partial composition is 7.5 wt/vol% or higher and 80 wt/vol% or lower.

[34] The glycated protein assay reagent according to [33], wherein the concentration of the propylene glycol in the 4-aminoantipyrine-containing partial composition or the Trinder reagent-containing partial composition is 20 wt/vol% or higher.

[35] The glycated protein assay reagent according to [33], wherein the concentration of the propylene glycol in the 4-aminoantipyrine-containing partial composition or the Trinder reagent-containing partial composition is 25 wt/vol% or higher.

[36] The glycated protein assay reagent according to [33], wherein the concentration of the propylene glycol in the 4-aminoantipyrine-containing partial composition or the Trinder reagent-containing partial composition is 62.5 wt/vol% or lower.

[37] The glycated protein assay reagent according to [33], wherein the concentration of the propylene glycol in the 4-aminoantipyrine-containing partial composition or the Trinder reagent-containing partial composition is 60 wt/vol% or lower.

[38] The glycated protein assay reagent according to any one of [1] to [30], wherein the 4-aminoantipyrine-containing partial composition or the Trinder reagent-containing partial composition contains the stabilizer of the protease, the stabilizer of the protease is trimethylene glycol, and the concentration of the trimethylene glycol in the 4-aminoantipyrine-containing partial composition or the Trinder reagent-containing partial composition is 40 wt/vol% or higher and 80 wt/vol% or lower.

[39] The glycated protein assay reagent according to [38], wherein the concentration of the trimethylene glycol in the 4-aminoantipyrine-containing partial composition or the Trinder reagent-containing partial composition is 42.5 wt/vol% or higher.

[40] The glycated protein assay reagent according to [38], wherein the concentration of the trimethylene glycol in the 4-aminoantipyrine-containing partial composition or the Trinder reagent-containing partial composition is 45 wt/vol% or higher.

[41] The glycated protein assay reagent according to [38], wherein the concentration of the trimethylene glycol in the 4-aminoantipyrine-containing partial composition or the Trinder reagent-containing partial composition is 75 wt/vol% or lower.

[42] The glycated protein assay reagent according to [38], wherein the concentration of the trimethylene glycol in the 4-aminoantipyrine-containing partial composition or the Trinder reagent-containing partial composition is 70 wt/vol% or lower.

[43] The glycated protein assay reagent according to any one of [1] to [30], wherein the 4-aminoantipyrine-containing partial composition or the Trinder reagent-containing partial composition contains the stabilizer of the protease, the stabilizer of the protease is ethylene glycol, and the concentration of the ethylene glycol in the 4-aminoantipyrine-containing partial composition or the Trinder reagent-containing partial composition is 40 wt/vol% or higher and 80 wt/vol% or lower.

[44] The glycated protein assay reagent according to [43], wherein the concentration of the ethylene glycol in the 4-aminoantipyrine-containing partial composition or the Trinder reagent-containing partial composition is 42.5 wt/vol% or higher.

[45] The glycated protein assay reagent according to [43], wherein the concentration of the ethylene glycol in the 4-aminoantipyrine-containing partial composition or the Trinder reagent-containing partial composition is 45 wt/vol% or higher.

[46] The glycated protein assay reagent according to [43], wherein the concentration of the ethylene glycol in the 4-aminoantipyrine-containing partial composition or the Trinder reagent-containing partial composition is 75 wt/vol% or lower.

[47] The glycated protein assay reagent according to [43], wherein the concentration of the ethylene glycol in the 4-aminoantipyrine-containing partial composition or the Trinder reagent-containing partial composition is 70 wt/vol% or lower.

[48] The glycated protein assay reagent according to any one of [1] to [30] and [32], wherein the 4-aminoantipyrine-containing partial composition or the Trinder reagent-containing partial composition contains the stabilizer of the protease, the stabilizer of the protease is 2-carboxyphenylboronic acid, and the concentration of the 2-carboxyphenylboronic acid in the 4-aminoantipyrine-containing partial composition or the Trinder reagent-containing partial composition is 0.2 wt/vol% or higher and 10 wt/vol% or lower.

[49] The glycated protein assay reagent according to [48], wherein the concentration of the 2-carboxyphenylboronic acid in the 4-aminoantipyrine-containing partial composition or the Trinder reagent-containing partial composition is 0.45 wt/vol% or higher.

[50] The glycated protein assay reagent according to [48], wherein the concentration of the 2-carboxyphenylboronic acid in the 4-aminoantipyrine-containing partial composition or the Trinder reagent-containing partial composition is 0.5 wt/vol% or higher.

[51] The glycated protein assay reagent according to [48], wherein the concentration of the 2-carboxyphenylboronic acid in the 4-aminoantipyrine-containing partial composition or the Trinder reagent-containing partial composition is 7.5 wt/vol% or lower.

[52] The glycated protein assay reagent according to [48], wherein the concentration of the 2-carboxyphenylboronic acid in the 4-aminoantipyrine-containing partial composition or the Trinder reagent-containing partial composition is 5 wt/vol% or lower.

[53] The glycated protein assay reagent according to any one of [1] to [52], wherein the Trinder reagent-containing partial composition and the 4-aminoantipyrine-containing partial composition are liquid.

[54] The glycated protein assay reagent according to any one of [1] to [53], wherein the ferrocyanide is potassium ferrocyanide.

[55] The glycated protein assay reagent according to any one of [1] to [54], wherein the Trinder reagent is N,N-bis(4-sulfobutyl)-3-methylaniline.

[56] The glycated protein assay reagent according to any one of [1] to [55], wherein the glycated protein is glycated albumin.

[57] The glycated protein assay reagent according to any one of [1] to [56], wherein the glycated protein assay reagent is preserved at 2°C or higher and 10°C or lower.

[58] The glycated protein assay reagent according to any one of [1] to [57], wherein the stabilizer of the protease excludes dimethyl sulfoxide.

[59] The glycated protein assay reagent according to any one of [1] to [58], wherein one of the Trinder reagent-containing partial composition and the 4-aminoantipyrine-containing partial composition further contains peroxidase.

[60] A method for assaying a glycated protein, containing:

contacting a Trinder reagent, 4-aminoantipyrine, protease, a stabilizer of the protease, and ferrocyanide with a specimen; and

detecting color development depending on the glycated protein present in the specimen, wherein

at least before the contact, the Trinder reagent is contained in a Trinder reagent-containing partial composition,

at least before the contact, the 4-aminoantipyrine is contained in a 4-aminoantipyrine-containing partial composition,

one of the Trinder reagent-containing partial composition and the 4-aminoantipyrine-containing partial composition contains the protease and the stabilizer of the protease,

one of the Trinder reagent-containing partial composition and the 4-aminoantipyrine-containing partial composition contains the ferrocyanide, and

the stabilizer of the protease is selected from the group consisting of propylene glycol, trimethylene glycol, ethylene glycol and carboxyphenylboronic acid.

[61] The method for assaying a glycated protein according to [60], wherein the Trinder reagent-containing partial composition contains the protease and the stabilizer of the protease.

[62] The method for assaying a glycated protein according to [60] or [61], wherein the 4-aminoantipyrine-containing partial composition contains fructosyl amino acid oxidase.

[63] The method for assaying a glycated protein according to [60], wherein the 4-aminoantipyrine-containing partial composition contains the protease and the stabilizer of the protease.

[64] The method for assaying a glycated protein according to any one of [60], [61] and [63], wherein the Trinder reagent-containing partial composition contains fructosyl amino acid oxidase.

[65] The method for assaying a glycated protein according to [60], wherein one of the Trinder reagent-containing partial composition and the 4-aminoantipyrine-containing partial composition contains the protease, the stabilizer of the protease, and the ferrocyanide.

[66] The method for assaying a glycated protein according to [60], wherein the 4-aminoantipyrine-containing partial composition contains the protease, the stabilizer of the protease, and the ferrocyanide.

[67] The method for assaying a glycated protein according to any one of [60] to [66], wherein the concentration of the stabilizer of the protease is a concentration that increases the oxidation-reduction potential of the ferrocyanide above 0.058 V when the stabilizer of the protease and the ferrocyanide are mixed.

[68] The method for assaying a glycated protein according to any one of [60] to [62] and [65], wherein the Trinder reagent-containing partial composition contains the stabilizer of the protease and the ferrocyanide, and the concentration of the stabilizer of the protease in the Trinder reagent-containing partial composition is a concentration that increases the oxidation-reduction potential of the ferrocyanide above 0.058 V.

[69] The method for assaying a glycated protein according to any one of [60], [63], [65] and [66], wherein the 4-aminoantipyrine-containing partial composition contains the stabilizer of the protease and the ferrocyanide, and the concentration of the stabilizer of the protease in the 4-aminoantipyrine-containing partial composition is a concentration that increases the oxidation-reduction potential of the ferrocyanide above 0.058 V.

[70] The method for assaying a glycated protein according to any one of [60] to [69], wherein the 4-aminoantipyrine-containing partial composition further contains a chelating agent.

[71] The method for assaying a glycated protein according to [70], wherein the chelating agent is selected from the group consisting of ethylenediaminetetraacetic acid, glycol ether diaminetetraacetic acid, N-(2-hydroxyethyl)iminodiacetic acid, nitrilotris(methylphosphonic acid), nitrilotriacetic acid, trans-1,2-diaminocyclohexane-N,N,N,N-tetraacetic acid, iminodiacetic acid, diethylenetriamine-N,N,N',N'',N''-pentaacetic acid, 1,3-diamino-2-propanol-N,N,N',N'-tetraacetic acid, gluconic acid, malic acid, succinic acid, citric acid, salicylic acid, tartaric acid, N-[tris(hydroxymethyl)methyl]glycine, N,N-bis(2-hydroxyethyl) glycine, and salts thereof.

[72] The method for assaying a glycated protein according to [70], wherein the chelating agent is citric acid or a salt thereof.

[73] The method for assaying a glycated protein according to any one of [70] to [72], wherein the concentration of the chelating agent is 5 μmol/L or higher and 1000 mmol/L or lower.

[74] The method for assaying a glycated protein according to [73], wherein the concentration of the chelating agent is 10 μmol/L or higher.

[75] The method for assaying a glycated protein according to [73], wherein the concentration of the chelating agent is 50 μmol/L or higher.

[76] The method for assaying a glycated protein according to [73], wherein the concentration of the chelating agent is 100 mmol/L or lower.

[77] The method for assaying a glycated protein according to [73], wherein the concentration of the chelating agent is 25 mmol/L or lower.

[78] The method for assaying a glycated protein according to any one of [60] to [77], wherein the reaction system containing the stabilizer of the protease and the ferrocyanide and not containing glycated protein further contains N-tris(hydroxymethyl)methyl-2-aminoethanesulfonic acid and sodium chloride.

[79] The method for assaying a glycated protein according to any one of [60] to [78], wherein the oxidation-reduction potential is an oxidation-reduction potential measured using a sliver/silver chloride electrode as a reference electrode.

[80] The method for assaying a glycated protein according to any one of [60] to [79], wherein the oxidation-reduction potential is higher than 0.058 V and 0.400 V or lower.

[81] The method for assaying a glycated protein according to [80], wherein the oxidation-reduction potential is 0.070 V or higher.

[82] The method for assaying a glycated protein according to [80], wherein the oxidation-reduction potential is 0.084 V or higher.

[83] The method for assaying a glycated protein according to [80], wherein the oxidation-reduction potential is 0.112 V or higher.

[84] The method for assaying a glycated protein according to [80], wherein the oxidation-reduction potential is 0.300 V or lower.

[85] The method for assaying a glycated protein according to [80], wherein the oxidation-reduction potential is 0.250 V or lower.

[86] The method for assaying a glycated protein according to [80], wherein the oxidation-reduction potential is 0.235 V or lower.

[87] The method for assaying a glycated protein according to any one of [60] to [86], wherein the stabilizer of the protease is propylene glycol.

[88] The method for assaying a glycated protein according to [60], wherein the carboxyphenylboronic acid is 2-carboxyphenylboronic acid.

[89] The method for assaying a glycated protein according to any one of [60] to [87], wherein the 4-aminoantipyrine-containing partial composition or the Trinder reagent-containing partial composition contains the stabilizer of the protease, the stabilizer of the protease is propylene glycol, and the concentration of the propylene glycol in the 4-aminoantipyrine-containing partial composition or the Trinder reagent-containing partial composition is 7.5 wt/vol% or higher and 80 wt/vol% or lower.

[90] The method for assaying a glycated protein according to [89], wherein the concentration of the propylene glycol in the 4-aminoantipyrine-containing partial composition or the Trinder reagent-containing partial composition is 20 wt/vol% or higher.

[91] The method for assaying a glycated protein according to [89], wherein the concentration of the propylene glycol in the 4-aminoantipyrine-containing partial composition or the Trinder reagent-containing partial composition is 25 wt/vol% or higher.

[92] The method for assaying a glycated protein according to [89], wherein the concentration of the propylene glycol in the 4-aminoantipyrine-containing partial composition or the Trinder reagent-containing partial composition is 62.5 wt/vol% or lower.

[93] The method for assaying a glycated protein according to [89], wherein the concentration of the propylene glycol in the 4-aminoantipyrine-containing partial composition or the Trinder reagent-containing partial composition is 60 wt/vol% or lower.

[94] The method for assaying a glycated protein according to any one of [60] to [86], wherein the 4-aminoantipyrine-containing partial composition or the Trinder reagent-containing partial composition contains the stabilizer of the protease, the stabilizer of the protease is trimethylene glycol, and the concentration of the trimethylene glycol in the 4-aminoantipyrine-containing partial composition or the Trinder reagent-containing partial composition is 40 wt/vol% or higher and 80 wt/vol% or lower.

[95] The method for assaying a glycated protein according to [94], wherein the concentration of the trimethylene glycol in the 4-aminoantipyrine-containing partial composition or the Trinder reagent-containing partial composition is 42.5 wt/vol% or higher.

[96] The method for assaying a glycated protein according to [94], wherein the concentration of the trimethylene glycol in the 4-aminoantipyrine-containing partial composition or the Trinder reagent-containing partial composition is 45 wt/vol% or higher.

[97] The method for assaying a glycated protein according to [94], wherein the concentration of the trimethylene glycol in the 4-aminoantipyrine-containing partial composition or the Trinder reagent-containing partial composition is 75 wt/vol% or lower.

[98] The method for assaying a glycated protein according to [94], wherein the concentration of the trimethylene glycol in the 4-aminoantipyrine-containing partial composition or the Trinder reagent-containing partial composition is 70 wt/vol% or lower.

[99] The method for assaying a glycated protein according to any one of [60] to [86], wherein the 4-aminoantipyrine-containing partial composition or the Trinder reagent-containing partial composition contains the stabilizer of the protease, the stabilizer of the protease is ethylene glycol, and the concentration of the ethylene glycol in the 4-aminoantipyrine-containing partial composition or the Trinder reagent-containing partial composition is 40 wt/vol% or higher and 80 wt/vol% or lower.

[100] The method for assaying a glycated protein according to [99], wherein the concentration of the ethylene glycol in the 4-aminoantipyrine-containing partial composition or the Trinder reagent-containing partial composition is 42.5 wt/vol% or higher.

[101] The method for assaying a glycated protein according to [99], wherein the concentration of the ethylene glycol in the 4-aminoantipyrine-containing partial composition or the Trinder reagent-containing partial composition is 45 wt/vol% or higher.

[102] The method for assaying a glycated protein according to [99], wherein the concentration of the ethylene glycol in the 4-aminoantipyrine-containing partial composition or the Trinder reagent-containing partial composition is 75 wt/vol% or lower.

[103] The method for assaying a glycated protein according to [99], wherein the concentration of the ethylene glycol in the 4-aminoantipyrine-containing partial composition or the Trinder reagent-containing partial composition is 70 wt/vol% or lower.

[104] The method for assaying a glycated protein according to any one of [60] to [86] and [88], wherein the 4-aminoantipyrine-containing partial composition or the Trinder reagent-containing partial composition contains the stabilizer of the protease, the stabilizer of the protease is 2-carboxyphenylboronic acid, and the concentration of the 2-carboxyphenylboronic acid in the 4-aminoantipyrine-containing partial composition or the Trinder reagent-containing partial composition is 0.2 wt/vol% or higher and 10 wt/vol% or lower.

[105] The method for assaying a glycated protein according to [104], wherein the concentration of the 2-carboxyphenylboronic acid in the 4-aminoantipyrine-containing partial composition or the Trinder reagent-containing partial composition is 0.45 wt/vol% or higher.

[106] The method for assaying a glycated protein according to [104], wherein the concentration of the 2-carboxyphenylboronic acid in the 4-aminoantipyrine-containing partial composition or the Trinder reagent-containing partial composition is 0.5 wt/vol% or higher.

[107] The method for assaying a glycated protein according to [104], wherein the concentration of the 2-carboxyphenylboronic acid in the 4-aminoantipyrine-containing partial composition or the Trinder reagent-containing partial composition is 7.5 wt/vol% or lower.

[108] The method for assaying a glycated protein according to [104], wherein the concentration of the 2-carboxyphenylboronic acid in the 4-aminoantipyrine-containing partial composition or the Trinder reagent-containing partial composition is 5 wt/vol% or lower.

[109] The method for assaying a glycated protein according to any one of [60] to [108], wherein the Trinder reagent-containing partial composition and the 4-aminoantipyrine-containing partial composition are liquid.

[110] The method for assaying a glycated protein according to any one of [60] to [109], wherein the ferrocyanide is potassium ferrocyanide.

[111] The method for assaying a glycated protein according to any one of [60] to [110], wherein the Trinder reagent is N,N-bis(4-sulfobutyl)-3-methylaniline.

[112] The method for assaying a glycated protein according to any one of [60] to [111], wherein the glycated protein is glycated albumin.

[113] The method for assaying a glycated protein according to any one of [60] to [112], wherein the Trinder reagent-containing partial composition is preserved at 2°C or higher and 10°C or lower.

[114] The method for assaying a glycated protein according to any one of [60] to [113], wherein the 4-aminoantipyrine-containing partial composition is preserved at 2°C or higher and 10°C or lower.

[115] The method for assaying a glycated protein according to any one of [60] to [114], wherein the stabilizer of the protease excludes dimethyl sulfoxide.

[116] The method for assaying a glycated protein according to any one of [60] to [115], wherein one of the Trinder reagent-containing partial composition and the 4-aminoantipyrine-containing partial composition further contains peroxidase.

[117] A method for preserving a glycated protein assay reagent, containing:

> preparing a Trinder reagent-containing partial composition containing at least a Trinder reagent;
> preserving the Trinder reagent-containing partial composition;
> preparing a 4-aminoantipyrine-containing partial composition containing at least 4-aminoantipyrine; and
> preserving the 4-aminoantipyrine-containing partial composition, wherein
> one of the Trinder reagent-containing partial composition and the 4-aminoantipyrine-containing partial composition further contains protease and a stabilizer of the protease,
> one of the Trinder reagent-containing partial composition and the 4-aminoantipyrine-containing partial composition further contains ferrocyanide, and
> the stabilizer of the protease is selected from the group consisting of propylene glycol, trimethylene glycol, ethylene glycol and carboxyphenylboronic acid.

[118] The method for preserving a glycated protein assay reagent according to [117], wherein the Trinder reagent-containing partial composition contains the protease and the stabilizer of the protease.

[119] The method for preserving a glycated protein assay reagent according to [117] or [118], wherein the 4-aminoantipyrine-containing partial composition contains fructosyl amino acid oxidase.

[120] The method for preserving a glycated protein assay reagent according to [117], wherein the 4-aminoantipyrine-containing partial composition contains the protease and the stabilizer of the protease.

[121] The method for preserving a glycated protein assay reagent according to any one of [117], [118], and [120], wherein the Trinder reagent-containing partial composition contains fructosyl amino acid oxidase.

[122] The method for preserving a glycated protein assay reagent according to [117], wherein one of the Trinder reagent-containing partial composition and the 4-aminoantipyrine-containing partial composition contains the protease, the stabilizer of the protease, and the ferrocyanide.

[123] The method for preserving a glycated protein assay reagent according to [117], wherein the 4-aminoantipyrine-containing partial composition contains the protease, the stabilizer of the protease, and the ferrocyanide.

[124] The method for preserving a glycated protein assay reagent according to any one of [117] to [123], wherein the concentration of the stabilizer of the protease is a concentration that increases the oxidation-reduction potential of the ferrocyanide above 0.058 V when the stabilizer of the protease and the ferrocyanide are mixed.

[125] The method for preserving a glycated protein assay reagent according to any one of [117] to [119] and [122], wherein the Trinder reagent-containing partial composition contains the stabilizer of the protease and the ferrocyanide, and the concentration of the stabilizer of the protease in the Trinder reagent-containing partial composition is a concentration that increases the oxidation-reduction potential of the ferrocyanide above 0.058 V.

[126] The method for preserving a glycated protein assay reagent according to any one of [117], [120], [122] and [123], wherein the 4-aminoantipyrine-containing partial composition contains the stabilizer of the protease and the ferrocyanide, and the concentration of the stabilizer of the protease in the 4-aminoantipyrine-containing partial composition is a concentration that increases the oxidation-reduction potential of the ferrocyanide above 0.058 V.

[127] The method for preserving a glycated protein assay reagent according to any one of [117] to [126], wherein the 4-aminoantipyrine-containing partial composition further contains a chelating agent.

[128] The method for preserving a glycated protein assay reagent according to [127], wherein the chelating agent is selected from the group consisting of ethylenediaminetetraacetic acid, glycol ether diaminetetraacetic acid, N-(2-hydroxyethyl)iminodiacetic acid, nitrilotris(methylphosphonic acid), nitrilotriacetic acid, trans-1,2-diaminocyclohexane-N,N,N,N-tetraacetic acid, iminodiacetic acid, diethylenetriamine-N,N,N',N",N"-pentaacetic acid, 1,3-diamino-2-propanol-N,N,N',N'-tetraacetic acid, gluconic acid, malic acid, succinic acid, citric acid, salicylic acid, tartaric acid, N-[tris(hydroxymethyl)methyl]glycine, N,N-bis(2-hydroxyethyl)glycine, and salts thereof.

[129] The method for preserving a glycated protein assay reagent according to [127], wherein the chelating agent is citric acid or a salt thereof.

[130] The method for preserving a glycated protein assay reagent according to any one of [127] to [129], wherein the concentration of the chelating agent is 5 μmol/L or higher and 1000 mmol/L or lower.

[131] The method for preserving a glycated protein assay reagent according to [130], wherein the concentration of the chelating agent is 10 μmol/L or higher.

[132] The method for preserving a glycated protein assay reagent according to [130], wherein the concentration of the chelating agent is 50 μmol/L or higher.

[133] The method for preserving a glycated protein assay reagent according to [130], wherein the concentration of the chelating agent is 100 mmol/L or lower.

[134] The method for preserving a glycated protein assay reagent according to [130], wherein the concentration of the chelating agent is 25 mmol/L or lower.

[135] The method for preserving a glycated protein assay reagent according to any one of [117] to [134], wherein the reaction system containing the stabilizer of the protease and the ferrocyanide and not containing glycated protein further contains N-tris(hydroxymethyl)methyl-2-aminoethanesulfonic acid and sodium chloride.

[136] The method for preserving a glycated protein assay reagent according to any one of [117] to [135], wherein the oxidation-reduction potential is an oxidation-reduction potential measured using a sliver/silver chloride electrode as a reference electrode.

[137] The method for preserving a glycated protein assay reagent according to any one of [117] to [136], wherein the oxidation-reduction potential is higher than 0.058 V and 0.400 V or lower.

[138] The method for preserving a glycated protein assay reagent according to [137], wherein the oxidation-reduction potential is 0.070 V or higher.

[139] The method for preserving a glycated protein assay reagent according to [137], wherein the oxidation-reduction potential is 0.084 V or higher.

[140] The method for preserving a glycated protein assay reagent according to [137], wherein the oxidation-reduction potential is 0.112 V or higher.

[141] The method for preserving a glycated protein assay reagent according to [137], wherein the oxidation-reduction potential is 0.300 V or lower.

[142] The method for preserving a glycated protein assay reagent according to [137], wherein the oxidation-reduction potential is 0.250 V or lower.

[143] The method for preserving a glycated protein assay reagent according to [137], wherein the oxidation-reduction potential is 0.235 V or lower.

[144] The method for preserving a glycated protein assay reagent according to any one of [117] to [143], wherein the stabilizer of the protease is propylene glycol.

[145] The method for preserving a glycated protein assay reagent according to [117], wherein the carboxyphenylboronic acid is 2-carboxyphenylboronic acid.

[146] The method for preserving a glycated protein assay reagent according to any one of [117] to [144], wherein the 4-aminoantipyrine-containing partial composition or the Trinder reagent-containing partial composition contains the stabilizer of the protease, the stabilizer of the protease is propylene glycol, and the concentration of the propylene glycol in the 4-aminoantipyrine-containing partial composition or the Trinder reagent-containing partial composition is 7.5 wt/vol% or higher and 80 wt/vol% or lower.

[147] The method for preserving a glycated protein assay reagent according to [146], wherein the concentration of the propylene glycol in the 4-aminoantipyrine-containing partial composition or the Trinder reagent-containing partial composition is 20 wt/vol% or higher.

[148] The method for preserving a glycated protein assay reagent according to [146], wherein the concentration of the propylene glycol in the 4-aminoantipyrine-containing partial composition or the Trinder reagent-containing partial composition is 25 wt/vol% or higher.

[149] The method for preserving a glycated protein assay reagent according to [146], wherein the concentration of the propylene glycol in the 4-aminoantipyrine-containing partial composition or the Trinder reagent-containing partial composition is 62.5 wt/vol% or lower.

[150] The method for preserving a glycated protein assay reagent according to [146], wherein the concentration of the propylene glycol in the 4-aminoantipyrine-containing partial composition or the Trinder reagent-containing partial composition is 60 wt/vol% or lower.

[151] The method for preserving a glycated protein assay reagent according to any one of [117] to [143], wherein the 4-aminoantipyrine-containing partial composition or the Trinder reagent-containing partial composition contains the stabilizer of the protease, the stabilizer of the protease is trimethylene glycol, and the concentration of the trimethylene glycol in the 4-aminoantipyrine-containing partial composition or the Trinder reagent-containing partial composition is 40 wt/vol% or higher and 80 wt/vol% or lower.

[152] The method for preserving a glycated protein assay reagent according to [151], wherein the concentration of the trimethylene glycol in the 4-aminoantipyrine-containing partial composition or the Trinder reagent-containing partial composition is 42.5 wt/vol% or higher.

[153] The method for preserving a glycated protein assay reagent according to [151], wherein the concentration of the trimethylene glycol in the 4-aminoantipyrine-containing partial composition or the Trinder reagent-containing partial composition is 45 wt/vol% or higher.

[154] The method for preserving a glycated protein assay reagent according to [151], wherein the concentration of the trimethylene glycol in the 4-aminoantipyrine-containing partial composition or the Trinder reagent-containing partial composition is 75 wt/vol% or lower.

[155] The method for preserving a glycated protein assay reagent according to [151], wherein the concentration of the trimethylene glycol in the 4-aminoantipyrine-containing partial composition or the Trinder reagent-containing partial composition is 70 wt/vol% or lower.

[156] The method for preserving a glycated protein assay reagent according to any one of [117] to [143], wherein the 4-aminoantipyrine-containing partial composition or the Trinder reagent-containing partial composition contains the stabilizer of the protease, the stabilizer of the protease is ethylene glycol, and the concentration of the ethylene glycol in the 4-aminoantipyrine-containing partial composition or the Trinder reagent-containing partial composition is 40 wt/vol% or higher and 80 wt/vol% or lower.

[157] The method for preserving a glycated protein assay reagent according to [156], wherein the concentration of the ethylene glycol in the 4-aminoantipyrine-containing partial composition or the Trinder reagent-containing partial composition is 42.5 wt/vol% or higher.

[158] The method for preserving a glycated protein assay reagent according to [156], wherein the concentration of the ethylene glycol in the 4-aminoantipyrine-containing partial composition or the Trinder reagent-containing partial composition is 45 wt/vol% or higher.

[159] The method for preserving a glycated protein assay reagent according to [156], wherein the concentration of the ethylene glycol in the 4-aminoantipyrine-containing partial composition or the Trinder reagent-containing partial composition is 75 wt/vol% or lower.

[160] The method for preserving a glycated protein assay reagent according to [156], wherein the concentration of the ethylene glycol in the 4-aminoantipyrine-containing partial composition or the Trinder reagent-containing partial composition is 70 wt/vol% or lower.

[161] The method for preserving a glycated protein assay reagent according to any one of [117] to [143] and [145], wherein the 4-aminoantipyrine-containing partial composition or the Trinder reagent-containing partial composition contains the stabilizer of the protease, the stabilizer of the protease is 2-carboxyphenylboronic acid, and the concentration of the 2-carboxyphenylboronic acid in the 4-aminoantipyrine-containing partial composition or the Trinder reagent-containing partial composition is 0.2 wt/vol% or higher and 10 wt/vol% or lower.

[162] The method for preserving a glycated protein assay reagent according to [161], wherein the concentration of the 2-carboxyphenylboronic acid in the 4-aminoantipyrine-containing partial composition or the Trinder reagent-containing partial composition is 0.45 wt/vol% or higher.

[163] The method for preserving a glycated protein assay reagent according to [161], wherein the concentration of the 2-carboxyphenylboronic acid in the 4-aminoantipyrine-containing partial composition or the Trinder reagent-containing partial composition is 0.5 wt/vol% or higher.

[164] The method for preserving a glycated protein assay reagent according to [161], wherein the concentration of the 2-carboxyphenylboronic acid in the 4-aminoantipyrine-containing partial composition or the Trinder reagent-containing partial composition is 7.5 wt/vol% or lower.

[165] The method for preserving a glycated protein assay reagent according to [161], wherein the concentration of the 2-carboxyphenylboronic acid in the 4-aminoantipyrine-containing partial composition or the Trinder reagent-containing partial composition is 5 wt/vol% or lower.

[166] The method for preserving a glycated protein assay reagent according to any one of [117] to [165], wherein the Trinder reagent-containing partial composition and the 4-aminoantipyrine-containing partial composition are liquid.

[167] The method for preserving a glycated protein assay reagent according to any one of [117] to [166], wherein the ferrocyanide is potassium ferrocyanide.

[168] The method for preserving a glycated protein assay reagent according to any one of [117] to [167], wherein the Trinder reagent is N,N-bis(4-sulfobutyl)-3-methylaniline.

[169] The method for preserving a glycated protein assay reagent according to any one of [117] to [168], wherein the glycated protein is glycated albumin.

[170] The method for preserving a glycated protein assay reagent according to any one of [117] to [169], wherein the Trinder reagent-containing partial composition is preserved at 2°C or higher and 10°C or lower.

[171] The method for preserving a glycated protein assay reagent according to any one of [117] to [170], wherein the 4-aminoantipyrine-containing partial composition is preserved at 2°C or higher and 10°C or lower.

[172] The method for preserving a glycated protein assay reagent according to any one of [117] to [171], wherein the stabilizer of the protease excludes dimethyl sulfoxide.

[173] The method for preserving a glycated protein assay reagent according to any one of [117] to [172], wherein one of the Trinder reagent-containing partial composition and the 4-aminoantipyrine-containing partial composition further contains peroxidase.

[174] A method for stabilizing a glycated protein assay reagent, containing:

preparing a Trinder reagent-containing partial composition containing at least a Trinder reagent; and
preparing a 4-aminoantipyrine-containing partial composition containing at least 4-aminoantipyrine, wherein one of the Trinder reagent-containing partial composition and the 4-aminoantipyrine-containing partial composition further comprises protease and a stabilizer of the protease,
one of the Trinder reagent-containing partial composition and the 4-aminoantipyrine-containing partial composition further comprises ferrocyanide, and
the stabilizer of the protease is selected from the group consisting of propylene glycol, trimethylene glycol, ethylene glycol and carboxyphenylboronic acid.

[175] The method for stabilizing a glycated protein assay reagent according to [174], wherein the Trinder reagent-containing partial composition further comprises the protease and the stabilizer of the protease.

[176] The method for stabilizing a glycated protein assay reagent according to [174] or [175], wherein the 4-aminoantipyrine-containing partial composition further comprises fructosyl amino acid oxidase.

[177] The method for stabilizing a glycated protein assay reagent according to [174], wherein the 4-aminoantipyrine-containing partial composition further comprises the protease and the stabilizer of the protease.

[178] The method for stabilizing a glycated protein assay reagent according to any one of [174], [175], and [177], wherein the Trinder reagent-containing partial composition further comprises fructosyl amino acid oxidase.

[179] The method for stabilizing a glycated protein assay reagent according to [174], wherein one of the Trinder reagent-containing partial composition and the 4-aminoantipyrine-containing partial composition further comprises the protease, the stabilizer of the protease, and the ferrocyanide.

[180] The method for stabilizing a glycated protein assay reagent according to [174], wherein the 4-aminoantipyrine-containing partial composition further comprises the protease, the stabilizer of the protease, and the ferrocyanide.

[181] The method for stabilizing a glycated protein assay reagent according to any one of [174] to [180], wherein the concentration of the stabilizer of the protease is a concentration that increases the oxidation-reduction potential of the ferrocyanide above 0.058 V when the stabilizer of the protease and the ferrocyanide are mixed.

[182] The method for stabilizing a glycated protein assay reagent according to any one of [174] to [176] and [179], wherein the Trinder reagent-containing partial composition further comprises the stabilizer of the protease and the ferrocyanide, and the concentration of the stabilizer of the protease in the Trinder reagent-containing partial composition is a concentration that increases the oxidation-reduction potential of the ferrocyanide above 0.058 V.

[183] The method for stabilizing a glycated protein assay reagent according to any one of [174], [177], [179] and [180], wherein the 4-aminoantipyrine-containing partial composition further comprises the stabilizer of the protease and the ferrocyanide, and the concentration of the stabilizer of the protease in the 4-aminoantipyrine-containing partial composition is a concentration that increases the oxidation-reduction potential of the ferrocyanide above 0.058 V.

[184] The method for stabilizing a glycated protein assay reagent according to any one of [174] to [183], wherein the 4-aminoantipyrine-containing partial composition further comprises a chelating agent.

[185] The method for stabilizing a glycated protein assay reagent according to [184], wherein the chelating agent is selected from the group consisting of ethylenediaminetetraacetic acid, glycol ether diaminetetraacetic acid, N-(2-hydroxyethyl)iminodiacetic acid, nitrilotris(methylphosphonic acid), nitrilotriacetic acid, trans-1,2-diaminocyclohexane-N,N,N,N-tetraacetic acid, iminodiacetic acid, diethylenetriamine-N,N,N',N",N"-pentaacetic acid, 1,3-diamino-2-propanol-N,N,N',N'-tetraacetic acid, gluconic acid, malic acid, succinic acid, citric acid, salicylic acid, tartaric acid, N-[tris(hydroxymethyl)methyl]glycine, N,N-bis(2-hydroxyethyl)glycine, and salts thereof.

[186] The method for stabilizing a glycated protein assay reagent according to [184], wherein the chelating agent is citric acid or a salt thereof.

[187] The method for stabilizing a glycated protein assay reagent according to any one of [184] to [186], wherein the concentration of the chelating agent is 5 μmol/L or higher and 1000 mmol/L or lower.

[188] The method for stabilizing a glycated protein assay reagent according to [187], wherein the concentration of the chelating agent is 10 μmol/L or higher.

[189] The method for stabilizing a glycated protein assay reagent according to [187], wherein the concentration of the chelating agent is 50 μmol/L or higher.

[190] The method for stabilizing a glycated protein assay reagent according to [187], wherein the concentration of the chelating agent is 100 mmol/L or lower.

[191] The method for stabilizing a glycated protein assay reagent according to [187], wherein the concentration of the chelating agent is 25 mmol/L or lower.

[192] The method for stabilizing a glycated protein assay reagent according to any one of [174] to [191], wherein the reaction system containing the stabilizer of the protease and the ferrocyanide and not containing glycated protein further contains N-tris(hydroxymethyl)methyl-2-aminoethanesulfonic acid and sodium chloride.

[193] The method for stabilizing a glycated protein assay reagent according to any one of [174] to [192], wherein the oxidation-reduction potential is an oxidation-reduction potential measured using a sliver/silver chloride electrode as a reference electrode.

[194] The method for stabilizing a glycated protein assay reagent according to any one of [174] to [193], wherein the oxidation-reduction potential is higher than 0.058 V and 0.400 V or lower.

[195] The method for stabilizing a glycated protein assay reagent according to [194], wherein the oxidation-reduction potential is 0.070 V or higher.

[196] The method for stabilizing a glycated protein assay reagent according to [194], wherein the oxidation-reduction potential is 0.084 V or higher.

[197] The method for stabilizing a glycated protein assay reagent according to [194], wherein the oxidation-reduction potential is 0.112 V or higher.

[198] The method for stabilizing a glycated protein assay reagent according to [194], wherein the oxidation-reduction potential is 0.300 V or lower.

[199] The method for stabilizing a glycated protein assay reagent according to [194], wherein the oxidation-reduction potential is 0.250 V or lower.

[200] The method for stabilizing a glycated protein assay reagent according to [194], wherein the oxidation-reduction potential is 0.235 V or lower.

[201] The method for stabilizing a glycated protein assay reagent according to any one of [174] to [200], wherein the stabilizer of the protease is propylene glycol.

[202] The method for stabilizing a glycated protein assay reagent according to [174], wherein the carboxyphenylboronic acid is 2-carboxyphenylboronic acid.

[203] The method for stabilizing a glycated protein assay reagent according to any one of [174] to [201], wherein the 4-aminoantipyrine-containing partial composition or the Trinder reagent-containing partial composition contains the stabilizer of the protease, the stabilizer of the protease is propylene glycol, and the concentration of the propylene glycol in the 4-aminoantipyrine-containing partial composition or the Trinder reagent-containing partial composition is 7.5 wt/vol% or higher and 80 wt/vol% or lower.

[204] The method for stabilizing a glycated protein assay reagent according to [203], wherein the concentration of the propylene glycol in the 4-aminoantipyrine-containing partial composition or the Trinder reagent-containing partial composition is 20 wt/vol% or higher.

[205] The method for stabilizing a glycated protein assay reagent according to [203], wherein the concentration of the propylene glycol in the 4-aminoantipyrine-containing partial composition or the Trinder reagent-containing partial composition is 25 wt/vol% or higher.

[206] The method for stabilizing a glycated protein assay reagent according to [203], wherein the concentration of the propylene glycol in the 4-aminoantipyrine-containing partial composition or the Trinder reagent-containing partial composition is 62.5 wt/vol% or lower.

[207] The method for stabilizing a glycated protein assay reagent according to [203], wherein the concentration of the propylene glycol in the 4-aminoantipyrine-containing partial composition or the Trinder reagent-containing partial composition is 60 wt/vol% or lower.

[208] The method for stabilizing a glycated protein assay reagent according to any one of [174] to [200], wherein the 4-aminoantipyrine-containing partial composition or the Trinder reagent-containing partial composition contains the stabilizer of the protease, the stabilizer of the protease is trimethylene glycol, and the concentration of the trimethylene glycol in the 4-aminoantipyrine-containing partial composition or the Trinder reagent-containing partial composition is 40 wt/vol% or higher and 80 wt/vol% or lower.

[209] The method for stabilizing a glycated protein assay reagent according to [208], wherein the concentration of the trimethylene glycol in the 4-aminoantipyrine-containing partial composition or the Trinder reagent-containing partial composition is 42.5 wt/vol% or higher.

[210] The method for stabilizing a glycated protein assay reagent according to [208], wherein the concentration of the trimethylene glycol in the 4-aminoantipyrine-containing partial composition or the Trinder reagent-containing partial composition is 45 wt/vol% or higher.

[211] The method for stabilizing a glycated protein assay reagent according to [208], wherein the concentration of the trimethylene glycol in the 4-aminoantipyrine-containing partial composition or the Trinder reagent-containing partial composition is 75 wt/vol% or lower.

[212] The method for stabilizing a glycated protein assay reagent according to [208], wherein the concentration of the trimethylene glycol in the 4-aminoantipyrine-containing partial composition or the Trinder reagent-containing partial composition is 70 wt/vol% or lower.

[213] The method for stabilizing a glycated protein assay reagent according to any one of [174] to [200], wherein the 4-aminoantipyrine-containing partial composition or the Trinder reagent-containing partial composition contains the stabilizer of the protease, the stabilizer of the protease is ethylene glycol, and the concentration of the ethylene glycol in the 4-aminoantipyrine-containing partial composition or the Trinder reagent-containing partial composition is 40 wt/vol% or higher and 80 wt/vol% or lower.

[214] The method for stabilizing a glycated protein assay reagent according to [213], wherein the concentration of the ethylene glycol in the 4-aminoantipyrine-containing partial composition or the Trinder reagent-containing partial composition is 42.5 wt/vol% or higher.

[215] The method for stabilizing a glycated protein assay reagent according to [213], wherein the concentration of the ethylene glycol in the 4-aminoantipyrine-containing partial composition or the Trinder reagent-containing partial composition is 45 wt/vol% or higher.

[216] The method for stabilizing a glycated protein assay reagent according to [213], wherein the concentration of the ethylene glycol in the 4-aminoantipyrine-containing partial composition or the Trinder reagent-containing partial composition is 75 wt/vol% or lower.

[217] The method for stabilizing a glycated protein assay reagent according to [213], wherein the concentration of the ethylene glycol in the 4-aminoantipyrine-containing partial composition or the Trinder reagent-containing partial composition is 70 wt/vol% or lower.

[218] The method for stabilizing a glycated protein assay reagent according to any one of [174] to [200] and [202], wherein the 4-aminoantipyrine-containing partial composition or the Trinder reagent-containing partial composition contains the stabilizer of the protease, the stabilizer of the protease is 2-carboxyphenylboronic acid, and the concentration of the 2-carboxyphenylboronic acid in the 4-aminoantipyrine-containing partial composition or the Trinder reagent-containing partial composition is 0.2 wt/vol% or higher and 10 wt/vol% or lower.

[219] The method for stabilizing a glycated protein assay reagent according to [218], wherein the concentration of the 2-carboxyphenylboronic acid in the 4-aminoantipyrine-containing partial composition or the Trinder reagent-containing partial composition is 0.45 wt/vol% or higher.

[220] The method for stabilizing a glycated protein assay reagent according to [218], wherein the concentration of the 2-carboxyphenylboronic acid in the 4-aminoantipyrine-containing partial composition or the Trinder reagent-containing partial composition is 0.5 wt/vol% or higher.

[221] The method for stabilizing a glycated protein assay reagent according to [218], wherein the concentration of the 2-carboxyphenylboronic acid in the 4-aminoantipyrine-containing partial composition or the Trinder reagent-containing partial composition is 7.5 wt/vol% or lower.

[222] The method for stabilizing a glycated protein assay reagent according to [218], wherein the concentration of the 2-carboxyphenylboronic acid in the 4-aminoantipyrine-containing partial composition or the Trinder reagent-containing partial composition is 5 wt/vol% or lower.

[223] The method for stabilizing a glycated protein assay reagent according to any one of [174] to [222], wherein the Trinder reagent-containing partial composition and the 4-aminoantipyrine-containing partial composition are liquid.

[224] The method for stabilizing a glycated protein assay reagent according to any one of [174] to [223], wherein the ferrocyanide is potassium ferrocyanide.

[225] The method for stabilizing a glycated protein assay reagent according to any one of [174] to [224], wherein the Trinder reagent is N,N-bis(4-sulfobutyl)-3-methylaniline.

[226] The method for stabilizing a glycated protein assay reagent according to any one of [174] to [225], wherein the glycated protein is glycated albumin.

[227] The method for stabilizing a glycated protein assay reagent according to any one of [174] to [226], wherein the Trinder reagent-containing partial composition is preserved at 2°C or higher and 10°C or lower.

[228] The method for stabilizing a glycated protein assay reagent according to any one of [174] to [227], wherein the 4-aminoantipyrine-containing partial composition is preserved at 2°C or higher and 10°C or lower.

[229] The method for stabilizing a glycated protein assay reagent according to any one of [174] to [228], wherein the stabilizer of the protease excludes dimethyl sulfoxide.

[230] The method for stabilizing a glycated protein assay reagent according to any one of [174] to [229], wherein one of the Trinder reagent-containing partial composition and the 4-aminoantipyrine-containing partial composition further contains peroxidase.

[231] The method for stabilizing a glycated protein assay reagent according to any one of [174] to [230], wherein the ratio of the assay sensitivity of the Trinder reagent-containing partial composition and the 4-aminoantipyrine-containing partial composition preserved at 5°C for 1 year to the assay sensitivity of the Trinder reagent-containing partial composition and the 4-aminoantipyrine-containing partial composition preserved at 5°C for 28 days is 80% or more and 110% or less.

Advantageous Effects of Invention

The present invention can provide a glycated protein assay reagent which suppresses elevation in reagent blank, a method for assaying a glycated protein, a method for preserving a glycated protein assay reagent, and a method for stabilizing a glycated protein assay reagent.

Brief Description of Drawings

[Figure 1] Figure 1 is a graph showing the relationship between a reagent blank and the oxidation-reduction potential of ferrocyanide after preservation for 14 days of a partial composition according to Example 2.

[Figure 2] Figure 2 is a graph showing the relationship between the pH of a ferrocyanide-containing liquid partial composition according to Example 4 plus a chelating agent added to the ferrocyanide-containing liquid partial composition, and a reagent blank.

[Figure 3] Figure 3 is a graph showing the relationship between a reagent blank and the oxidation-reduction potential of ferrocyanide after preservation for 1 year of a partial composition according to Example 7.

[Figure 4] Figure 4 is a graph showing the relationship between the pH of a ferrocyanide-containing liquid partial composition according to Example 9 plus a chelating agent added to the ferrocyanide-containing liquid partial composition, and a reagent blank.

Description of Embodiments

Hereinafter, a preferred mode for carrying out the present invention (hereinafter, referred to as an "embodiment") will be described in detail. The embodiment given below illustrates an apparatus or a method for embodying the technical idea of the invention and does not limit the technical idea of the invention to combinations of constituent members, etc. described below.

The glycated protein assay reagent according to an embodiment contains at least a Trinder reagent, 4-aminoantipyrine, protease, a stabilizer of the protease, and ferrocyanide, wherein at least the Trinder reagent is contained in a Trinder reagent-containing partial composition, at least the 4-aminoantipyrine is contained in a 4-aminoantipyrine-containing partial composition, the stabilizer is a stabilizer that increases the oxidation-reduction potential of the ferrocyanide above 0.058 V when the stabilizer is mixed with the ferrocyanide, and the oxidation-reduction potential is an oxidation-reduction potential in a reaction system containing the stabilizer and the ferrocyanide and not containing glycated protein.

In the present disclosure, the reagent is a reagent that assays an analyte and is, for example, an assembly of partial compositions. The reagent is capable of detecting an analyte in itself. The partial composition is a composition constituting a portion of the reagent. The partial composition cannot assay an analyte in itself.

The ferrocyanide may be contained in at least one of the Trinder reagent-containing partial composition and the 4-aminoantipyrine-containing partial composition. Alternatively, the ferrocyanide may be contained in a ferrocyanide-containing partial composition different from the Trinder reagent-containing partial composition and the 4-aminoantipyrine-containing partial composition.

The glycated protein assay reagent according to an embodiment may be preserved, distributed and used in the form of a kit. Two-reagent kits and three-reagent kits are exemplified as the form of the kit. For example, in the case of a two-reagent kit, the ferrocyanide is contained in at least one of the Trinder reagent-containing partial composition and the 4-aminoantipyrine-containing partial composition. For example, in the case of a three-reagent kit, the ferrocyanide is contained in a ferrocyanide-containing partial composition different from the Trinder reagent-containing partial composition and the 4-aminoantipyrine-containing partial composition. The three-reagent kit in which the Trinder reagent, the 4-aminoantipyrine, and the ferrocyanide are preserved in separate forms tends to have a high specimen sensitivity preservation rate because reduction in sensitivity is suppressed as compared with the two-reagent kit. However, the glycated protein assay reagent according to an embodiment of the two-reagent system is not excluded.

The glycated protein is, for example, glycated albumin (GA) or glycated hemoglobin (HbA1c, etc.).

The Trinder reagent-containing partial composition may further comprise at least one of fructosyl amino acid oxidase, the ferrocyanide, the protease, the stabilizer of the protease, and peroxidase. Alternatively, the Trinder reagent-containing partial composition may further comprise at least one of fructosyl amino acid oxidase, the ferrocyanide, and peroxidase. In this case, at least one of the 4-aminoantipyrine-containing partial composition and the ferrocyanide-containing partial composition can further comprise the protease and the stabilizer of the protease. The 4-aminoantipyrine-containing partial composition may further comprise the ferrocyanide. At least one of the 4-aminoantipyrine-containing partial composition and the ferrocyanide-containing partial composition may further comprise fructosyl amino acid oxidase or peroxidase.

The 4-aminoantipyrine-containing partial composition may further comprise at least one of fructosyl amino acid oxidase, the ferrocyanide, the protease, the stabilizer of the protease, and peroxidase. Alternatively, the 4-aminoantipyrine-containing partial composition may further comprise at least one of fructosyl amino acid oxidase, the ferrocyanide, and peroxidase. In this case, at least one of the Trinder reagent-containing partial composition and the ferrocyanide-containing partial composition can further comprise the protease and the stabilizer of the protease. The Trinder reagent-containing partial composition may further comprise the ferrocyanide. At least one of the Trinder reagent-containing partial composition and the ferrocyanide-containing partial composition may further comprise fructosyl amino acid oxidase or peroxidase.

Upon contact of the glycated protein assay reagent according to an embodiment with a glucose-bound protein (glycated protein) contained in a specimen of an analyte (sample), the glycated protein is degraded into a glycated amino acid or a glycated peptide by the protease. The glycated amino acid or the glycated peptide is degraded into an amino acid or a peptide and glucosone by the fructosyl amino acid oxidase. In this respect, water and oxygen are reacted by the fructosyl amino acid oxidase to form hydrogen peroxide. The Trinder reagent and the 4-aminoantipyrine undergo oxidative condensation reaction in the presence of the hydrogen peroxide and the peroxidase to emit color. In the case of using, for example, N,N-bis(4-sulfobutyl)-3-methylaniline (TODB) as the Trinder reagent, chromogenic reaction to emit violet-blue color occurs. The violet-blue color thus emitted is detected by the measurement of absorbance at a wavelength of 546 nm. The concentration of the glycated protein contained in the specimen is determined on the basis of the absorbance at a wavelength of 546 nm.

In the glycated protein assay reagent according to an embodiment, when the ferrocyanide is contained in at least one of the Trinder reagent-containing partial composition and the 4-aminoantipyrine-containing partial composition, a usual method for using the glycated protein assay reagent according to an embodiment involves first adding the Trinder reagent-containing partial composition to a sample, leaving the mixture of the sample and the Trinder reagent-containing partial composition for several minutes, for example, approximately 5 to 10 minutes, then adding the 4-aminoantipyrine-containing partial composition to the mixture, and measuring the degree of color development. In this way, the glycated protein can be assayed. However, in some cases, the Trinder reagent-containing partial composition and the 4-aminoantipyrine-containing partial composition may be added and mixed at the same time when added to the sample, or the Trinder reagent-containing partial composition and the 4-aminoantipyrine-containing partial composition may be mixed in advance and immediately used. Alternatively, the 4-aminoantipyrine-containing partial composition may be added first to the sample, and then, the Trinder reagent-containing partial composition can be added.

In the glycated protein assay reagent according to an embodiment, when the ferrocyanide is contained in the ferrocyanide-containing partial composition, for example, the 4-aminoantipyrine-containing partial composition and the ferrocyanide-containing partial composition are mixed in advance to provide a 4-aminoantipyrine- and ferrocyanide-containing partial composition. In this case, the glycated protein can be assayed by first adding the Trinder reagent-containing partial composition to a sample, leaving the mixture of the sample and the Trinder reagent-containing partial composition for several minutes, for example, approximately 5 to 10 minutes, then adding the 4-aminoantipyrine- and ferrocyanide-containing partial composition to the mixture, and measuring the degree of color development. However, in some cases, the Trinder reagent-containing partial composition and the 4-aminoantipyrine- and ferrocyanide-containing partial composition may be added and mixed at the same time when added to the sample, or the Trinder reagent-containing partial composition and the 4-aminoantipyrine- and ferrocyanide-containing partial composition may be mixed in advance and immediately used. Alternatively, the 4-aminoantipyrine- and ferrocyanide-containing partial composition may be added first to the sample, and then, the Trinder reagent-containing partial composition can be added. Alternatively, the Trinder reagent-containing partial composition and the ferrocyanide-containing partial composition may be mixed in advance to provide a Trinder reagent- and ferrocyanide-containing partial composition. In this case, the glycated protein can be assayed by first adding the 4-aminoantipyrine-containing partial composition to a sample, leaving the mixture of the sample and the 4-aminoantipyrine-containing partial composition for several minutes, for example, approximately 5 to 10 minutes, then adding the Trinder reagent- and ferrocyanide-containing partial composition to the mixture, and measuring the degree of color development. However, in some cases, the 4-aminoantipyrine-containing partial composition and the Trinder reagent- and ferrocyanide-containing partial composition may be added and mixed at the same time when added to the sample, or the 4-aminoantipyrine-containing partial composition and the Trinder reagent- and ferrocyanide-containing partial composition may be mixed in advance and immediately used. Alternatively, the Trinder reagent- and ferrocyanide-containing partial composition may be added first to the sample, and then, the 4-aminoantipyrine-containing partial composition can be added. Alternatively, the Trinder reagent-containing partial composition and the 4-aminoantipyrine-containing partial composition may be mixed in advance to provide a Trinder reagent- and 4-aminoantipyrine-containing partial composition. In this case, first, the ferrocyanide-containing partial composition may be mixed with a sample, and the mixture of the sample and the ferrocyanide-containing partial composition can be left for several minutes, for example, approximately 5 to 10 minutes, followed by the mixing therewith of the Trinder reagent- and 4-aminoantipyrine-containing partial composition. In some cases, the ferrocyanide-containing partial composition and the Trinder reagent- and 4-aminoantipyrine-containing partial composition may be added and mixed at the same time when added to the sample, or the ferrocyanide-containing partial composition and the Trinder reagent- and 4-aminoantipyrine-containing partial composition may be mixed in advance and added. Alternatively, the Trinder reagent- and 4-aminoantipyrine-containing partial composition may be mixed first with the sample, and then, the ferrocyanide-containing partial composition can be added. Alternatively, the sample, the Trinder reagent-containing partial composition, the 4-aminoantipyrine-containing partial composition, and the ferrocyanide-containing partial composition may be added and mixed at the same time, or the Trinder reagent-containing partial composition, the 4-aminoantipyrine-containing partial composition, and the ferrocyanide-containing partial composition may be mixed in advance and added to the sample.

For the quantification of the glycated protein, usually, one or more substances for calibrations (calibrators) having a known glycated protein concentration are often assayed and calibrated. Those skilled in the art can carry out a preparation method for the calibrators or a calibration method on the basis of information known in the art. For example, these methods can be appropriately carried out with reference to the description of International Publication No. WO 2001/094618, Japanese Patent Laid-Open No. 2005-261383, etc. Calibration is usually performed on a regular basis (e.g., once a month) in order to correct variation in assay value ascribable to reduction in sensitivity during assay reagent preservation. On the other hand, it is known that a reagent that suppresses reduction in sensitivity can produce stable assay values over a long period by only initial calibration, without calibration in subsequent assay, and permits non-calibration assay.

The protease can be contained in at least one of the Trinder reagent-containing partial composition, the 4-aminoantipyrine-containing partial composition and the ferrocyanide-containing partial composition. The protease can degrade the glycated protein to effectively form a glycated amino acid or a glycated peptide. The protease is, for example, endopeptidase. Serine endopeptidase is exemplified as the endopeptidase. Protease derived from microbes such as the genus *Bacillus,* the genus *Streptomyces,* the genus *Tritirachium,* and the genus *Aspergillus* and genetically recombinant protease thereof are exemplified as the protease. Alcalase, Neutrase, Esperase, and Savinase (all manufactured by Novozymes A/S), Bioprase OP, Bioprase SP-20FG, Bioprase 30L, Bioprase 30G, Bioprase AL-15FG, Bioprase APL-30, and Protease CL-15 (all manufactured by Nagase ChemteX Corp.), Protin SD PC-10CF, Thermoase PC-10F, Protin SD-AY10, and Protin SD-NY10 (all manufactured by Amano Enzyme Inc.), Multifect PR6L, Optimase PR40L, Optimase PR40X, and Optimase PR40E (all manufactured by Danisco Japan Ltd.), Orientase 22BF, Nucleicin, Orientase 10NL, and Orientase 90N (all manufactured by HBI Enzymes Inc.), Aroase AP-10, Aroase NP-10, and Aroase XA-10 (all manufactured by Yakult Pharmaceutical Industry Co., Ltd.), subtilisin, bacterial proteinase type XXIV, protease *Bacillus licheniformis-derived* type VIII, protease *Bacillus polymyxa-derived* type IX, thermolysin *Geobacillus stearothermophilus-derived* type X, protease *Bacillus polymyxa-derived* type XV, protease *Bacillus* sp.-derived type XXVII, and protease *Bacillus licheniformis*-derived type XXXI (all manufactured by Sigma-Aldrich Co., LLC), thermolysin (manufactured by FUJIFILM Wako Pure Chemical Corp.), Dispase I and Dispase II (both manufactured by Godo Shusei Co., Ltd.), and Neutral Proteinase (manufactured by TOYOBO USA) are exemplified as the protease derived from the genus *Bacillus.* Pronase and protease *Streptomyces* griseus-derived type XIV (both manufactured by Sigma-Aldrich Co., LLC), alkalophilic proteinase (manufactured by TOYOBO USA), and Denazyme PMC SOFTER (manufactured by Nagase ChemteX Corp.) are exemplified as the protease derived from the genus *Streptomyces.* Proteinase K (manufactured by Sigma-Aldrich Co., LLC) is exemplified as the protease derived from the genus *Tritirachium.* Protease P "Amano" 3SD, protease A "Amano" SD, and protease M "Amano" SD (all manufactured by Amano Enzyme Inc.), Sumizyme MP, Sumizyme LPL-G, Sumizyme LP50D, and Sumizyme AP (all manufactured by Shinnihon Chemicals Corp.), Orientase OP and Orientase AY (both manufactured by HBI Enzymes Inc.), protease *Aspergillus* saitoi-derived type XIII, protease *Aspergillus* sojae-derived type XIX, protease *Aspergillus* melleus-derived type XXIII, and protease *Aspergillus* oryzae-derived (all manufactured by Sigma-Aldrich Co., LLC), Denazyme AP and Denapsin 2P (both manufactured by Nagase ChemteX Corp.), and Pantidase NP-2, Pantidase MP, and Pantidase P (all manufactured by Yakult Pharmaceutical Industry Co., Ltd.) are exemplified as the protease derived from the genus *Aspergillus.* Among them, Alcalase, Bioprase SP-20FG, Protin SD-AY10, Multifect PR6L, Optimase PR40L, Aroase XA-10, subtilisin, Pronase, proteinase K, and PR "Amano" K are preferable.

From another viewpoint, protease of enzyme commission No. EC: 3.4 or EC: 3.4.21 is exemplified as the preferable protease. Protease of EC:3.4.21.62 is also another preferred example.

The stabilizer of the protease according to the present embodiment can be contained in at least one of the Trinder reagent-containing partial composition, the 4-aminoantipyrine-containing partial composition and the ferrocyanide-containing partial composition. The stabilizer of the protease according to the present embodiment increases the oxidation-reduction potential of the ferrocyanide above 0.058 V when the stabilizer of the protease and the ferrocyanide are mixed. The oxidation-reduction potential is an oxidation-reduction potential to be measured in a reaction system containing the stabilizer of the protease and the ferrocyanide and not containing glycated protein. The reaction system may further comprise N-tris(hydroxymethyl)methyl-2-aminoethanesulfonic acid and sodium chloride. The oxidation-reduction potential may be measured using a sliver/silver chloride electrode as a reference electrode. The reaction system to assay the oxidation-reduction potential will be more specifically described later in Example 1.

The lower limit of the oxidation-reduction potential may be, for example, higher than 0.058 V, 0.070 V or higher, 0.084 V or higher, or 0.112 V or higher. The upper limit of the oxidation-reduction potential may be, for example, 0.400 V or lower, 0.300 V or lower, 0.250 V or lower, or 0.235 V or lower.

Propylene glycol, trimethylene glycol, ethylene glycol, and carboxyphenylboronic acid, and combinations thereof are used as the stabilizer of the protease. 2-carboxyphenylboronic acid is exemplified as the carboxyphenylboronic acid. The stabilizer is preferably propylene glycol in terms of effective concentration, solubility, and cost, etc. In the present embodiment, the stabilizer of the protease excludes dimethyl sulfoxide. A stabilizer that does not increase the oxidation-reduction potential of the ferrocyanide above 0.058 V when the stabilizer of the protease and the ferrocyanide are mixed is excluded from the stabilizer of the protease according to the present embodiment.

The ferrocyanide can be contained in at least one of the Trinder reagent-containing partial composition, the 4-aminoantipyrine-containing partial composition and the ferrocyanide-containing partial composition. The ferrocyanide prevents bilirubin contained in a specimen from affecting the assay of the glycated protein. Examples of the ferrocyanide may be any compound containing a ferrocyanide ion. Potassium ferrocyanide ($Fe(CN)_6K_4$) and sodium ferrocyanide ($Fe(CN)_6Na_4$) are exemplified as the ferrocyanide.

In the glycated protein assay reagent according to an embodiment, when the ferrocyanide is contained in at least one of the Trinder reagent-containing partial composition and the 4-aminoantipyrine-containing partial composition, the protease and the stabilizer of the protease are contained in, for example, one of the Trinder reagent-containing partial composition and the 4-aminoantipyrine-containing partial composition. Alternatively, the protease, the stabilizer of the protease and the ferrocyanide are contained in, for example, one of the Trinder reagent-containing partial composition and the 4-aminoantipyrine-containing partial composition. Preferably, the protease, the stabilizer of the protease, and the ferrocyanide are contained in the 4-aminoantipyrine-containing partial composition.

In the glycated protein assay reagent according to an embodiment, when the ferrocyanide is contained in the ferrocyanide-containing partial composition, the protease and the stabilizer of the protease are contained in, for example, one of the Trinder reagent-containing partial composition, the 4-aminoantipyrine-containing partial composition, and the ferrocyanide-containing partial composition. For example, the protease and the stabilizer of the protease are contained in the Trinder reagent-containing partial composition. Alternatively, the protease and the stabilizer of the protease are contained in the 4-aminoantipyrine-containing partial composition. Alternatively, the protease and the stabilizer of the protease are contained in the ferrocyanide-containing partial composition.

The fructosyl amino acid oxidase contained in at least one of the Trinder reagent-containing partial composition, the 4-aminoantipyrine-containing partial composition and the ferrocyanide-containing partial composition can be fructosyl amino acid oxidase that acts effectively on a glycated amino acid or a glycated peptide derived from a human protein. Fructosyl amino acid oxidase derived from the genus *Gibberella,* the genus *Aspergillus,* the genus *Candida,* the genus *Penicillium,* the genus *Fusarium,* the genus *Acremonium,* the genus *Debaryomyces,* and the genus *Corynebacterium,* and genetically recombinant fructosyl amino acid oxidase variants thereof are exemplified as the fructosyl amino acid oxidase. Further specifically, ketoamine oxidase (KAOD, manufactured by Asahi Kasei Pharma Corp.; described in Clinica Chimica Acta, 2002, 324, p. 61-71), variant KAOD (KAOD-V, manufactured by Asahi Kasei Pharma Corp.; described in Patent Literature 1), and FAOD-E (manufactured by Kikkoman Corp.) are exemplified as the fructosyl amino acid oxidase.

N-(3-sulfopropyl)aniline (HALPS), N-ethyl-N-(3-sulfopropyl)-3-methylaniline (TOPS), N-ethyl-N-(2-hydroxy-3-sulfopropyl)-3,5-dimethylaniline (MAOS), N-(3-sulfopropyl)-3,5-dimethoxyaniline (HDAPS), N-(2-hydroxy-3-sulfopropyl)-3,5-dimethoxyaniline (HDAOS), N-ethyl-N-(3-sulfopropyl)-3,5-dimethoxyaniline (DAPS), N-ethyl-N-(2-hydroxy-3-sulfopropyl)-3,5-dimethoxyaniline (DAOS), N-ethyl-N-(3-sulfopropyl)aniline (ALPS), N-ethyl-N-(3-sulfopropyl)-3-methoxyaniline (ADPS), N-ethyl-N-(2-hydroxy-3-sulfopropyl)-3-methoxyaniline (ADOS), N-ethyl-N-(2-hydroxy-3-sulfopropyl)-3-methylaniline (TOOS), N,N-bis(4-sulfobutyl)-3-methylaniline (TODB), and 3-hydroxy-2,4,6-triiodobenzoic acid (HTIB), and salts thereof are specifically exemplified as the Trinder reagent contained in the Trinder reagent-containing partial composition. Among them, TOPS, ALPS, TOOS, and TODB are preferable.

In the case of using TOPS, the wavelength of color development is 550 nm. In the case of using ALPS, the wavelength of color development is 561 nm. In the case of using TOOS, the wavelength of color development is 555 nm. In the case of using TODB, the wavelength of color development is 550 nm. However, the wavelength of assay using an apparatus for assaying absorbance does not have to be in exact agreement with the wavelength of color development. For example, an apparatus for assaying absorbance at a wavelength of 546 nm is capable of assaying chromogenic reaction in the case of using each of TOPS, ALPS, TOOS, and TODB.

If the Trinder reagent and the 4-aminoantipyrine are contained in the same reagent, they are spontaneously oxidatively condensed and emit color during preservation. Therefore, the Trinder reagent is contained in the Trinder reagent-containing partial composition and the 4-aminoantipyrine is contained in the 4-aminoantipyrine-containing partial composition.

In conventional techniques, nonspecific chromogenic reaction occurs due to peroxide derived from an assay reagent, a specimen, etc., and this absorbance may reduce assay accuracy. Such nonspecific color development is called as reagent blank. Regarding this problem, the peroxidase contained in one of the Trinder reagent-containing partial composition, the 4-aminoantipyrine-containing partial composition, and the ferrocyanide-containing partial composition accelerates the oxidative condensation reaction of the Trinder reagent and the 4-aminoantipyrine and also functions to reduce a reagent blank by eliminating hydrogen peroxide. Peroxidase of any origin can be used. Peroxidase derived from plants such as horseradish, and peroxidase derived from microbes such as bacteria and mold are exemplified as the peroxidase. Specifically, peroxidase from horseradish (manufactured by Sigma-Aldrich Co., LLC), peroxidase derived from horseradish (manufactured by FUJIFILM Wako Pure Chemical Corp.), PO "AMANO" 3 (manufactured by Amano Enzyme Inc.) are exemplified as the peroxidase.

At least one of the 4-aminoantipyrine-containing partial composition and the ferrocyanide-containing partial composition may further comprise a chelating agent. Use of a salt of the chelating agent suppresses pH variation and is therefore preferred.

Ethylenediaminetetraacetic acid, glycol ether diaminetetraacetic acid, N-(2-hydroxyethyl)iminodiacetic acid, nitrilotris(methylphosphonic acid), nitrilotriacetic acid, trans-1,2-diaminocyclohexane-N,N,N,N-tetraacetic acid, iminodiacetic acid, diethylenetriamine-N,N,N',N'',N''-pentaacetic acid, 1,3-diamino-2-propanol-N,N,N',N'-tetraacetic acid, gluconic acid, malic acid, succinic acid, citric acid, salicylic acid, tartaric acid, N-[tris(hydroxymethyl)methyl]glycine, N,N-bis(2-hydroxyethyl)glycine, and salts thereof are exemplified as the chelating agent. The chelating agent is preferably, for example, citric acid or a salt thereof.

The salt of the chelating agent is not particularly limited by its type as long as a salt is formed. Any acid-addition salt or base-addition salt can be used, and the form of a zwitterion may be adopted. Examples of the base-addition salt include base-addition salts with inorganic bases such as sodium, potassium, magnesium, calcium, and aluminum, and base-addition salts with organic bases such as methylamine, 2-aminoethanol, arginine, lysine, and ornithine. Among them, a base-addition salt with an inorganic base is preferable. Typical examples of the base-addition salt include sodium salt.

Each of the 4-aminoantipyrine-containing partial composition, the Trinder reagent-containing partial composition, and the ferrocyanide-containing partial composition may further comprise a solvent such as water or an organic solvent. Alcohol solvents (e.g., alcohols having 1 to 18 carbon atoms, specifically, methanol, butanol, ethylene glycol, and glycerin), ketone solvents (acetone and methyl ethyl ketone, etc.), and ether solvents (diethyl ether, ethylene glycol monoalkyl ether, and cyclic ether typified by tetrahydrofuran, etc.) are exemplified as the organic solvent.

Each of the 4-aminoantipyrine-containing partial composition, the Trinder reagent-containing partial composition, and the ferrocyanide-containing partial composition may comprise a buffer. Buffers containing N-tris(hydroxymethyl) methyl-2-aminoethanesulfonic acid (TES), 3-[4-(2-hydroxyethyl)-1-piperazinyl]propanesulfonic acid (EPPS), 2-[4-(2-hydroxyethyl)-1-piperazinyl]ethanesulfonic acid (HEPES), 2-hydroxy-3-[4-(2-hydroxyethyl)-1-piperazinyl]propanesulfonic acid (HEPPSO), N-(2-acetamido)-2-aminoethanesulfonic acid (ACES), N-(2-acetamido)iminodiacetic acid (ADA), N,N-bis(2-hydroxyethyl)-2-aminoethanesulfonic acid (BES), N,N-bis(2-hydroxyethyl)glycine (Bicine), bis(2-hydroxyethyl)iminotris(hydroxymethyl)methane (Bis-Tris), N-cyclohexyl-3-aminopropanesulfonic acid (CAPS), N-cyclohexyl-2-hydroxy-3-aminopropanesulfonic acid (CAPSO), N-cyclohexyl-2-aminoethanesulfonic acid (CHES), 3-[N,N-bis(2-hydroxyethyl)amino]-2-hydroxypropanesulfonic acid (DIPSO), 2-morpholinoethanesulfonic acid (MES), 3-morpholinopropanesulfonic acid (MOPS), 2-hydroxy-3-morpholinopropanesulfonic acid (MOPSO), piperazine-1,4-bis(2-ethanesulfonic acid) (PIPES), piperazine-1,4-bis(2-hydroxy-3-propanesulfonic acid) (POPSO), N-tris(hydroxymethyl)methyl-3-aminopropanesulfonic acid (TAPS), 2-hydroxy-N-tris(hydroxymethyl)methyl-3-aminopropanesulfonic acid (TAPSO), N-[tris(hydroxymethyl)methyl]glycine (Tricine), and tris(hydroxymethyl)aminomethane (Tris), and boric acid, ammonia, glycine, carbonic acid, acetic acid, phosphoric acid, diethanolamine, p-phenolsulfonic acid, 2-amino-2-methylpropane-1,3-diol, cacodylic acid, maleic acid, veronal and 3,3-dimethylglutaric acid are exemplified as the buffer.

Each of the 4-aminoantipyrine-containing partial composition, the Trinder reagent-containing partial composition, and the ferrocyanide-containing partial composition may further comprise other additives such as an antiseptic, an enzyme stabilizer, a surfactant, and other enzymes. Sodium azide and ProClin are exemplified as the antiseptic. Any enzyme stabilizer can be used as long as the stabilizer stabilizes an enzyme during preservation. Sugars such as cyclodextrin, sucrose, mannose, fructose, lactose, galactose and trehalose, and sugar alcohols such as sorbitol and mannitol are exemplified as the enzyme stabilizer. Any cationic surfactant, anionic surfactant, amphoteric surfactant, and nonionic surfactant may be used as the surfactant. Polyoxyethylene alkyl ether, polyoxyethylene alkyl phenyl ether, polyoxyethylene polyoxypropylene alkyl ether, sorbitan fatty acid ester, polyoxyethylene sorbitan fatty acid ester, glycerin fatty acid ester, and polyoxyethylene polyoxypropylene block polymers are exemplified as the nonionic surfactant. Alkyl betaine, betaine acetate, sulfobetaine, and alkylamine oxide are exemplified as the amphoteric surfactant. Ascorbic acid oxidase, bilirubin oxidase, and catalase, which are used for preventing ascorbic acid, bilirubin, or endogenous peroxide contained in a specimen from affecting assay values are exemplified as other enzymes.

The concentration of the 4-aminoantipyrine in the 4-aminoantipyrine-containing partial composition can be a concentration sufficient for reaction with the generated hydrogen peroxide. The lower limit is, for example, 0.01 mmol/L or higher, preferably 0.1 mmol/L or higher, more preferably 0.5 mmol/L or higher. The upper limit is, for example, 100 mmol/L or lower, preferably 50 mmol/L or lower, more preferably 30 mmol/L or lower, from the viewpoint of cost.

The concentration of the protease in the 4-aminoantipyrine-containing partial composition, the Trinder reagent-containing partial composition, or the ferrocyanide-containing partial composition can be a concentration at which the glycated protein can be degraded within an appropriate time. The lower limit is, for example, 100 U/mL or higher, preferably 1 kU/mL or higher, more preferably 10 kU/mL or higher. The upper limit is, for example, 1000 kU/mL or lower, preferably 200 kU/mL or lower, more preferably 100 kU/mL or lower, from the viewpoint of cost.

The concentration of the stabilizer of the protease in the glycated protein assay reagent according to an embodiment is a concentration that can increase the oxidation-reduction potential of the ferrocyanide above 0.058 V when the stabilizer of the protease and the ferrocyanide are mixed in a reaction system containing the stabilizer of the protease and the ferrocyanide and not containing glycated protein.

In the glycated protein assay reagent according to an embodiment, for example, when the Trinder reagent-containing partial composition contains the stabilizer of the protease and the ferrocyanide, the concentration of the stabilizer of the protease in the Trinder reagent-containing partial composition is adjusted so as to be the same as the concentration of the stabilizer of the protease that can increase the oxidation-reduction potential of the ferrocyanide above 0.058 V when the stabilizer of the protease and the ferrocyanide are mixed in a reaction system containing the stabilizer of the protease and the ferrocyanide and not containing glycated protein.

For example, when the 4-aminoantipyrine-containing partial composition contains the stabilizer of the protease and the ferrocyanide, the concentration of the stabilizer of the protease in the 4-aminoantipyrine-containing partial composition is adjusted so as to be the same as the concentration of the stabilizer of the protease that can increase the oxidation-reduction potential of the ferrocyanide above 0.058 V when the stabilizer of the protease and the ferrocyanide are mixed in a reaction system containing the stabilizer of the protease and the ferrocyanide and not containing glycated protein.

For example, when the Trinder reagent-containing partial composition contains the stabilizer of the protease and the 4-aminoantipyrine-containing partial composition contains the ferrocyanide, the concentration after mixing the Trinder reagent-containing partial composition with the 4-aminoantipyrine-containing partial composition is adjusted so as to be the same as the concentration of the stabilizer of the protease that can increase the oxidation-reduction potential of the ferrocyanide above 0.058 V when the stabilizer of the protease and the ferrocyanide are mixed in a reaction system containing the stabilizer of the protease and the ferrocyanide and not containing glycated protein.

For example, when the 4-aminoantipyrine-containing partial composition contains the stabilizer of the protease and the Trinder reagent-containing partial composition contains the ferrocyanide, the concentration after mixing the 4-aminoantipyrine-containing partial composition with the Trinder reagent-containing partial composition is adjusted so as to be the same as the concentration of the stabilizer of the protease that can increase the oxidation-reduction potential of the ferrocyanide above 0.058 V when the stabilizer of the protease and the ferrocyanide are mixed in a reaction system containing the stabilizer of the protease and the ferrocyanide and not containing glycated protein.

For example, when the 4-aminoantipyrine-containing partial composition or the Trinder reagent-containing partial composition contains the stabilizer of the protease and the ferrocyanide is contained in the ferrocyanide-containing partial composition, the concentration of the stabilizer of the protease after mixing any one containing the protease stabilizer, of the Trinder reagent-containing partial composition and the 4-aminoantipyrine-containing partial composition, with the ferrocyanide-containing partial composition is adjusted so as to be the same as the concentration of the stabilizer of the protease that can increase the oxidation-reduction potential of the ferrocyanide above 0.058 V when the stabilizer of the protease and the ferrocyanide are mixed in a reaction system containing the stabilizer of the protease and the ferrocyanide and not containing glycated protein.

For example, when the ferrocyanide-containing partial composition contains the ferrocyanide and the stabilizer of the protease, the concentration of the stabilizer of the protease in the ferrocyanide-containing partial composition is adjusted so as to be the same as the concentration of the stabilizer of the protease that can increase the oxidation-reduction potential of the ferrocyanide above 0.058 V when the stabilizer of the protease and the ferrocyanide are mixed in a reaction system containing the stabilizer of the protease and the ferrocyanide and not containing glycated protein.

When the 4-aminoantipyrine-containing partial composition or the Trinder reagent-containing partial composition contains the stabilizer of the protease and the stabilizer of the protease is propylene glycol, the lower limit of the concentration of the propylene glycol in the 4-aminoantipyrine-containing partial composition or the Trinder reagent-containing partial composition is, for example, 7.5 wt/vol% or higher, 10 wt/vol% or higher, 15 wt/vol% or higher, 20 wt/vol% or higher, 22.5 wt/vol% or higher, 25 wt/vol% or higher, 27.5 wt/vol% or higher, 30 wt/vol% or higher, 35 wt/vol% or higher, or 37.5 wt/vol% or higher. The upper limit of the concentration of the propylene glycol in the 4-aminoantipyrine-containing partial composition or the Trinder reagent-containing partial composition is, for example, 80 wt/vol% or lower, 62.5 wt/vol% or lower, 60 wt/vol% or lower, 57.5 wt/vol% or lower, 55 wt/vol% or lower, 52.5 wt/vol% or lower, or 50 wt/vol% or lower.

When the 4-aminoantipyrine-containing partial composition or the Trinder reagent-containing partial composition contains the stabilizer of the protease and the stabilizer of the protease is trimethylene glycol, the lower limit of the concentration of the trimethylene glycol in the 4-aminoantipyrine-containing partial composition or the Trinder reagent-containing partial composition is, for example, 40 wt/vol% or higher, 42.5 wt/vol% or higher, 45 wt/vol% or higher, 47.5 wt/vol% or higher, 50 wt/vol% or higher, 52.5 wt/vol% or higher, 55 wt/vol% or higher, 57.5 wt/vol% or higher, 60 wt/vol% or higher, or 62.5 wt/vol% or higher. The upper limit of the concentration of the trimethylene glycol in the 4-aminoantipyrine-containing partial composition or the Trinder reagent-containing partial composition is, for example, 80 wt/vol% or lower, 75 wt/vol% or lower, or 70 wt/vol% or lower.

When the 4-aminoantipyrine-containing partial composition or the Trinder reagent-containing partial composition contains the stabilizer of the protease and the stabilizer of the protease is ethylene glycol, the lower limit of the concentration of the ethylene glycol in the 4-aminoantipyrine-containing partial composition or the Trinder reagent-containing partial composition is, for example, 40 wt/vol% or higher, 42.5 wt/vol% or higher, 45 wt/vol% or higher, 47.5 wt/vol% or higher, 50 wt/vol% or higher, 52.5 wt/vol% or higher, 55 wt/vol% or higher, 57.5 wt/vol% or higher, 60 wt/vol% or higher, or 62.5 wt/vol% or higher. The upper limit of the concentration of the ethylene glycol in the 4-aminoantipyrine-containing partial composition or the Trinder reagent-containing partial composition is, for example, 80 wt/vol% or lower, 75 wt/vol% or lower, or 70 wt/vol% or lower.

When the 4-aminoantipyrine-containing partial composition or the Trinder reagent-containing partial composition contains the stabilizer of the protease and the stabilizer of the protease is 2-carboxyphenylboronic acid, the lower limit of the concentration of the 2-carboxyphenylboronic acid in the 4-aminoantipyrine-containing partial composition or the Trinder reagent-containing partial composition is, for example, 0.2 wt/vol% or higher, 0.45 wt/vol% or higher, 0.5 wt/vol% or higher, 0.55 wt/vol% or higher, 0.6 wt/vol% or higher, or 0.75 wt/vol% or higher. The upper limit of the concentration of the 2-carboxyphenylboronic acid in the 4-aminoantipyrine-containing partial composition or the Trinder reagent-containing partial composition is, for example, 10 wt/vol% or lower, 7.5 wt/vol% or lower, or 5 wt/vol% or lower.

When the ferrocyanide-containing partial composition contains the stabilizer of the protease and the stabilizer of the protease is propylene glycol, the lower limit of the concentration of the propylene glycol in the ferrocyanide-containing partial composition is, for example, 7.5 wt/vol% or higher, 10 wt/vol% or higher, 15 wt/vol% or higher, 20 wt/vol% or higher, 22.5 wt/vol% or higher, 25 wt/vol% or higher, 27.5 wt/vol% or higher, 30 wt/vol% or higher, 35 wt/vol% or higher, or 37.5 wt/vol% or higher. The upper limit of the concentration of the propylene glycol in the ferrocyanide-containing partial composition is, for example, 80 wt/vol% or lower, 62.5 wt/vol% or lower, 60 wt/vol% or lower, 57.5 wt/vol% or lower, 55 wt/vol% or lower, 52.5 wt/vol% or lower, or 50 wt/vol% or lower.

When the ferrocyanide-containing partial composition contains the stabilizer of the protease and the stabilizer of the protease is trimethylene glycol, the lower limit of the concentration of the trimethylene glycol in the ferrocyanide-containing partial composition is, for example, 40 wt/vol% or higher, 42.5 wt/vol% or higher, 45 wt/vol% or higher, 47.5 wt/vol% or higher, 50 wt/vol% or higher, 52.5 wt/vol% or higher, 55 wt/vol% or higher, 57.5 wt/vol% or higher, 60 wt/vol% or higher, or 62.5 wt/vol% or higher. The upper limit of the concentration of the trimethylene glycol in the ferrocyanide-containing partial composition is, for example, 80 wt/vol% or lower, 75 wt/vol% or lower, or 70 wt/vol% or lower.

When the ferrocyanide-containing partial composition contains the stabilizer of the protease and the stabilizer of the protease is ethylene glycol, the lower limit of the concentration of the ethylene glycol in the ferrocyanide-containing partial composition is, for example, 40 wt/vol% or higher, 42.5 wt/vol% or higher, 45 wt/vol% or higher, 47.5 wt/vol% or higher, 50 wt/vol% or higher, 52.5 wt/vol% or higher, 55 wt/vol% or higher, 57.5 wt/vol% or higher, 60 wt/vol% or higher, or 62.5 wt/vol% or higher. The upper limit of the concentration of the ethylene glycol in the ferrocyanide-containing partial composition is, for example, 80 wt/vol% or lower, 75 wt/vol% or lower, or 70 wt/vol% or lower.

When the ferrocyanide-containing partial composition contains the stabilizer of the protease and the stabilizer of the protease is 2-carboxyphenylboronic acid, the lower limit of the concentration of the 2-carboxyphenylboronic acid in the ferrocyanide-containing partial composition is, for example, 0.2 wt/vol% or higher, 0.45 wt/vol% or higher, 0.5 wt/vol% or higher, 0.55 wt/vol% or higher, 0.6 wt/vol% or higher, or 0.75 wt/vol% or higher. The upper limit of the concentration of the 2-carboxyphenylboronic acid in the ferrocyanide-containing partial composition is, for example, 10 wt/vol% or lower, 7.5 wt/vol% or lower, or 5 wt/vol% or lower.

The concentration of the ferrocyanide in the Trinder reagent-containing partial composition, the 4-aminoantipyrine-containing partial composition or the ferrocyanide-containing partial composition can be a concentration sufficient for suppressing the affect of bilirubin contained in a specimen on the assay of the glycated protein. The lower limit is, for example, 0.001 mmol/L or higher, preferably 0.01 mmol/L or higher, more preferably 0.02 mmol/L or higher. The upper limit is, for example, 10 mmol/L or lower, preferably 1 mmol/L or lower, 0.9 mmol/L or lower, 0.8 mmol/L or lower, 0.7 mmol/L or lower, or 0.6 mmol/L or lower, more preferably 0.5 mmol/L or lower, from the viewpoint of cost.

The concentration of the fructosyl amino acid oxidase in the Trinder reagent-containing partial composition, the 4-aminoantipyrine-containing partial composition, or the ferrocyanide-containing partial composition can be a concentration sufficient for reaction with the generated glycated amino acid. The lower limit is, for example, 0.1 U/mL or higher, preferably 1 U/mL or higher, more preferably 10 U/mL or higher. The upper limit is, for example, 1000 U/mL or lower, preferably 200 U/mL or lower, more preferably 100 U/mL or lower, from the viewpoint of cost.

The concentration of the Trinder reagent in the Trinder reagent-containing partial composition can be a concentration sufficient for reaction with the generated hydrogen peroxide. The lower limit is, for example, 0.01 mmol/L or higher, preferably 0.1 mmol/L or higher, more preferably 0.5 mmol/L or higher. The upper limit is, for example, 100 mmol/L or lower, preferably 10 mmol/L or lower, more preferably 5 mmol/L or lower, from the viewpoint of cost.

The concentration of the peroxidase in the Trinder reagent-containing partial composition, the 4-aminoantipyrine-containing partial composition, or the ferrocyanide-containing partial composition can be a concentration sufficient for reaction with the generated hydrogen peroxide. The lower limit is, for example, 0.01 U/mL or higher, preferably 0.1 U/mL or higher, more preferably 1 U/mL or higher. The upper limit is, for example, 500 U/mL or lower, preferably 100 U/mL or lower, more preferably 50 U/mL or lower, from the viewpoint of cost.

The lower limit of the concentration of the chelating agent in the 4-aminoantipyrine-containing partial composition or the ferrocyanide-containing partial composition is, for example, 5 $\mu$mol/L or higher, preferably 10 $\mu$mol/L or higher, 50 $\mu$mol/L or higher, 75 $\mu$mol/L or higher, or 1 mmol/L or higher, more preferably 1.25 mmol/L or higher. The upper limit of the concentration of the chelating agent in the 4-aminoantipyrine-containing partial composition or the ferrocyanide-containing partial composition is, for example, 1000 mmol/L or lower, preferably 100 mmol/L or lower, 25 mmol/L or lower, 15 mmol/l or lower, or 10 mmol/l or lower, more preferably 5 mmol/L or lower.

The Trinder reagent-containing partial composition may be liquid and may be preserved until mixed with the 4-aminoantipyrine-containing partial composition. In the Trinder reagent-containing partial composition, the contained components are mixed. The 4-aminoantipyrine-containing partial composition may be liquid and may be preserved until mixed with the Trinder reagent-containing partial composition. In the 4-aminoantipyrine-containing partial composition, the contained components are mixed. The 4-aminoantipyrine-containing partial composition and the Trinder reagent-containing partial composition may be each individually packaged.

When the glycated protein assay reagent according to an embodiment contains the ferrocyanide-containing partial composition, the ferrocyanide-containing partial composition may be liquid and may be preserved until mixed with the Trinder reagent-containing partial composition and the 4-aminoantipyrine-containing partial composition. In the ferrocyanide-containing partial composition, the contained components are mixed. The ferrocyanide-containing partial composition, the 4-aminoantipyrine-containing partial composition, and the Trinder reagent-containing partial composition may be each individually packaged.

The lower limit of the pH of the 4-aminoantipyrine-containing partial composition and/or the ferrocyanide-containing partial composition is, for example, 5 or larger, 5.5 or larger, 6 or larger, 6.5 or larger, or 7 or larger. The pH of the 4-aminoantipyrine-containing partial composition and/or the ferrocyanide-containing partial composition may be, for example, larger than 7. The upper limit of the pH of the 4-aminoantipyrine-containing partial composition and/or the ferrocyanide-containing partial composition is, for example, 10 or smaller, 9.5 or smaller, or 9 or smaller.

A glycated protein assay reagent not containing stabilizer of the protease may present problems associated with preservation stability. Regarding this problem, the glycated protein assay reagent according to an embodiment contains the stabilizer of the protease and therefore has favorable preservation stability over a long period. Hence, the glycated protein can be accurately assayed over a long period.

The glycated protein assay reagent according to an embodiment can be stably preserved, for example, for a practical period required for distribution. The glycated protein assay reagent according to an embodiment can be preserved, for example, for at least 1 month. The lower limit of the preservation period of the glycated protein assay reagent according to an embodiment is, for example, 1 day or longer, 1 month or longer, or 6 months or 1 year or longer. The upper limit of the preservation period of the glycated protein assay reagent according to an embodiment is, for example, 5 years or shorter, 2 years or shorter, 1 year and 6 months or shorter, 1 year and 3 months or shorter, 6 months or shorter, or 7 days or shorter.

The lower limit of the preservation temperature of the glycated protein assay reagent according to an embodiment is, for example, 2°C or higher, 4°C or higher, or 5°C or higher. The upper limit of the preservation temperature of the glycated protein assay reagent according to an embodiment is, for example, 10°C or lower, 8°C or lower, or 5°C or lower.

In the glycated protein assay reagent according to an embodiment, when the ferrocyanide is contained in at least one of the Trinder reagent-containing partial composition and the 4-aminoantipyrine-containing partial composition, the ratio of glycated protein assay sensitivity in the case of using in combination the Trinder reagent-containing partial composition or the 4-aminoantipyrine-containing partial composition preserved at 5°C for 28 days and the 4-aminoantipyrine-containing partial composition or the Trinder reagent-containing partial composition preserved at 30°C for 28 days to glycated protein assay sensitivity in the case of using in combination the Trinder reagent-containing partial composition or the 4-aminoantipyrine-containing partial composition preserved at 5°C for 14 days and the 4-aminoantipyrine-containing partial composition or the Trinder reagent-containing partial composition preserved at 30°C for 14 days is referred to as a sensitivity preservation rate. The lower limit of the sensitivity preservation rate is, for example, 80% or more, 85% or more, 88% or more, 90% or more, 91% or more, 92% or more, 93% or more, 94% or more, or 95% or more. The upper limit is, for example, 120% or less, 110% or less, or 105% or less.

In the glycated protein assay reagent according to an embodiment, when the ferrocyanide is contained in the 4-aminoantipyrine-containing partial composition, the ratio of glycated protein assay sensitivity in the case of using in combination the Trinder reagent-containing partial composition preserved at 5°C for 28 days and the 4-aminoantipyrine-containing partial composition preserved at 30°C for 28 days to glycated protein assay sensitivity in the case of using in combination the Trinder reagent-containing partial composition preserved at 5°C for 14 days and the 4-aminoantipyrine-containing partial composition preserved at 30°C for 14 days is referred to as a sensitivity preservation rate. The lower limit of the sensitivity preservation rate is, for example, 80% or more, 85% or more, 88% or more, 90% or more, 91% or more, 92% or more, 93% or more, 94% or more, or 95% or more. The upper limit is, for example, 120% or less, 110% or less, or 105% or less.

In the glycated protein assay reagent according to an embodiment, when the ferrocyanide is contained in the Trinder reagent-containing partial composition, the ratio of glycated protein assay sensitivity in the case of using in combination the 4-aminoantipyrine-containing partial composition preserved at 5°C for 28 days and the Trinder reagent-containing partial composition preserved at 30°C for 28 days to glycated protein assay sensitivity in the case of using in combination the 4-aminoantipyrine-containing partial composition preserved at 5°C for 14 days and the Trinder reagent-containing partial composition preserved at 30°C for 14 days is referred to as a sensitivity preservation rate. The lower limit of the sensitivity preservation rate is, for example, 80% or more, 85% or more, 88% or more, 90% or more, 91% or more, 92% or more, 93% or more, 94% or more, or 95% or more. The upper limit is, for example, 120% or less, 110% or less, or 105% or less.

In the glycated protein assay reagent according to an embodiment, when the ferrocyanide is contained in the ferrocyanide-containing partial composition, the ratio of glycated protein assay sensitivity in the case of using in combination the Trinder reagent-containing partial composition or the 4-aminoantipyrine-containing partial composition preserved at 5°C for 28 days and the 4-aminoantipyrine-containing partial composition or the Trinder reagent-containing partial composition and the ferrocyanide-containing partial composition preserved at 30°C for 28 days to glycated protein assay sensitivity in the case of using in combination the Trinder reagent-containing partial composition or the 4-aminoantipyrine-containing partial composition preserved at 5°C for 14 days and the 4-aminoantipyrine-containing partial composition or the Trinder reagent-containing partial composition and the ferrocyanide-containing partial composition preserved at 30°C for 14 days is referred to as a sensitivity preservation rate. The lower limit of the sensitivity preservation rate is, for example, 80% or more, 85% or more, 88% or more, 90% or more, 91% or more, 92% or more, 93% or more, 94% or more, or 95% or more. The upper limit is, for example, 120% or less, 110% or less, or 105% or less.

In the glycated protein assay reagent according to an embodiment, when the ferrocyanide is contained in the ferrocyanide-containing partial composition, the ratio of glycated protein assay sensitivity in the case of using in combination the Trinder reagent-containing partial composition preserved at 5°C for 28 days and the 4-aminoantipyrine-containing partial composition and the ferrocyanide-containing partial composition preserved at 30°C for 28 days to glycated protein assay sensitivity in the case of using in combination the Trinder reagent-containing partial composition preserved at 5°C for 14 days and the 4-aminoantipyrine-containing partial composition and the ferrocyanide-containing partial composition preserved at 30°C for 14 days is referred to as a sensitivity preservation rate. The lower limit of the sensitivity preservation rate is, for example, 80% or more, 85% or more, 88% or more, 90% or more, 91% or more, 92% or more, 93% or more, 94% or more, or 95% or more. The upper limit is, for example, 120% or less, 110% or less, or 105% or less.

In the glycated protein assay reagent according to an embodiment, when the glycated protein assay reagent contains the Trinder reagent-containing partial composition and the 4-aminoantipyrine-containing partial composition, the ratio of glycated protein assay sensitivity in the case of using in combination the Trinder reagent-containing partial composition and the 4-aminoantipyrine-containing partial composition preserved at 5°C for 1 year to glycated protein assay sensitivity in the case of using in combination the Trinder reagent-containing partial composition and the 4-aminoantipyrine-containing partial composition preserved at 5°C for 28 days is referred to as a long-term sensitivity preservation rate. Alternatively, when the glycated protein assay reagent contains the Trinder reagent-containing partial composition, the 4-aminoantipyrine-containing partial composition and the ferrocyanide-containing partial composition, the ratio of glycated protein assay sensitivity in the case of using in combination the Trinder reagent-containing partial composition, the 4-aminoantipyrine-containing partial composition, and the ferrocyanide-containing partial composition preserved at 5°C for 1 year to glycated protein assay sensitivity in the case of using in combination the Trinder reagent-containing partial composition, the 4-aminoantipyrine-containing partial composition, and the ferrocyanide-containing partial composition preserved at 5°C for 28 days is referred to as a long-term sensitivity preservation rate. The lower limit of the long-term sensitivity preservation rate is, for example, 80% or more, 85% or more, 88% or more, 90% or more, 91% or more, 92% or more, 93% or more, 94% or more, or 95% or more. The upper limit is, for example, 120% or less, 110% or less, or 105% or less.

Examples

Next, Examples of the present invention will be described. Examples which are outside the scope of the claims are provided purely as reference Examples.

Hitachi Automatic Analyzer 7170 model (manufactured by Hitachi High-Tech Corp.) was used in the measurement of absorbance in Examples. In the apparatus, the main wavelength was set to 546 nm, and the subwavelength was set to 700 nm. The absorbance Abs of a specimen was calculated according to the following expression (1):

$$Abs = Abs_{546} - Abs_{700} \quad (1)$$

wherein $Abs_{546}$ represents absorbance at a wavelength of 546 nm, and $Abs_{700}$ represents absorbance at a wavelength of 700 nm.

[Reference Example 1: Mechanism of occurrence of reagent blank]

(Preparation of Trinder reagent-containing liquid partial composition)

The following reagent components were dissolved in purified water so as to achieve the described concentrations. The solution was adjusted to pH 7.6 with sodium hydroxide to prepare a Trinder reagent-containing liquid partial composition.

100 mmol/L N-tris(hydroxymethyl)methyl-2-aminoethanesulfonic acid (manufactured by Dojindo Laboratories Co., Ltd.)
2 mmol/L N,N-bis(4-sulfobutyl)-3-methylaniline, disodium salt (manufactured by Dojindo Laboratories Co., Ltd.)

(Preparation of 4-aminoantipyrine- and ferrocyanide-containing liquid partial composition)

The following reagent components were dissolved in purified water so as to achieve the described concentrations. The solution was adjusted to pH 7.5 with sodium hydroxide to prepare a 4-aminoantipyrine- and ferrocyanide-containing liquid partial composition.

200 mmol/L 3,3-dimethylglutaric acid (manufactured by Tokyo Chemical Industry Co., Ltd.)
5 mmol/L 4-aminoantipyrine (manufactured by ACTEC Laboratories Inc.)
Stabilizer having a concentration described later
Ferrocyanide or mixture of ferrocyanide and ferricyanide having a concentration described later

(Stabilizer added to 4-aminoantipyrine- and ferrocyanide-containing liquid partial composition)

30 wt/vol% dimethyl sulfoxide (manufactured by Nacalai Tesque, Inc.)
30 wt/vol% ethanol (manufactured by Kanto Chemical Co., Inc.)
30 wt/vol% ethylene glycol (manufactured by FUJIFILM Wako Pure Chemical Corp.)
30 wt/vol% propylene glycol (manufactured by FUJIFILM Wako Pure Chemical Corp.)

(Ferrocyanide or mixture of ferrocyanide and ferricyanide added to 4-aminoantipyrine- and ferrocyanide-containing liquid partial composition)

0.1 mmol/L potassium ferrocyanide (manufactured by Kokusan Chemical Co., Ltd.)
0.09 mmol/L potassium ferrocyanide (manufactured by Kokusan Chemical Co., Ltd.) and 0.01 mmol/L potassium ferricyanide (manufactured by FUJIFILM Wako Pure Chemical Corp.)
0.07 mmol/L potassium ferrocyanide (manufactured by Kokusan Chemical Co., Ltd.) and 0.03 mmol/L potassium ferricyanide (manufactured by FUJIFILM Wako Pure Chemical Corp.)

(Sample)

The following sample was provided.
Purified water

(Assay procedure)

To 180 µL of the Trinder reagent-containing liquid partial composition, 4.5 µL of purified water was added, and the mixture was reacted at 37°C for 5 minutes. Then, 45 µL of the 4-aminoantipyrine- and ferrocyanide-containing liquid partial composition was added thereto, and the mixture was further reacted at 37°C for 5 minutes. Change in absorbance was measured from absorbance Abs1 5 minutes after the purified water was added to the Trinder reagent-containing liquid partial composition to absorbance Abs2 5 minutes after the 4-aminoantipyrine- and ferrocyanide-containing liquid partial composition was added to the mixed solution of the Trinder reagent-containing liquid partial composition and the purified water. The change in absorbance, $A_c$, was given according to the following expression (2).

$$A_C = (Abs2) - (Abs1) \times 180 / 225 \quad (2)$$

wherein 180 represents the liquid quantity of the Trinder reagent-containing liquid partial composition, and 225 represents the total liquid quantity of the Trinder reagent-containing liquid partial composition and the 4-aminoantipyrine- and ferrocyanide-containing liquid partial composition.

The change in absorbance when purified water was used as a sample is defined as a reagent blank Acb. The results are shown in Table 1.

[Table 1]

| 4-Aminoantipyrine- and ferrocyanide-containing liquid partial composition | | | Reagent blank (mAbs) |
|---|---|---|---|
| Stabilizer | Potassium ferrocyanide concentration (mmol/L) | Potassium ferricyanide concentration (mmol/L) | |
| None | 0.00 | 0.00 | 0.0 |
| | 0.10 | 0.00 | 0.1 |
| | 0.09 | 0.01 | 1.0 |
| | 0.07 | 0.03 | 2.4 |
| Dimethyl sulfoxide | 0.00 | 0.00 | -0.1 |
| | 0.10 | 0.00 | 1.3 |
| | 0.09 | 0.01 | 4.4 |
| | 0.07 | 0.03 | 7.3 |
| Ethanol | 0.00 | 0.00 | 0.4 |
| | 0.10 | 0.00 | 0.5 |
| | 0.09 | 0.01 | 1.1 |
| | 0.07 | 0.03 | 2.6 |
| Ethylene glycol | 0.00 | 0.00 | 0.0 |
| | 0.10 | 0.00 | 0.5 |
| | 0.09 | 0.01 | 0.4 |
| | 0.07 | 0.03 | 1.4 |
| Propylene glycol | 0.00 | 0.00 | 0.2 |
| | 0.10 | 0.00 | 0.3 |
| | 0.09 | 0.01 | 0.6 |
| | 0.07 | 0.03 | 1.7 |

As is evident from Table 1, the addition of ferricyanide tended to elevate a reagent blank. This suggested that ferricyanide contributes to the measured values of the reagent blank. In the case of adding dimethyl sulfoxide to the stabilizer, even ferrocyanide caused a reagent blank of 1 mAbs or more, suggesting the possibility that dimethyl sulfoxide converts ferrocyanide to ferricyanide that causes a reagent blank.

[Reference Example 2: Reagent blank when reagent configuration was changed]

(Preparation of Trinder reagent-containing liquid partial composition (1))

The following reagent components were dissolved in purified water so as to achieve the described concentrations. The solution was adjusted to pH 7.6 with sodium hydroxide to prepare a Trinder reagent-containing liquid partial composition (1).

100 mmol/L N-tris(hydroxymethyl)methyl-2-aminoethanesulfonic acid (manufactured by Dojindo Laboratories Co., Ltd.)
2 mmol/L N,N-bis(4-sulfobutyl)-3-methylaniline, disodium salt (manufactured by Dojindo Laboratories Co., Ltd.)

(Preparation of Trinder reagent- and ferrocyanide-containing liquid partial composition (1))

The following reagent components were dissolved in purified water so as to achieve the described concentrations. The solution was adjusted to pH 7.6 with sodium hydroxide to prepare a Trinder reagent- and ferrocyanide-containing liquid partial composition (1).

100 mmol/L N-tris(hydroxymethyl)methyl-2-aminoethanesulfonic acid (manufactured by Dojindo Laboratories Co., Ltd.)
2 mmol/L N,N-bis(4-sulfobutyl)-3-methylaniline, disodium salt (manufactured by Dojindo Laboratories Co., Ltd.)
0.025 mmol/L potassium ferrocyanide (manufactured by Kokusan Chemical Co., Ltd.)

(Preparation of 4-aminoantipyrine-containing liquid partial composition (1))

The following reagent components were dissolved in purified water so as to achieve the described concentrations. The solution was adjusted to pH 7.6 with sodium hydroxide to prepare a 4-aminoantipyrine-containing liquid partial composition (1).

100 mmol/L N-tris(hydroxymethyl)methyl-2-aminoethanesulfonic acid (manufactured by Dojindo Laboratories Co., Ltd.)
1.25 mmol/L 4-aminoantipyrine (manufactured by ACTEC Laboratories Inc.)

(Preparation of 4-aminoantipyrine- and ferrocyanide-containing liquid partial composition (1))

The following reagent components were dissolved in purified water so as to achieve the described concentrations. The solution was adjusted to pH 7.6 with sodium hydroxide to prepare a 4-aminoantipyrine- and ferrocyanide-containing liquid partial composition (1).

100 mmol/L N-tris(hydroxymethyl)methyl-2-aminoethanesulfonic acid (manufactured by Dojindo Laboratories Co., Ltd.)
1.25 mmol/L 4-aminoantipyrine (manufactured by ACTEC Laboratories Inc.)
0.025 mmol/L potassium ferrocyanide (manufactured by Kokusan Chemical Co., Ltd.)

(Preparation of 4-aminoantipyrine- and ferrocyanide-containing liquid partial composition (2))

The following reagent components were dissolved in purified water so as to achieve the described concentrations. The solution was adjusted to pH 7.5 with sodium hydroxide to prepare a 4-aminoantipyrine- and ferrocyanide-containing liquid partial composition (2).

200 mmol/L 3,3-dimethylglutaric acid (manufactured by Tokyo Chemical Industry Co., Ltd.)
5 mmol/L 4-aminoantipyrine (manufactured by ACTEC Laboratories Inc.)
0.1 mmol/L potassium ferrocyanide (manufactured by Kokusan Chemical Co., Ltd.)
Stabilizer having a concentration described later

(Stabilizer added to 4-aminoantipyrine- and ferrocyanide-containing liquid partial composition (2))

30 wt/vol% dimethyl sulfoxide (manufactured by Nacalai Tesque, Inc.)

30 wt/vol% propylene glycol (manufactured by FUJIFILM Wako Pure Chemical Corp.)

(Preparation of 4-aminoantipyrine-containing liquid partial composition (2))

The following reagent components were dissolved in purified water so as to achieve the described concentrations. The solution was adjusted to pH 7.5 with sodium hydroxide to prepare a 4-aminoantipyrine-containing liquid partial composition (2).

200 mmol/L 3,3-dimethylglutaric acid (manufactured by Tokyo Chemical Industry Co., Ltd.)
5 mmol/L 4-aminoantipyrine (manufactured by ACTEC Laboratories Inc.)
Stabilizer having a concentration described later

(Stabilizer added to 4-aminoantipyrine-containing liquid partial composition (2))

30 wt/vol% propylene glycol (manufactured by FUJIFILM Wako Pure Chemical Corp.)

(Preparation of Trinder reagent- and ferrocyanide-containing liquid partial composition (2))

The following reagent components were dissolved in purified water so as to achieve the described concentrations. The solution was adjusted to pH 7.5 with sodium hydroxide to prepare a Trinder reagent- and ferrocyanide-containing liquid partial composition (2).

200 mmol/L 3,3-dimethylglutaric acid (manufactured by Tokyo Chemical Industry Co., Ltd.)
8 mmol/L N,N-bis(4-sulfobutyl)-3-methylaniline, disodium salt (manufactured by Dojindo Laboratories Co., Ltd.)
0.1 mmol/L potassium ferrocyanide (manufactured by Kokusan Chemical Co., Ltd.)
Stabilizer having a concentration described later

(Stabilizer added to Trinder reagent- and ferrocyanide-containing liquid partial composition (2))

30 wt/vol% dimethyl sulfoxide (manufactured by Nacalai Tesque, Inc.)
30 wt/vol% propylene glycol (manufactured by FUJIFILM Wako Pure Chemical Corp.)

(Preparation of Trinder reagent-containing liquid partial composition (2))

The following reagent components were dissolved in purified water so as to achieve the described concentrations. The solution was adjusted to pH 7.5 with sodium hydroxide to prepare a Trinder reagent-containing liquid partial composition (2).

200 mmol/L 3,3-dimethylglutaric acid (manufactured by Tokyo Chemical Industry Co., Ltd.)
8 mmol/L N,N-bis(4-sulfobutyl)-3-methylaniline, disodium salt (manufactured by Dojindo Laboratories Co., Ltd.)
Stabilizer having a concentration described later

(Stabilizer added to Trinder reagent-containing liquid partial composition (2))

30 wt/vol% propylene glycol (manufactured by FUJIFILM Wako Pure Chemical Corp.)

(Sample)

The following sample was provided.
Purified water

(Assay procedure)

To 180 μL of the Trinder reagent-containing liquid partial composition (1), 4.5 μL of purified water was added, and the mixture was reacted at 37°C for 5 minutes. Then, 45 μL of the 4-aminoantipyrine- and ferrocyanide-containing liquid partial composition (2) was added thereto, and the mixture was further reacted at 37°C for 5 minutes. Change in absorbance was measured from absorbance Abs1 5 minutes after the purified water was added to the Trinder reagent-containing liquid partial composition (1) to absorbance Abs2 5 minutes after the 4-aminoantipyrine- and ferrocyanide-containing liquid partial composition (2) was added to the mixed solution of the Trinder reagent-containing liquid partial composition (1) and the purified water. The change in absorbance, $A_C$, was given according to the following expression (3).

$$A_C = (Abs2) - (Abs1) \times 180 / 225 \quad (3)$$

wherein 180 represents the liquid quantity of the Trinder reagent-containing liquid partial composition (1), and 225 represents the total liquid quantity of the Trinder reagent-containing liquid partial composition (1) and the 4-aminoantipyrine- and ferrocyanide-containing liquid partial composition (2).

To 180 μL of the Trinder reagent- and ferrocyanide-containing liquid partial composition (1), 4.5 μL of purified water was added, and the mixture was reacted at 37°C for 5 minutes. Then, 45 μL of the 4-aminoantipyrine-containing liquid partial composition (2) was added thereto, and the mixture was further reacted at 37°C for 5 minutes. Change in absorbance was measured from absorbance Abs1 5 minutes after the purified water was added to the Trinder reagent- and ferrocyanide-containing liquid partial composition (1) to absorbance Abs2 5 minutes after the 4-aminoantipyrine-containing liquid partial composition (2) was added to the mixed solution of the Trinder reagent- and ferrocyanide-containing liquid partial composition (1) and the purified water. The change in absorbance, $A_C$, was given according to the following expression (4).

$$A_C = (Abs2) - (Abs1) \times 180 / 225 \quad (4)$$

wherein 180 represents the liquid quantity of the Trinder reagent- and ferrocyanide-containing liquid partial composition (1), and 225 represents the total liquid quantity of the Trinder reagent- and ferrocyanide-containing liquid partial composition (1) and the 4-aminoantipyrine-containing liquid partial composition (2) .

To 180 μL of the 4-aminoantipyrine-containing liquid partial composition (1), 4.5 μL of purified water was added, and the mixture was reacted at 37°C for 5 minutes. Then, 45 μL of the Trinder reagent- and ferrocyanide-containing liquid partial composition (2) was added thereto, and the mixture was further reacted at 37°C for 5 minutes. Change in absorbance was measured from absorbance Abs1 5 minutes after the purified water was added to the 4-aminoantipyrine-containing liquid partial composition (1) to absorbance Abs2 5 minutes after the Trinder reagent- and ferrocyanide-containing liquid partial composition (2) was added to the mixed solution of the 4-aminoantipyrine-containing liquid partial composition (1) and the purified water. The change in absorbance, $A_C$, was given according to the following expression (5).

$$A_C = (Abs2) - (Abs1) \times 180 / 225 \quad (5)$$

wherein 180 represents the liquid quantity of the 4-aminoantipyrine-containing liquid partial composition (1), and 225 represents the total liquid quantity of the 4-aminoantipyrine-containing liquid partial composition (1) and the Trinder reagent- and ferrocyanide-containing liquid partial composition (2).

To 180 μL of the 4-aminoantipyrine- and ferrocyanide-containing liquid partial composition (1), 4.5 μL of purified water was added, and the mixture was reacted at 37°C for 5 minutes. Then, 45 μL of the Trinder reagent-containing liquid partial composition (2) was added thereto, and the mixture was further reacted at 37°C for 5 minutes. Change in absorbance was measured from absorbance Abs1 5 minutes after the purified water was added to the 4-aminoantipyrine- and ferrocyanide-containing liquid partial composition (1) to absorbance Abs2 5 minutes after the Trinder reagent-containing liquid partial composition (2) was added to the mixed solution of the 4-aminoantipyrine- and ferrocyanide-containing liquid partial composition (1) and the purified water. The change in absorbance, $A_C$, was given according to the following expression (6).

$$A_C = (Abs2) - (Abs1) \times 180 / 225 \quad (6)$$

wherein 180 represents the liquid quantity of the 4-aminoantipyrine- and ferrocyanide-containing liquid partial composition (1), and 225 represents the total liquid quantity of the 4-aminoantipyrine- and ferrocyanide-containing liquid partial composition (1) and the Trinder reagent-containing liquid partial composition (2).

To 180 μL of the 4-aminoantipyrine- and ferrocyanide-containing liquid partial composition (2), 4.5 μL of purified water was added, and the mixture was reacted at 37°C for 5 minutes. Then, 45 μL of the Trinder reagent-containing liquid partial composition (1) was added thereto, and the mixture was further reacted at 37°C for 5 minutes. Change in absorbance was measured from absorbance Abs1 5 minutes after the purified water was added to the 4-aminoantipyrine- and ferrocyanide-containing liquid partial composition (2) to absorbance Abs2 5 minutes after the Trinder reagent-containing liquid partial composition (1) was added to the mixed solution of the 4-aminoantipyrine- and ferrocyanide-containing liquid partial composition (2) and the purified water. The change in absorbance, $A_C$, was given according to the following expression (7).

$$A_C = (Abs2) - (Abs1) \times 180 / 225 \quad (7)$$

wherein 180 represents the liquid quantity of the 4-aminoantipyrine- and ferrocyanide-containing liquid partial composition (2), and 225 represents the total liquid quantity of the 4-aminoantipyrine- and ferrocyanide-containing liquid partial composition (2) and the Trinder reagent-containing liquid partial composition (1).

To 180 μL of the Trinder reagent- and ferrocyanide-containing liquid partial composition (2), 4.5 μL of purified water was added, and the mixture was reacted at 37°C for 5 minutes. Then, 45 μL of the 4-aminoantipyrine-containing liquid partial composition (1) was added thereto, and the mixture was further reacted at 37°C for 5 minutes. Change in absorbance was measured from absorbance Abs1 5 minutes after the purified water was added to the Trinder reagent- and ferrocyanide-containing liquid partial composition (2) to absorbance Abs2 5 minutes after the 4-aminoantipyrine-containing liquid partial composition (1) was added to the mixed solution of the Trinder reagent- and ferrocyanide-containing liquid partial composition (2) and the purified water. The change in absorbance, $A_C$, was given according to the following expression (8).

$$A_C = (Abs2) - (Abs1) \times 180 / 225 \quad (8)$$

wherein 180 represents the liquid quantity of the Trinder reagent- and ferrocyanide-containing liquid partial composition (2), and 225 represents the total liquid quantity of the Trinder reagent- and ferrocyanide-containing liquid partial composition (2) and the 4-aminoantipyrine-containing liquid partial composition (1).

The change in absorbance when purified water was used as a sample is defined as a reagent blank Acb. The results are shown in Table 2.

[Table 2]

| Stabilizer | Composition reacted first with sample | Composition reacted second with sample | Reagent blank (mAbs) |
|---|---|---|---|
| Dimethyl sulfoxide | Trinder reagent-containing liquid partial composition (1) | 4-Aminoantipyrine- and ferrocyanide-containing liquid partial composition (2) | 4.4 |
| | 4-Aminoantipyrine-containing liquid partial composition (1) | Trinder reagent- and ferrocyanide-containing liquid partial composition (2) | 4.8 |
| | 4-Aminoantipyrine- and ferrocyanide-containing liquid partial composition (2) | Trinder reagent-containing liquid partial composition (1) | 17.4 |
| | Trinder reagent- and ferrocyanide-containing liquid partial composition (2) | 4-Aminoantipyrine-containing liquid partial composition (1) | 9.7 |
| Propylene glycol | Trinder reagent-containing liquid partial composition (1) | 4-Aminoantipyrine- and ferrocyanide-containing liquid partial composition (2) | 0.3 |
| | Trinder reagent- and ferrocyanide-containing liquid partial composition (1) | 4-Aminoantipyrine-containing liquid partial composition (2) | 0.3 |
| | 4-Aminoantipyrine-containing liquid partial composition (1) | Trinder reagent- and ferrocyanide-containing liquid partial composition (2) | 0.5 |
| | 4-Aminoantipyrine- and ferrocyanide-containing liquid partial composition (1) | Trinder reagent-containing liquid partial composition (2) | 0.4 |
| | 4-Aminoantipyrine- and ferrocyanide-containing liquid partial composition (2) | Trinder reagent-containing liquid partial composition (1) | 1.9 |
| | Trinder reagent- and ferrocyanide-containing liquid partial composition (2) | 4-Aminoantipyrine-containing liquid partial composition (1) | 2.8 |

As shown in Table 2, both when the liquid partial composition reacted first with the sample contained a Trinder reagent and when the liquid partial composition reacted first with the sample contained 4-aminoantipyrine, use of dimethyl sulfoxide as the stabilizer increased the reagent blank as compared with use of propylene glycol as the stabilizer. It was also found that the reagent blank-suppressing effect does not largely depend on the order of addition of the reagents or how to combine the reagents with ferrocyanide.

[Example 1: Oxidation-reduction potential measurement of ferrocyanide in presence of stabilizer]

(Preparation of ferrocyanide-containing liquid composition)

The following reagent components were dissolved in purified water so as to achieve the described concentrations. The solution was adjusted to pH 7.5 with sodium hydroxide to prepare a ferrocyanide-containing liquid composition.

100 mmol/L N-tris(hydroxymethyl)methyl-2-aminoethanesulfonic acid (manufactured by Dojindo Laboratories Co., Ltd.)
1 mmol/L potassium ferrocyanide (manufactured by Kokusan Chemical Co., Ltd.)
150 mmol/L sodium chloride (manufactured by FUJIFILM Wako Pure Chemical Corp.)
Stabilizer having a concentration described later

(Stabilizer added to ferrocyanide-containing liquid composition)

6 wt/vol% propylene glycol (manufactured by FUJIFILM Wako Pure Chemical Corp.)
7.5 wt/vol% propylene glycol (manufactured by FUJIFILM Wako Pure Chemical Corp.)
10 wt/vol% propylene glycol (manufactured by FUJIFILM Wako Pure Chemical Corp.)
30 wt/vol% propylene glycol (manufactured by FUJIFILM Wako Pure Chemical Corp.)
37.5 wt/vol% propylene glycol (manufactured by FUJIFILM Wako Pure Chemical Corp.)
40 wt/vol% propylene glycol (manufactured by FUJIFILM Wako Pure Chemical Corp.)
50 wt/vol% propylene glycol (manufactured by FUJIFILM Wako Pure Chemical Corp.)
62.5 wt/vol% propylene glycol (manufactured by FUJIFILM Wako Pure Chemical Corp.)
30 wt/vol% trimethylene glycol (manufactured by FUJIFILM Wako Pure Chemical Corp.)
37.5 wt/vol% trimethylene glycol (manufactured by FUJIFILM Wako Pure Chemical Corp.)
50 wt/vol% trimethylene glycol (manufactured by FUJIFILM Wako Pure Chemical Corp.)
62.5 wt/vol% trimethylene glycol (manufactured by FUJIFILM Wako Pure Chemical Corp.)
30 wt/vol% ethylene glycol (manufactured by FUJIFILM Wako Pure Chemical Corp.)
37.5 wt/vol% ethylene glycol (manufactured by FUJIFILM Wako Pure Chemical Corp.)
50 wt/vol% ethylene glycol (manufactured by FUJIFILM Wako Pure Chemical Corp.)
62.5 wt/vol% ethylene glycol (manufactured by FUJIFILM Wako Pure Chemical Corp.)
37.5 wt/vol% ethanol (manufactured by FUJIFILM Wako Pure Chemical Corp.)
37.5 wt/vol% 1-propanol (manufactured by FUJIFILM Wako Pure Chemical Corp.)
37.5 wt/vol% glycerin (manufactured by FUJIFILM Wako Pure Chemical Corp.)
0.6 wt/vol% 2-carboxyphenylboronic acid (manufactured by Tokyo Chemical Industry Co., Ltd.)
0.75 wt/vol% 2-carboxyphenylboronic acid (manufactured by Tokyo Chemical Industry Co., Ltd.)
1 wt/vol% 2-carboxyphenylboronic acid (manufactured by Tokyo Chemical Industry Co., Ltd.)
1 wt/vol% lauryl trimethylammonium chloride (manufactured by FUJIFILM Wako Pure Chemical Corp.)
1 wt/vol% benzyl triethylammonium chloride (manufactured by FUJIFILM Wako Pure Chemical Corp.)
1 wt/vol% benzyl tributylammonium chloride (manufactured by FUJIFILM Wako Pure Chemical Corp.)
1 wt/vol% calcium chloride (manufactured by FUJIFILM Wako Pure Chemical Corp.)
1 wt/vol% boric acid (manufactured by FUJIFILM Wako Pure Chemical Corp.)
150 mmol/L sodium chloride (manufactured by FUJIFILM Wako Pure Chemical Corp.) (a total of 300 mmol/L sodium chloride combined with the 150 mmol/L sodium chloride described above)
30 wt/vol% dimethyl sulfoxide (manufactured by Nacalai Tesque, Inc.)
37.5 wt/vol% dimethyl sulfoxide (manufactured by Nacalai Tesque, Inc.)
50 wt/vol% dimethyl sulfoxide (manufactured by Nacalai Tesque, Inc.)
62.5 wt/vol% dimethyl sulfoxide (manufactured by Nacalai Tesque, Inc.)

(Assay procedure)

To a cell for voltammetry (BAS Inc.), 10 mL of the ferrocyanide-containing liquid composition was added, and cyclic voltammetry was performed using the following electrodes and apparatus.

Working electrode: GCE glass carbon electrode, 6 mm in outside diameter $\times$ 3 mm in inside diameter (BAS Inc.)
Counter electrode: Pt counter electrode 23 cm (coil-like), 23 cm in length $\times$ 0.5 mm in electrode diameter (BAS Inc.)
Reference electrode: RE-1B aqueous reference electrode (Ag/AgCl) (BAS Inc.)
Apparatus: AUTOMATIC POLARIZATION SYSTEM HZ-7000 (Hokuto Denko Corp.)

For cyclic voltammetry measurement, potentials applied to the working electrode were changed against time. The sweep rate was set to 0.1 V/sec. The initial potential was regarded as a spontaneous potential, and the potentials were swept to the positive side until the first set potential. Subsequently, the sweep of the electrode potentials was reversed to the negative side from the first set potential to the second set potential. Further, the sweep of the electrode potentials was reversed to the positive side from the second set potential to the first set potential. The first set potential and the second set potential were 0.5 V and - 0.5 V, respectively. The cyclic voltammetry measurement was carried out at 25°C.

The oxidation-reduction potential (E'0) was calculated as a mean [(Ered + Eox) / 2] of a potential value (Eox) when oxidation current exhibited a peak and a potential value (Ered) when reduction current exhibited a peak. The measurement results are shown in Table 3.

[Table 3-1]

| Stabilizer | Potential at oxidation current peak (V) | Potential at reduction current peak (V) | Oxidation-reduction potential (V) |
|---|---|---|---|
| None | 0.260 | 0.157 | 0.208 |
| 6 wt/vol% propylene glycol | 0.256 | 0.142 | 0.199 |
| 7.5 wt/vol% propylene glycol | 0.257 | 0.138 | 0.197 |
| 10 wt/vol% propylene glycol | 0.241 | 0.141 | 0.191 |
| 30 wt/vol% propylene glycol | 0.201 | 0.109 | 0.155 |
| 37.5 wt/vol% propylene glycol | 0.202 | 0.085 | 0.143 |
| 40 wt/vol% propylene glycol | 0.174 | 0.085 | 0.129 |
| 50 wt/vol% propylene glycol | 0.165 | 0.085 | 0.125 |
| 62.5 wt/vol% propylene glycol | 0.133 | 0.055 | 0.094 |
| 30 wt/vol% trimethylene glycol | 0.200 | 0.095 | 0.147 |
| 37.5 wt/vol% trimethylene glycol | 0.172 | 0.098 | 0.135 |
| 50 wt/vol% trimethylene glycol | 0.151 | 0.073 | 0.112 |
| 62.5 wt/vol% trimethylene glycol | 0.122 | 0.047 | 0.084 |
| 30 wt/vol% ethylene glycol | 0.202 | 0.110 | 0.156 |
| 37.5 wt/vol% ethylene glycol | 0.183 | 0.101 | 0.142 |
| 50 wt/vol% ethylene glycol | 0.164 | 0.075 | 0.119 |
| 62.5 wt/vol% ethylene glycol | 0.139 | 0.060 | 0.099 |
| 37.5 wt/vol% ethanol | 0.165 | 0.087 | 0.126 |

[Table 3-2]

| 37.5 wt/vol% 1-propanol | 0.215 | 0.119 | 0.167 |
|---|---|---|---|
| 37.5 wt/vol% glycerin | 0.214 | 0.143 | 0.178 |
| 0.6 wt/vol% 2-carboxyphenylboronic acid | 0.261 | 0.150 | 0.206 |
| 0.75 wt/vol% 2-carboxyphenylboronic acid | 0.268 | 0.117 | 0.209 |
| 1 wt/vol% 2-carboxyphenylboronic acid | 0.301 | 0.148 | 0.208 |
| 1 wt/vol% lauryl trimethylammonium chloride | 0.148 | 0.087 | 0.118 |
| 1 wt/vol% benzyl triethylammonium chloride | 0.239 | 0.157 | 0.198 |

(continued)

| | | | |
|---|---|---|---|
| 1 wt/vol% benzyl tributylammonium chloride | 0.246 | 0.154 | 0.200 |
| 1 wt/vol% calcium chloride | 0.271 | 0.207 | 0.239 |
| 1 wt/vol% boric acid | 0.225 | 0.158 | 0.205 |
| 300 mmol/L sodium chloride | 0.250 | 0.188 | 0.219 |
| 30 wt/vol% dimethyl sulfoxide | 0.127 | -0.012 | 0.058 |
| 37.5 wt/vol% dimethyl sulfoxide | 0.062 | -0.035 | 0.013 |
| 50 wt/vol% dimethyl sulfoxide | -0.022 | -0.119 | -0.071 |
| 62.5 wt/vol% dimethyl sulfoxide | -0.126 | -0.397 | -0.262 |

As is evident from Table 3, it was revealed that the oxidation-reduction potential of ferrocyanide varies depending on the type or concentration of the stabilizer; and dimethyl sulfoxide, propylene glycol, trimethylene glycol and ethylene glycol decrease the oxidation-reduction potential in a concentration-dependent manner. On the other hand, 2-carboxyphenylboronic acid did not substantially change the oxidation-reduction potential. It was revealed that, particularly, dimethyl sulfoxide markedly decreases the oxidation-reduction potential as compared with propylene glycol, trimethylene glycol and ethylene glycol. This suggested that ferrocyanide mixed with a specific stabilizer easily generates ferricyanide since decreased oxidation-reduction potentials generally facilitate oxidation.

[Example 2: Change in reagent blank and specimen sensitivity stability depending on type of protease stabilizer]

(Preparation of Trinder reagent-containing liquid partial composition)

The following reagent components were dissolved in purified water so as to achieve the described concentrations. The solution was adjusted to pH 7.6 with sodium hydroxide to prepare a Trinder reagent-containing liquid partial composition.

        100 mmol/L N-tris(hydroxymethyl)methyl-2-aminoethanesulfonic acid (manufactured by Dojindo Laboratories Co., Ltd.)
        0.05 wt/vol% sodium azide (manufactured by Merck KGaA)
        1 mmol/L sodium ethylenediaminetetraacetate (manufactured by Kokusan Chemical Co., Ltd.)
        6 wt/vol% D(-)-sorbitol (manufactured by FUJIFILM Wako Pure Chemical Corp.)
        2 mmol/L N,N-bis(4-sulfobutyl)-3-methylaniline, disodium salt (manufactured by Dojindo Laboratories Co., Ltd.)
        10 U/mL peroxidase (manufactured by Amano Enzyme Inc.)
        30 U/mL ketoamine oxidase (manufactured by Asahi Kasei Pharma Corp.)

(Two-reagent system: Preparation of 4-aminoantipyrine- and ferrocyanide-containing liquid partial composition)

The following reagent components were dissolved in purified water so as to achieve the described concentrations. The solution was adjusted to pH 7.5 with sodium hydroxide to prepare a 4-aminoantipyrine- and ferrocyanide-containing liquid partial composition containing a stabilizer.

        200 mmol/L 3,3-dimethylglutaric acid (manufactured by Tokyo Chemical Industry Co., Ltd.)
        0.05 wt/vol% ProClin 300 (manufactured by Sigma-Aldrich Co., LLC)
        5 mmol/L 4-aminoantipyrine (manufactured by ACTEC Laboratories Inc.)
        0.1 mmol/L potassium ferrocyanide (manufactured by Kokusan Chemical Co., Ltd.)
        500 U/mL catalase (manufactured by Nagase ChemteX Corp.)
        40 kU/mL protease for glycated albumin (manufactured by Asahi Kasei Pharma Corp.; obtained in accordance with the method described in Japanese Patent No. 4565807)
        Stabilizer having a concentration described later

(Two-reagent system: Stabilizer contained in 4-aminoantipyrine- and ferrocyanide-containing liquid partial composition)

30 wt/vol% propylene glycol (manufactured by FUJIFILM Wako Pure Chemical Corp.)

37.5 wt/vol% propylene glycol (manufactured by FUJIFILM Wako Pure Chemical Corp.)

50 wt/vol% trimethylene glycol (manufactured by FUJIFILM Wako Pure Chemical Corp.)

62.5 wt/vol% trimethylene glycol (manufactured by FUJIFILM Wako Pure Chemical Corp.)

50 wt/vol% ethylene glycol (manufactured by FUJIFILM Wako Pure Chemical Corp.)

62.5 wt/vol% ethylene glycol (manufactured by FUJIFILM Wako Pure Chemical Corp.)

0.6 wt/vol% 2-carboxyphenylboronic acid (manufactured by Tokyo Chemical Industry Co., Ltd.)

0.75 wt/vol% 2-carboxyphenylboronic acid (manufactured by Tokyo Chemical Industry Co., Ltd.)

30 wt/vol% dimethyl sulfoxide (manufactured by Nacalai Tesque, Inc.)

37.5 wt/vol% dimethyl sulfoxide (manufactured by Nacalai Tesque, Inc.)

(Three-reagent system: Preparation of 4-aminoantipyrine-containing liquid partial composition (1))

The following reagent components were dissolved in purified water so as to achieve the described concentrations. The solution was adjusted to pH 7.5 with sodium hydroxide to prepare a 4-aminoantipyrine-containing liquid partial composition (1) containing a stabilizer.

250 mmol/L 3,3-dimethylglutaric acid (manufactured by Tokyo Chemical Industry Co., Ltd.)

0.05 wt/vol% ProClin 300 (manufactured by Sigma-Aldrich Co., LLC)

6.25 mmol/L 4-aminoantipyrine (manufactured by ACTEC Laboratories Inc.)

625 U/mL catalase (manufactured by Nagase ChemteX Corp.)

50 kU/mL protease for glycated albumin (manufactured by Asahi Kasei Pharma Corp.; obtained in accordance with the method described in Japanese Patent No. 4565807)

Stabilizer having a concentration described later

(Three-reagent system: Stabilizer contained in 4-aminoantipyrine-containing liquid partial composition (1))

37.5 wt/vol% propylene glycol (manufactured by FUJIFILM Wako Pure Chemical Corp.)

50 wt/vol% propylene glycol (manufactured by FUJIFILM Wako Pure Chemical Corp.)

62.5 wt/vol% trimethylene glycol (manufactured by FUJIFILM Wako Pure Chemical Corp.)

62.5 wt/vol% ethylene glycol (manufactured by FUJIFILM Wako Pure Chemical Corp.)

0.75 wt/vol% 2-carboxyphenylboronic acid (manufactured by Tokyo Chemical Industry Co., Ltd.)

37.5 wt/vol% dimethyl sulfoxide (manufactured by Nacalai Tesque, Inc.)

(Three-reagent system: Preparation of ferrocyanide-containing liquid partial composition (1))

The following reagent components were dissolved in purified water so as to achieve the described concentrations. The solution was adjusted to pH 7.0 with sodium hydroxide to prepare a ferrocyanide-containing liquid partial composition (1).

100 mmol/L 3,3-dimethylglutaric acid (manufactured by Tokyo Chemical Industry Co., Ltd.)

0.05 wt/vol% ProClin 300 (manufactured by Sigma-Aldrich Co., LLC)

0.5 mmol/L potassium ferrocyanide (manufactured by Kokusan Chemical Co., Ltd.)

(Three-reagent system: Preparation of 4-aminoantipyrine-containing liquid partial composition (2))

The following reagent components were dissolved in purified water so as to achieve the described concentrations. The solution was adjusted to pH 7.0 with sodium hydroxide to prepare a 4-aminoantipyrine-containing liquid partial composition (2).

100 mmol/L 3,3-dimethylglutaric acid (manufactured by Tokyo Chemical Industry Co., Ltd.)

0.05 wt/vol% ProClin 300 (manufactured by Sigma-Aldrich Co., LLC)

25 mmol/L 4-aminoantipyrine (manufactured by ACTEC Laboratories Inc.)

(Three-reagent system: Preparation of ferrocyanide-containing liquid partial composition (2))

The following reagent components were dissolved in purified water so as to achieve the described concentrations. The solution was adjusted to pH 7.5 with sodium hydroxide to prepare a ferrocyanide-containing liquid partial composition (2) containing a stabilizer.

250 mmol/L 3,3-dimethylglutaric acid (manufactured by Tokyo Chemical Industry Co., Ltd.)
0.05 wt/vol% ProClin 300 (manufactured by Sigma-Aldrich Co., LLC)
0.125 mmol/L potassium ferrocyanide (manufactured by Kokusan Chemical Co., Ltd.)
625 U/mL catalase (manufactured by Nagase ChemteX Corp.)
50 kU/mL protease for glycated albumin (manufactured by Asahi Kasei Pharma Corp.; obtained in accordance with the method described in Japanese Patent No. 4565807)
Stabilizer having a concentration described later

(Three-reagent system: Stabilizer contained in ferrocyanide-containing liquid partial composition (2))

37.5 wt/vol% propylene glycol (manufactured by FUJIFILM Wako Pure Chemical Corp.)
37.5 wt/vol% dimethyl sulfoxide (manufactured by Nacalai Tesque, Inc.)

(Sample)

The following samples were provided.

Control serum L (manufactured by Asahi Kasei Pharma Corp.)
Control serum H (manufactured by Asahi Kasei Pharma Corp.)

(Reagent preservation)

The prepared Trinder reagent-containing liquid partial composition was preserved at 5°C for 14 days or 28 days. Also, the 4-aminoantipyrine- and ferrocyanide-containing partial composition, the 4-aminoantipyrine-containing liquid partial composition, and the ferrocyanide-containing liquid partial composition were preserved at 30°C for 14 days or 28 days.

(Assay preparation)

The 4-aminoantipyrine-containing liquid partial composition (1) and the ferrocyanide-containing liquid partial composition (1) of the three-reagent system were mixed at a volume ratio of 4:1 to prepare a 4-aminoantipyrine- and ferrocyanide-containing liquid partial composition (1) for use in assay.
The 4-aminoantipyrine-containing liquid partial composition (2) and the ferrocyanide-containing liquid partial composition (2) of the three-reagent system were mixed at a volume ratio of 1:4 to prepare a 4-aminoantipyrine- and ferrocyanide-containing liquid partial composition (2) for use in assay.

(Assay procedure)

To 180 $\mu$L of the Trinder reagent-containing liquid partial composition, 4.5 $\mu$L of the sample was added, and the mixture was reacted at 37°C for 5 minutes. Then, 45 $\mu$L of the 4-aminoantipyrine- and ferrocyanide-containing liquid partial composition was added thereto, and the mixture was further reacted at 37°C for 5 minutes. Change in absorbance was measured from absorbance Abs1 5 minutes after the sample was added to the Trinder reagent-containing liquid partial composition to absorbance Abs2 5 minutes after the 4-aminoantipyrine- and ferrocyanide-containing liquid partial composition was added to the mixed solution of the Trinder reagent-containing liquid partial composition and the sample. The change in absorbance, $A_C$, was given according to the following expression (9).

$$A_C = (Abs2) - (Abs1) \times 180 / 225 \quad (9)$$

wherein 180 represents the liquid quantity of the Trinder reagent-containing liquid partial composition, and 225 represents the total liquid quantity of the Trinder reagent-containing liquid partial composition and the 4-aminoantipyrine- and ferrocyanide-containing liquid partial composition.

Change in absorbance measured using the sample was defined as Acs, and change in absorbance measured using purified water instead of the sample was defined as a reagent blank Acb. A value obtained by subtracting the change in absorbance, Acb, of the reagent blank from the change in absorbance, Acs, measured using the sample was calculated as sensitivity ΔAbs according to the following expression (10).

$$\Delta Abs = Acs - Acb \quad (10)$$

The sensitivity of the partial composition preserved for 14 days was defined as ΔAbs14, and the sensitivity of the partial composition preserved for 28 days was defined as ΔAbs28. The sensitivity preservation rate was calculated as the ratio of ΔAbs28 to ΔAbs14 according to the following expression (11). The sensitivity preservation rate is shown in Table 4, together with the results about the reagent blank Acb of the partial composition preserved for 14 days.

Sensitivity preservation rate (%) = ΔAbs28 / ΔAbs14 × 100 (11)                    (11)

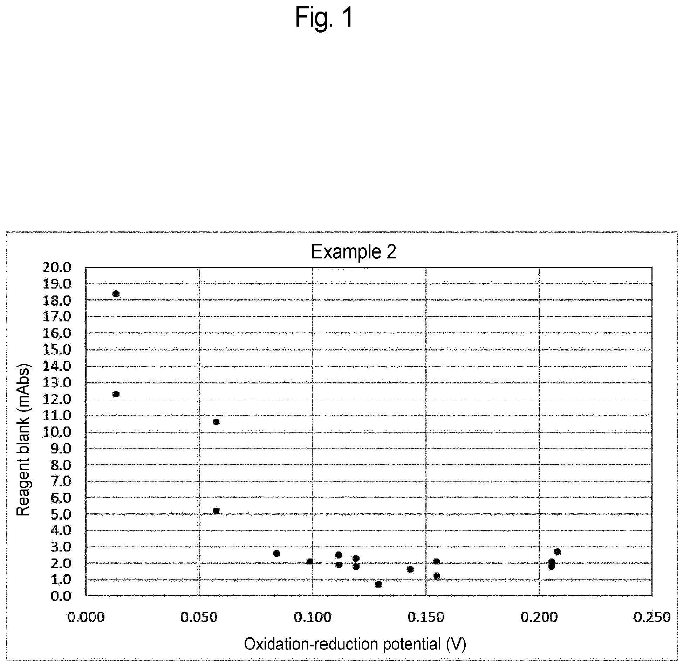

Table 5 and Figure 1 show the relationship between the reagent blank and the oxidation-reduction potential of ferrocyanide after preservation for 14 days of the partial composition. The oxidation-reduction potential value at the concentration when the stabilizer and the ferrocyanide were mixed was used as the oxidation-reduction potential of ferrocyanid. For example, when the stabilizer and the ferrocyanide were contained in the same partial composition (the 4-aminoantipyrine- and ferrocyanide-containing liquid partial composition of the two-reagent system, and the ferrocyanide-containing liquid partial composition (2) of the three-reagent system), the oxidation-reduction potential value at the stabilizer concentration in the partial composition was used. When the stabilizer and the ferrocyanide were contained in separate partial compositions (the 4-aminoantipyrine-containing liquid partial composition (1) and the ferrocyanide-containing liquid partial composition (1) of the three-reagent system), the oxidation-reduction potential value at a stabilizer concentration in a mixture of the 4-aminoantipyrine-containing liquid partial composition (1) and the ferrocyanide-containing liquid partial composition (1) was used.

[Table 4-1]

| Reagent type | 4-Aminoantipyrine- and ferrocyanide-containing liquid partial composition | Reagent blank after 14-day preservation (mAbs) | Sample | Sensitivity preservation rate (%) |
|---|---|---|---|---|
| Two-reagent system | 30 wt/vol% propylene glycol | 2.1 | Control serum L | 87.1 |
| | | | Control serum H | 85.1 |
| Two-reagent system | 37.5 wt/vol% propylene glycol | 1.6 | Control serum L | 90.9 |
| | | | Control serum H | 90.4 |
| Three-reagent system | 4-Aminoantipyrine-containing liquid partial composition (1) 37.5 wt/vol% propylene glycol | 1.2 | Control serum L | 96.5 |
| | Ferrocyanide-containing liquid partial composition (1) | | Control serum H | 95.5 |
| Three-reagent system | 4-Aminoantipyrine-containing liquid partial composition (1) 50 wt/vol% propylene glycol | 0.7 | Control serum L | 100.9 |
| | Ferrocyanide-containing liquid partial composition (1) | | Control serum H | 99.1 |

[Table 4-2]

| Three-reagent system | 4-Aminoantipyrine-containing liquid partial composition (2) | 1.6 | Control serum L | 95.3 |
| --- | --- | --- | --- | --- |
| | Ferrocyanide-containing liquid partial composition (2) 37.5 wt/vol% propylene glycol | | Control serum H | 94.7 |
| Two-reagent system | 50 wt/vol% trimethylene glycol | 1.9 | Control serum L | 91.1 |
| | | | Control serum H | 92.1 |
| Two-reagent system | 62.5 wt/vol% trimethylene glycol | 2.6 | Control serum L | 92.7 |
| | | | Control serum H | 92.9 |
| Three-reagent system | 4-Aminoantipyrine-containing liquid partial composition (1) 62.5 wt/vol% trimethylene glycol | 2.5 | Control serum L | 96.5 |
| | Ferrocyanide-containing liquid partial composition (1) | | Control serum H | 95.8 |

[Table 4-3]

| Two-reagent system | 50 wt/vol% ethylene glycol | 2.3 | Control serum L | 87.7 |
| --- | --- | --- | --- | --- |
| | | | Control serum H | 87.6 |
| Two-reagent system | 62.5 wt/vol% ethylene glycol | 2.1 | Control serum L | 88.0 |
| | | | Control serum H | 89.6 |
| Three-reagent system | 4-Aminoantipyrine-containing liquid partial composition (1) 62.5 wt/vol% ethylene glycol | 1.8 | Control serum L | 92.4 |
| | Ferrocyanide-containing liquid partial composition (1) | | Control serum H | 91.3 |
| Two-reagent system | 0.6 wt/vol% 2-carboxyphenylboronic acid | 2.1 | Control serum L | 91.1 |
| | | | Control serum H | 90.5 |
| Two-reagent system | 0.75 wt/vol% 2-carboxyphenylboronic acid | 2.7 | Control serum L | 91.9 |
| | | | Control serum H | 90.6 |

[Table 4-4]

| Three-reagent system | 4-Aminoantipyrine-containing liquid partial composition (1) 0.75 wt/vol% 2-carboxyphenylboronic acid | 1.8 | Control serum L | 96.1 |
| --- | --- | --- | --- | --- |
| | Ferrocyanide-containing liquid partial composition (1) | | Control serum H | 95.1 |
| Two-reagent system | 30 wt/vol% dimethyl sulfoxide | 5.2 | Control serum L | 87.4 |
| | | | Control serum H | 86.7 |
| Two-reagent system | 37.5 wt/vol% dimethyl sulfoxide | 12.3 | Control serum L | 91.2 |
| | | | Control serum H | 90.8 |
| Three-reagent system | 4-Aminoantipyrine-containing liquid partial composition (1) 37.5 wt/vol% dimethyl sulfoxide | 10.6 | Control serum L | 98.5 |
| | Ferrocyanide-containing liquid partial composition (1) | | Control serum H | 98.3 |
| Three-reagent system | 4-Aminoantipyrine-containing liquid partial composition (2) | 18.4 | Control serum L | 96.9 |
| | Ferrocyanide-containing liquid partial composition (2) 37.5 wt/vol% dimethyl sulfoxide | | Control serum H | 96.6 |

[Table 5-1]

| Reagent type | 4-Aminoantipyrine- and ferrocyanide-containing liquid partial composition | Reagent blank after 14-d preservation (mAbs) | Oxidation-reduction potential (V) |
|---|---|---|---|
| Two-reagent system | 30 wt/vol% propylene glycol | 2.1 | 0.155 |
| Two-reagent system | 37.5 wt/vol% propylene glycol | 1.6 | 0.143 |
| Three-reagent system | 4-Aminoantipyrine-containing liquid partial composition (1) 37.5 wt/vol% propylene glycol | 1.2 | 0.155 |
| | Ferrocyanide-containing liquid partial composition (1) | | |
| Three-reagent system | 4-Aminoantipyrine-containing liquid partial composition (1) 50 wt/vol% propylene glycol | 0.7 | 0.129 |
| | Ferrocyanide-containing liquid partial composition (1) | | |
| Three-reagent system | 4-Aminoantipyrine-containing liquid partial composition (2) | 1.6 | 0.143 |
| | Ferrocyanide-containing liquid partial composition (2) 37.5 wt/vol% propylene glycol | | |
| Two-reagent system | 50 wt/vol% trimethylene glycol | 1.9 | 0.112 |
| Two-reagent system | 62.5 wt/vol% trimethylene glycol | 2.6 | 0.084 |

[Table 5-2]

| | | | |
|---|---|---|---|
| Three-reagent system | 4-Aminoantipyrine-containing liquid partial composition (1) 62.5 wt/vol% trimethylene glycol | 2.5 | 0.112 |
| | Ferrocyanide-containing liquid partial composition (1) | | |
| Two-reagent system | 50 wt/vol% ethylene glycol | 2.3 | 0.119 |
| Two-reagent system | 62.5 wt/vol% ethylene glycol | 2.1 | 0.099 |
| Three-reagent system | 4-Aminoantipyrine-containing liquid partial composition (1) 62.5 wt/vol% ethylene glycol | 1.8 | 0.119 |
| | Ferrocyanide-containing liquid partial composition (1) | | |
| Two-reagent system | 0.6 wt/vol% 2-carboxyphenylboronic acid | 2.1 | 0.206 |
| Two-reagent system | 0.75 wt/vol% 2-carboxyphenylboronic acid | 2.7 | 0.209 |
| Three-reagent system | 4-Aminoantipyrine-containing liquid partial composition (1) 0.75 wt/vol% 2-carboxyphenylboronic acid | 1.8 | 0.206 |
| | Ferrocyanide-containing liquid partial composition (1) | | |

[Table 5-3]

| | | | |
|---|---|---|---|
| Two-reagent system | 30 wt/vol% dimethyl sulfoxide | 5.2 | 0.058 |
| Two-reagent system | 37.5 wt/vol% dimethyl sulfoxide | 12.3 | 0.013 |
| Three-reagent system | 4-Aminoantipyrine-containing liquid partial composition (1) 37.5 wt/vol% dimethyl sulfoxide | 10.6 | 0.058 |
| | Ferrocyanide-containing liquid partial composition (1) | | |
| Three-reagent system | 4-Aminoantipyrine-containing liquid partial composition (2) | 18.4 | 0.013 |
| | Ferrocyanide-containing liquid partial composition (2) 37.5 wt/vol% dimethyl sulfoxide | | |

As is evident from Table 4, Table 5, and Figure 1, it was found that both in the two-reagent system and in the three-reagent system, the reagent blank-suppressing effect is pronounced for a stabilizer that gives an oxidation-reduction potential higher than the oxidation-reduction potential of ferrocyanide (0.058 V) upon mixing the stabilizer with the ferrocyanide. It was also found that for all the stabilizers, the change of the two-reagent system to the three-reagent system improves a specimen sensitivity preservation rate. It was further found that a higher stabilizer concentration produces a higher stabilizing effect on the specimen sensitivity.

[Example 3: Effect of chelating agent addition]

(Preparation of Trinder reagent-containing liquid partial composition)

The following reagent components were dissolved in purified water so as to achieve the described concentrations. The solution was adjusted to pH 7.6 with sodium hydroxide to prepare a Trinder reagent-containing liquid partial composition.

    100 mmol/L N-tris(hydroxymethyl)methyl-2-aminoethanesulfonic acid (manufactured by Dojindo Laboratories Co., Ltd.)
    0.05 wt/vol% sodium azide (manufactured by Merck KGaA)
    1 mmol/L sodium ethylenediaminetetraacetate (manufactured by Kokusan Chemical Co., Ltd.)
    6 wt/vol% D(-)-sorbitol (manufactured by FUJIFILM Wako Pure Chemical Corp.)
    2 mmol/L N,N-bis(4-sulfobutyl)-3-methylaniline, disodium salt (manufactured by Dojindo Laboratories Co., Ltd.)
    10 U/mL peroxidase (manufactured by Amano Enzyme Inc.)
    30 U/mL ketoamine oxidase (manufactured by Asahi Kasei Pharma Corp.)

(Two-reagent system: Preparation of 4-aminoantipyrine- and ferrocyanide-containing liquid partial composition)

The following reagent components were dissolved in purified water so as to achieve the described concentrations. The solution was adjusted to pH 7.5 with sodium hydroxide to prepare a 4-aminoantipyrine- and ferrocyanide-containing liquid partial composition containing propylene glycol as a stabilizer, the partial composition containing trisodium citrate or containing no trisodium citrate.

    200 mmol/L 3,3-dimethylglutaric acid (manufactured by Tokyo Chemical Industry Co., Ltd.)
    0.05 wt/vol% ProClin 300 (manufactured by Sigma-Aldrich Co., LLC)
    5 mmol/L 4-aminoantipyrine (manufactured by ACTEC Laboratories Inc.)
    0.1 mmol/L potassium ferrocyanide (manufactured by Kokusan Chemical Co., Ltd.)
    500 U/mL catalase (manufactured by Nagase ChemteX Corp.)
    40 kU/mL protease for glycated albumin (manufactured by Asahi Kasei Pharma Corp.; obtained in accordance with the method described in Japanese Patent No. 4565807)
    1 mmol/L trisodium citrate (manufactured by FUJIFILM Wako Pure Chemical Corp.)
    Stabilizer having a concentration described later

(Two-reagent system: Stabilizer contained in 4-aminoantipyrine- and ferrocyanide-containing liquid partial composition)

    30 wt/vol% propylene glycol (manufactured by FUJIFILM Wako Pure Chemical Corp.)
    37.5 wt/vol% propylene glycol (manufactured by FUJIFILM Wako Pure Chemical Corp.)

(Three-reagent system: Preparation of 4-aminoantipyrine-containing liquid partial composition (1))

The following reagent components were dissolved in purified water so as to achieve the described concentrations. The solution was adjusted to pH 7.5 with sodium hydroxide to prepare a 4-aminoantipyrine-containing liquid partial composition (1) containing a stabilizer, the partial composition containing trisodium citrate or containing no trisodium citrate.

    250 mmol/L 3,3-dimethylglutaric acid (manufactured by Tokyo Chemical Industry Co., Ltd.)
    0.05 wt/vol% ProClin 300 (manufactured by Sigma-Aldrich Co., LLC)

6.25 mmol/L 4-aminoantipyrine (manufactured by ACTEC Laboratories Inc.)
625 U/mL catalase (manufactured by Nagase ChemteX Corp.)
50 kU/mL protease for glycated albumin (manufactured by Asahi Kasei Pharma Corp.; obtained in accordance with the method described in Japanese Patent No. 4565807)
1.25 mmol/L trisodium citrate (manufactured by FUJIFILM Wako Pure Chemical Corp.)
Stabilizer having a concentration described later

(Three-reagent system: Stabilizer contained in 4-aminoantipyrine-containing liquid partial composition (1))

37.5 wt/vol% propylene glycol (manufactured by FUJIFILM Wako Pure Chemical Corp.)
62.5 wt/vol% trimethylene glycol (manufactured by FUJIFILM Wako Pure Chemical Corp.)
62.5 wt/vol% ethylene glycol (manufactured by FUJIFILM Wako Pure Chemical Corp.)
0.75 wt/vol% 2-carboxyphenylboronic acid (manufactured by Tokyo Chemical Industry Co., Ltd.)

(Three-reagent system: Preparation of ferrocyanide-containing liquid partial composition (1))

The following reagent components were dissolved in purified water so as to achieve the described concentrations. The solution was adjusted to pH 7.0 with sodium hydroxide to prepare a ferrocyanide-containing liquid partial composition (1), the partial composition containing trisodium citrate or containing no trisodium citrate.

100 mmol/L 3,3-dimethylglutaric acid (manufactured by Tokyo Chemical Industry Co., Ltd.)
0.05 wt/vol% ProClin 300 (manufactured by Sigma-Aldrich Co., LLC)
0.5 mmol/L potassium ferrocyanide (manufactured by Kokusan Chemical Co., Ltd.)
5 mmol/L trisodium citrate (manufactured by FUJIFILM Wako Pure Chemical Corp.)

(Three-reagent system: Preparation of 4-aminoantipyrine-containing liquid partial composition (2))

The following reagent components were dissolved in purified water so as to achieve the described concentrations. The solution was adjusted to pH 7.0 with sodium hydroxide to prepare a partial composition containing 4-aminoantipyrine-containing liquid partial composition (2).

100 mmol/L 3,3-dimethylglutaric acid (manufactured by Tokyo Chemical Industry Co., Ltd.)
0.05 wt/vol% ProClin 300 (manufactured by Sigma-Aldrich Co., LLC)
25 mmol/L 4-aminoantipyrine (manufactured by ACTEC Laboratories Inc.)

(Three-reagent system: Preparation of ferrocyanide-containing liquid partial composition (2))

The following reagent components were dissolved in purified water so as to achieve the described concentrations. The solution was adjusted to pH 7.5 with sodium hydroxide to prepare a ferrocyanide-containing liquid partial composition (2) containing propylene glycol as a stabilizer, the partial composition containing trisodium citrate or containing no trisodium citrate.

250 mmol/L 3,3-dimethylglutaric acid (manufactured by Tokyo Chemical Industry Co., Ltd.)
0.05 wt/vol% ProClin 300 (manufactured by Sigma-Aldrich Co., LLC)
0.125 mmol/L potassium ferrocyanide (manufactured by Kokusan Chemical Co., Ltd.)
625 U/mL catalase (manufactured by Nagase ChemteX Corp.)
50 kU/mL protease for glycated albumin (manufactured by Asahi Kasei Pharma Corp.; obtained in accordance with the method described in Japanese Patent No. 4565807)
1.25 mmol/L trisodium citrate (manufactured by FUJIFILM Wako Pure Chemical Corp.)
37.5 wt/vol% propylene glycol (manufactured by FUJIFILM Wako Pure Chemical Corp.)

(Sample)

The following samples were provided.

Control serum L (manufactured by Asahi Kasei Pharma Corp.)

Control serum H (manufactured by Asahi Kasei Pharma Corp.)

(Reagent preservation)

The prepared Trinder reagent-containing liquid partial composition was preserved at 5°C for 14 days or 28 days. Also, the 4-aminoantipyrine- and ferrocyanide-containing liquid partial composition, the 4-aminoantipyrine-containing liquid partial composition, and the ferrocyanide-containing liquid partial composition were preserved at 30°C for 14 days or 28 days.

(Assay preparation)

The 4-aminoantipyrine-containing liquid partial composition (1) and the ferrocyanide-containing liquid partial composition (1) of the three-reagent system were mixed at a volume ratio of 4:1 to prepare a 4-aminoantipyrine- and ferrocyanide-containing liquid partial composition (1) for use in assay.

The 4-aminoantipyrine-containing liquid partial composition (2) and the ferrocyanide-containing liquid partial composition (2) of the three-reagent system were mixed at a volume ratio of 1:4 to prepare a 4-aminoantipyrine- and ferrocyanide-containing liquid partial composition (2) for use in assay.

(Assay procedure)

To 180 $\mu$L of the Trinder reagent-containing liquid partial composition, 4.5 $\mu$L of the sample was added, and the mixture was reacted at 37°C for 5 minutes. Then, 45 $\mu$L of the 4-aminoantipyrine- and ferrocyanide-containing liquid partial composition was added thereto, and the mixture was further reacted at 37°C for 5 minutes. Change in absorbance was measured from absorbance Abs1 5 minutes after the sample was added to the Trinder reagent-containing liquid partial composition to absorbance Abs2 5 minutes after the 4-aminoantipyrine- and ferrocyanide-containing liquid partial composition was added to the mixed solution of the Trinder reagent-containing liquid partial composition and the sample. The change in absorbance, $A_C$, was given according to the following expression (12).

$$A_C = (Abs2) - (Abs1) \times 180 / 225 \quad (12)$$

wherein 180 represents the liquid quantity of the Trinder reagent-containing liquid partial composition, and 225 represents the total liquid quantity of the Trinder reagent-containing liquid partial composition and the 4-aminoantipyrine- and ferrocyanide-containing liquid partial composition.

Change in absorbance measured using the sample was defined as Acs, and change in absorbance measured using purified water instead of the sample was defined as a reagent blank Acb. A value obtained by subtracting the change in absorbance, Acb, of the reagent blank from the change in absorbance, Acs, measured using the sample was calculated as sensitivity $\Delta$Abs according to the following expression (13).

$$\Delta Abs = Acs - Acb \quad (13)$$

The sensitivity of the partial composition preserved for 14 days was defined as AAbs14, and the sensitivity of the partial composition preserved for 28 days was defined as $\Delta$Abs28. The sensitivity preservation rate was calculated as the ratio of $\Delta$Abs28 to $\Delta$Abs14 according to the following expression (14). The sensitivity preservation rate is shown in Table 6, together with the results about the reagent blank Acb of the partial composition preserved for 14 days.

Sensitivity preservation rate (%) = $\Delta$Abs28 / $\Delta$Abs14 $\times$ 100 (14)         (14)

[Table 6-1]

| Reagent type | 4-Aminoantipyrine -containing liquid partial composition | Ferrocyanide -containing liquid partial composition | Reagent blank after 14-day preservatio n (mAbs) | Sampl e | Sensitivity preservatio n rate (%) |
|---|---|---|---|---|---|
| Two-reagent system | 30 wt/vol% propylene glycol | | 2.1 | Control serum L | 87.1 |
| | | | | Control serum H | 85.1 |
| Two-reagent system | 30 wt/vol% propylene glycol 1 mmol/L trisodium citrate | | 1.6 | Control serum L | 89.8 |
| | | | | Control serum H | 87.8 |
| Two-reagent system | 37.5 wt/vol% propylene glycol | | 1.6 | Control serum L | 90.9 |
| | | | | Control serum H | 90.4 |
| Two-reagent system | 37.5 wt/vol% propylene glycol 1 mmol/L trisodium citrate | | 1.1 | Control serum L | 93.5 |
| | | | | Control serum H | 92.8 |
| Three-reagent system | Partial composition (1) 37.5 wt/vol% propylene glycol | Partial composition (1) | 1.2 | Control serum L | 96.5 |
| | | | | Control serum H | 95.5 |
| Three-reagent system | | Partial composition (1) 5 mmol/L trisodium ci-trate | 1.2 | Control serum L | 96.6 |
| | | | | Control serum H | 95.0 |

[Table 6-2]

| Three-reagent system | Partial composition (1) 37.5 wt/vol% propylene glycol 1.25 mmol/L trisodium ci-trate | Partial composition (1) | 0.9 | Control serum L | 96.0 |
|---|---|---|---|---|---|
| | | | | Control serum H | 94.7 |
| Three-reagent system | | Partial composition (1) 5 mmol/L trisodium citrate | 1.0 | Control serum L | 96.3 |
| | | | | Control serum H | 95.0 |

(continued)

| Three-reagent system | Partial composition (2) | Partial composition (2) 37.5 wt/vol% propylene glycol | 1.6 | Control serum L | 95.3 |
| | | | | Control serum H | 94.7 |
| Three-reagent system | | Partial composition (2) 37.5 wt/vol% propylene glycol 1.25 mmol/L trisodium citrate | 1.7 | Control serum L | 95.3 |
| | | | | Control serum H | 95.1 |
| Three-reagent system | Partial composition (1) 62.5 wt/vol% trimethylene glycol | Partial composition (1) | 2.5 | Control serum L | 96.5 |
| | | | | Control serum H | 95.8 |
| Three-reagent system | | Partial composition (1) 5 mmol/L trisodium citrate | 1.8 | Control serum L | 96.3 |
| | | | | Control serum H | 95.6 |

[Table 6-3]

| Three-reagent system | Partial composition (1) 62.5 wt/vol% trimethylene glycol 1.25 mmol/L trisodium citrate | Partial composition (1) | 1.7 | Control serum L | 96.7 |
| | | | | Control serum H | 95.5 |
| Three-reagent system | | Partial composition (1) 5 mmol/L trisodium citrate | 1.6 | Control serum L | 97.2 |
| | | | | Control serum H | 96.4 |
| Three-reagent system | Partial composition (1) 62.5 wt/vol% ethylene glycol | Partial composition (1) | 1.8 | Control serum L | 92.4 |
| | | | | Control serum H | 91.3 |
| Three-reagent system | | Partial composition (1) 5 mmol/L trisodium citrate | 1.5 | Control serum L | 92.8 |
| | | | | Control serum H | 91.0 |
| Three-reagent system | Partial composition (1) 62.5 wt/vol%ethylene glycol 1.25 mmol/L trisodium citrate | Partial composition (1) | 1.4 | Control serum L | 93.0 |
| | | | | Control serum H | 90.8 |
| Three-reagent system | | Partial composition (1) 5 mmol/L trisodium citrate | 1.3 | Control serum L | 92.5 |
| | | | | Control serum H | 91.3 |

[Table 6-4]

| Three-reagent system | Partial composition (1) 0.75 wt/vol% 2-carboxyphenylboronic acid | Partial composition (1) | 1.8 | Control serum L | 96.1 |
| | | | | Control serum H | 95.1 |
| Three-reagent system | | Partial composition (1) 5 mmol/L trisodium citrate | 1.8 | Control serum L | 95.6 |
| | | | | Control serum H | 95.0 |

(continued)

| Three-reagent system | Partial composition (1) 0.75 wt/vol% 2-carboxyphenylboronic acid 1.25 mmol/L trisodium citrate | Partial composition (1) | 2.1 | Control serum L | 96.0 |
| --- | --- | --- | --- | --- | --- |
| | | | | Control serum H | 94.7 |
| Three-reagent system | | Partial composition (1) 5 mmol/L trisodium citrate | 2.1 | Control serum L | 95.2 |
| | | | | Control serum H | 94.6 |

As is evident from Table 6, when a stabilizer that gave an oxidation-reduction potential of ferrocyanide higher than 0.058 V upon mixing the stabilizer with the ferrocyanide was used, the reagent blank without the addition of the chelating agent (trisodium citrate) was almost the same as the reagent blank in the case of adding the chelating agent (trisodium citrate).

[Example 4: Effect of pH and chelating agent type]

(Preparation of Trinder reagent-containing liquid partial composition)

The following reagent components were dissolved in purified water so as to achieve the described concentrations. The solution was adjusted to pH 7.6 with sodium hydroxide to prepare a Trinder reagent-containing liquid partial composition.

100 mmol/L N-tris(hydroxymethyl)methyl-2-aminoethanesulfonic acid (manufactured by Dojindo Laboratories Co., Ltd.)
0.05 wt/vol% sodium azide (manufactured by Merck KGaA)
1 mmol/L sodium ethylenediaminetetraacetate (manufactured by Kokusan Chemical Co., Ltd.)
6 wt/vol% D(-)-sorbitol (manufactured by FUJIFILM Wako Pure Chemical Corp.)
2 mmol/L N,N-bis(4-sulfobutyl)-3-methylaniline, disodium salt (manufactured by Dojindo Laboratories Co., Ltd.)
10 U/mL peroxidase (manufactured by Amano Enzyme Inc.)
30 U/mL ketoamine oxidase (manufactured by Asahi Kasei Pharma Corp.)

(Three-reagent system: Preparation of 4-aminoantipyrine-containing liquid partial composition)

The following reagent components were dissolved in purified water so as to achieve the described concentrations. The solution was adjusted to pH 7.5 with sodium hydroxide to prepare a 4-aminoantipyrine-containing liquid partial composition containing propylene glycol as a stabilizer.

250 mmol/L 3,3-dimethylglutaric acid (manufactured by Tokyo Chemical Industry Co., Ltd.)
0.05 wt/vol% ProClin 300 (manufactured by Sigma-Aldrich Co., LLC)
6.25 mmol/L 4-aminoantipyrine (manufactured by ACTEC Laboratories Inc.)
625 U/mL catalase (manufactured by Nagase ChemteX Corp.)
50 kU/mL protease for glycated albumin (manufactured by Asahi Kasei Pharma Corp.; obtained in accordance with the method described in Japanese Patent No. 4565807)
50 wt/vol% propylene glycol (manufactured by FUJIFILM Wako Pure Chemical Corp.)

(Three-reagent system: Preparation of ferrocyanide-containing liquid partial composition (1))

The following reagent components were dissolved in purified water so as to achieve the described concentrations. The solution was pH-adjusted with sodium hydroxide to prepare a ferrocyanide-containing liquid partial composition (1) having distinctive pH.

100 mmol/L 3,3-dimethylglutaric acid (manufactured by Tokyo Chemical Industry Co., Ltd.)
0.05 wt/vol% ProClin 300 (manufactured by Sigma-Aldrich Co., LLC)
0.5 mmol/L potassium ferrocyanide (manufactured by Kokusan Chemical Co., Ltd.)
Adjusted to pH of a concentration described later

(pH of ferrocyanide-containing liquid partial composition (1))

pH 6.5
pH 7.0
pH 7.5
pH 8.0

(Three-reagent system: Preparation of ferrocyanide-containing liquid partial composition (2))

The following reagent components were dissolved in purified water so as to achieve the described concentrations. The solution was adjusted to pH 7.0 with sodium hydroxide to prepare a ferrocyanide-containing liquid partial composition (2) containing a chelating agent.

100 mmol/L 3,3-dimethylglutaric acid (manufactured by Tokyo Chemical Industry Co., Ltd.)
0.05 wt/vol% ProClin 300 (manufactured by Sigma-Aldrich Co., LLC)
0.5 mmol/L potassium ferrocyanide (manufactured by Kokusan Chemical Co., Ltd.)
Chelating agent having a concentration described later

(Chelating agent contained in ferrocyanide-containing liquid partial composition (2))

5 mmol/L sodium ethylenediaminetetraacetate (manufactured by Kokusan Chemical Co., Ltd., abbreviation: EDTA)
5 mmol/L glycol ether diaminetetraacetic acid (manufactured by Dojindo Laboratories Co., Ltd., abbreviation: EGTA)
5 mmol/L N-(2-hydroxyethyl)iminodiacetic acid (manufactured by Dojindo Laboratories Co., Ltd., abbreviation: HIDA)
5 mmol/L nitrilotris(methylphosphonic acid), trisodium salt (manufactured by Dojindo Laboratories Co., Ltd., abbreviation: NTPO)
5 mmol/L nitrilotriacetic acid (manufactured by Dojindo Laboratories Co., Ltd., abbreviation: NTA)
5 mmol/L trans-1,2-diaminocyclohexane-N,N,N,N-tetraacetic acid, monohydrate (manufactured by Dojindo Laboratories Co., Ltd., abbreviation: CyDTA)
5 mmol/L iminodiacetic acid (manufactured by Dojindo Laboratories Co., Ltd., abbreviation: IDA)
5 mmol/L diethylenetriamine-N,N,N',N'',N''-pentaacetic acid (manufactured by Dojindo Laboratories Co., Ltd., abbreviation: DTPA)
5 mmol/L 1,3-diamino-2-propanol-N,N,N',N'-tetraacetic acid (manufactured by Dojindo Laboratories Co., Ltd., abbreviation: DPTA-OH)
5 mmol/L sodium gluconate (manufactured by FUJIFILM Wako Pure Chemical Corp.)
5 mmol/L sodium L(-)-malate (manufactured by FUJIFILM Wako Pure Chemical Corp.)
5 mmol/L succinic acid (manufactured by Kanto Chemical Co., Inc.)
5 mmol/L trisodium citrate (manufactured by FUJIFILM Wako Pure Chemical Corp.)
5 mmol/L salicylic acid (manufactured by Nacalai Tesque, Inc.)
5 mmol/L potassium (+)-tartrate (manufactured by FUJIFILM Wako Pure Chemical Corp.)
5 mmol/L N-[tris(hydroxymethyl)methyl]glycine (manufactured by Dojindo Laboratories Co., Ltd., abbreviation: Tricine)
5 mmol/L N,N-bis(2-hydroxyethyl)glycine (manufactured by Dojindo Laboratories Co., Ltd., abbreviation: Bicine)

(Sample)

The following samples were provided.

Control serum L (manufactured by Asahi Kasei Pharma Corp.)
Control serum H (manufactured by Asahi Kasei Pharma Corp.)

(Reagent preservation)

The prepared Trinder reagent-containing liquid partial composition was preserved at 5°C for 14 days or 28 days. Also, the 4-aminoantipyrine-containing liquid partial composition and the ferrocyanide-containing liquid partial compositions (1) and (2) were preserved at 30°C for 14 days or 28 days.

(Assay preparation)

The 4-aminoantipyrine-containing liquid partial composition and the ferrocyanide-containing liquid partial composition (1) or (2) of the three-reagent system were mixed at a volume ratio of 4:1 to prepare a 4-aminoantipyrine- and ferrocyanide-containing liquid partial composition for use in assay.

(Assay procedure)

To 180 $\mu$L of the Trinder reagent-containing liquid partial composition, 4.5 $\mu$L of the sample was added, and the mixture was reacted at 37°C for 5 minutes. Then, 45 $\mu$L of the 4-aminoantipyrine- and ferrocyanide-containing liquid partial composition was added thereto, and the mixture was further reacted at 37°C for 5 minutes. Change in absorbance was measured from absorbance Absl 5 minutes after the sample was added to the Trinder reagent-containing liquid partial composition to absorbance Abs2 5 minutes after the 4-aminoantipyrine- and ferrocyanide-containing liquid partial composition was added to the mixed solution of the Trinder reagent-containing liquid partial composition and the sample. The change in absorbance, $A_C$, was given according to the following expression (15).

$$A_C = (Abs2) - (Abs1) \times 180 / 225 \quad (15)$$

wherein 180 represents the liquid quantity of the Trinder reagent-containing liquid partial composition, and 225 represents the total liquid quantity of the Trinder reagent-containing liquid partial composition and the 4-aminoantipyrine- and ferrocyanide-containing liquid partial composition.

Change in absorbance measured using the sample was defined as Acs, and change in absorbance measured using purified water instead of the sample was defined as a reagent blank Acb. A value obtained by subtracting the change in absorbance, Acb, of the reagent blank from the change in absorbance, Acs, measured using the sample was calculated as sensitivity $\Delta$Abs according to the following expression (16).

$$\Delta Abs = Acs - Acb \quad (16)$$

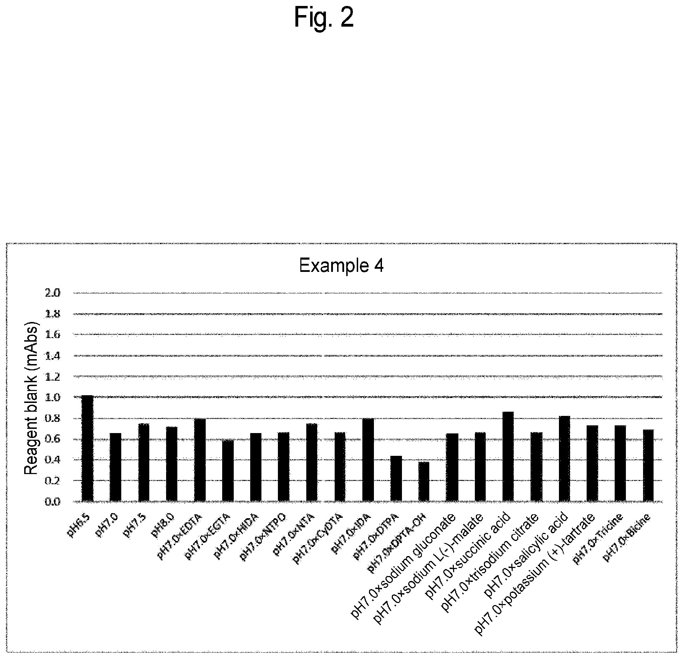

The sensitivity of the partial composition preserved for 14 days was defined as $\Delta$Abs14, and the sensitivity of the partial composition preserved for 28 days was defined as $\Delta$Abs28. The sensitivity preservation rate was calculated as the ratio of $\Delta$Abs28 to $\Delta$Abs14 according to the following expression (17). The sensitivity preservation rate is shown in Table 7 and Figure 2, together with the results about the reagent blank Acb of the partial composition preserved for 14 days.

Sensitivity preservation rate (%) = $\Delta$Abs28 / $\Delta$Abs14 $\times$ 100 (17)　　　　　　　　　　　(17)

[Table 7-1]

| Reagent type | Ferrocyanide-containing liquid partial composition | | Reagent blank after 14-d preservation (mAbs) | Sample | Sensitivity preservation rate (%) |
|---|---|---|---|---|---|
| | pH | Chelating agent type | | | |
| Three-reagent system | 6.5 | None | 1.0 | Control serum L | 101.8 |
| | | | | Control serum H | 99.6 |
| Three-reagent system | 7.0 | None | 0.7 | Control serum L | 101.8 |
| | | | | Control serum H | 100.4 |
| Three-reagent system | 7.5 | None | 0.7 | Control serum L | 101.8 |
| | | | | Control serum H | 100.5 |
| Three-reagent system | 8.0 | None | 0.7 | Control serum L | 102.9 |
| | | | | Control serum H | 101.0 |

(continued)

| Reagent type | Ferrocyanide-containing liquid partial composition | | Reagent blank after 14-d preservation (mAbs) | Sample | Sensitivity preservation rate (%) |
|---|---|---|---|---|---|
| | pH | Chelating agent type | | | |
| Three-reagent system | 7.0 | EDTA | 0.8 | Control serum L | 102.0 |
| | | | | Control serum H | 100.7 |
| Three-reagent system | 7.0 | EGTA | 0.6 | Control serum L | 102.1 |
| | | | | Control serum H | 100.8 |
| Three-reagent system | 7.0 | HIDA | 0.7 | Control serum L | 101.6 |
| | | | | Control serum H | 100.8 |
| Three-reagent system | 7.0 | NTPO | 0.7 | Control serum L | 101.6 |
| | | | | Control serum H | 100.5 |
| Three-reagent system | 7.0 | NTA | 0.8 | Control serum L | 102.4 |
| | | | | Control serum H | 100.5 |
| Three-reagent system | 7.0 | CyDTA | 0.7 | Control serum L | 101.9 |
| | | | | Control serum H | 100.8 |
| Three-reagent system | 7.0 | IDA | 0.8 | Control serum L | 101.8 |
| | | | | Control serum H | 100.4 |

[Table 7-2]

| Three-reagent system | 7.0 | DTPA | 0.4 | Control serum L | 102.8 |
|---|---|---|---|---|---|
| | | | | Control serum H | 100.4 |
| Three-reagent system | 7.0 | DPTA-OH | 0.4 | Control serum L | 101.9 |
| | | | | Control serum H | 100.6 |
| Three-reagent system | 7.0 | Sodium gluconate | 0.7 | Control serum L | 102.2 |
| | | | | Control serum H | 100.9 |
| Three-reagent system | 7.0 | Sodium L(-)-malate | 0.7 | Control serum L | 102.1 |
| | | | | Control serum H | 100.2 |
| Three-reagent system | 7.0 | Succinic acid | 0.9 | Control serum L | 101.4 |
| | | | | Control serum H | 99.9 |
| Three-reagent system | 7.0 | Trisodium citrate | 0.7 | Control serum L | 101.7 |
| | | | | Control serum H | 100.6 |
| Three-reagent system | 7.0 | Salicylic acid | 0.8 | Control serum L | 101.7 |
| | | | | Control serum H | 100.1 |
| Three-reagent system | 7.0 | Potassium (+)-tartrate | 0.7 | Control serum L | 101.9 |
| | | | | Control serum H | 100.7 |
| Three-reagent system | 7.0 | Tricine | 0.7 | Control serum L | 102.1 |
| | | | | Control serum H | 100.5 |

(continued)

| Three-reagent system | 7.0 | Bicine | 0.7 | Control serum L | 102.7 |
|---|---|---|---|---|---|
| | | | | Control serum H | 100.7 |

As is evident from Table 7 and Figure 2, varying values of pH of the ferrocyanide-containing liquid partial composition caused small change in reagent blank. Also, varying types of the chelating agent added to the ferrocyanide-containing liquid partial composition caused small change in reagent blank.

[Example 5: Effect of pH]

(Preparation of Trinder reagent-containing liquid partial composition)

The following reagent components were dissolved in purified water so as to achieve the described concentrations. The solution was adjusted to pH 7.6 with sodium hydroxide to prepare a Trinder reagent-containing liquid partial composition.

100 mmol/L N-tris(hydroxymethyl)methyl-2-aminoethanesulfonic acid (manufactured by Dojindo Laboratories Co., Ltd.)
0.05 wt/vol% sodium azide (manufactured by Merck KGaA)
1 mmol/L sodium ethylenediaminetetraacetate (manufactured by Kokusan Chemical Co., Ltd.)
6 wt/vol% D(-)-sorbitol (manufactured by FUJIFILM Wako Pure Chemical Corp.)
2 mmol/L N,N-bis(4-sulfobutyl)-3-methylaniline, disodium salt (manufactured by Dojindo Laboratories Co., Ltd.)
10 U/mL peroxidase (manufactured by Amano Enzyme Inc.)
30 U/mL ketoamine oxidase (manufactured by Asahi Kasei Pharma Corp.)

(Three-reagent system: Preparation of 4-aminoantipyrine-containing liquid partial composition (1))

The following reagent components were dissolved in purified water so as to achieve the described concentrations. The solution was adjusted to pH 7.5 with sodium hydroxide to prepare a 4-aminoantipyrine-containing liquid partial composition (1) containing a stabilizer, the partial composition containing trisodium citrate or containing no trisodium citrate.

250 mmol/L 3,3-dimethylglutaric acid (manufactured by Tokyo Chemical Industry Co., Ltd.)
0.05 wt/vol% ProClin 300 (manufactured by Sigma-Aldrich Co., LLC)
6.25 mmol/L 4-aminoantipyrine (manufactured by ACTEC Laboratories Inc.)
625 U/mL catalase (manufactured by Nagase ChemteX Corp.)
50 kU/mL protease for glycated albumin (manufactured by Asahi Kasei Pharma Corp.; obtained in accordance with the method described in Japanese Patent No. 4565807)
1.25 mmol/L trisodium citrate (manufactured by FUJIFILM Wako Pure Chemical Corp.)
Stabilizer having a concentration described later

(Three-reagent system: Stabilizer contained in 4-aminoantipyrine-containing liquid partial composition (1))

37.5 wt/vol% propylene glycol (manufactured by FUJIFILM Wako Pure Chemical Corp.)
62.5 wt/vol% trimethylene glycol (manufactured by FUJIFILM Wako Pure Chemical Corp.)
62.5 wt/vol% ethylene glycol (manufactured by FUJIFILM Wako Pure Chemical Corp.)
0.75 wt/vol% 2-carboxyphenylboronic acid (manufactured by Tokyo Chemical Industry Co., Ltd.)

(Three-reagent system: Preparation of ferrocyanide-containing liquid partial composition (1))

The following reagent components were dissolved in purified water so as to achieve the described concentrations. The solution was adjusted to pH 7.0 or pH 8.0 to prepare a ferrocyanide-containing liquid partial composition. The ferrocyanide-containing liquid partial composition of pH 8.0 was prepared as a partial composition containing trisodium citrate and a partial composition containing no trisodium citrate.

100 mmol/L 3,3-dimethylglutaric acid (manufactured by Tokyo Chemical Industry Co., Ltd.)
0.05 wt/vol% ProClin 300 (manufactured by Sigma-Aldrich Co., LLC)
0.5 mmol/L potassium ferrocyanide (manufactured by Kokusan Chemical Co., Ltd.)
5 mmol/L trisodium citrate (manufactured by FUJIFILM Wako Pure Chemical Corp.)

(Three-reagent system: Preparation of 4-aminoantipyrine-containing liquid partial composition (2))

The following reagent components were dissolved in purified water so as to achieve the described concentrations. The solution was adjusted to pH 7.0 or pH 8.0 with sodium hydroxide to prepare a 4-aminoantipyrine-containing liquid partial composition. The 4-aminoantipyrine-containing liquid partial composition adjusted to pH 8.0 was prepared as a partial composition containing trisodium citrate and a partial composition containing no trisodium citrate.

100 mmol/L 3,3-dimethylglutaric acid (manufactured by Tokyo Chemical Industry Co., Ltd.)
0.05 wt/vol% ProClin 300 (manufactured by Sigma-Aldrich Co., LLC)
25 mmol/L 4-aminoantipyrine (manufactured by ACTEC Laboratories Inc.)
25 mmol/L trisodium citrate (manufactured by FUJIFILM Wako Pure Chemical Corp.) (added only to the partial composition adjusted to pH 8.0)

(Three-reagent system: Preparation of ferrocyanide-containing liquid partial composition (2))

The following reagent components were dissolved in purified water so as to achieve the described concentrations. The solution was adjusted to pH 7.5 with sodium hydroxide to prepare a ferrocyanide-containing liquid partial composition containing propylene glycol as a stabilizer, the partial composition containing trisodium citrate or containing no trisodium citrate.

250 mmol/L 3,3-dimethylglutaric acid (manufactured by Tokyo Chemical Industry Co., Ltd.)
0.05 wt/vol% ProClin 300 (manufactured by Sigma-Aldrich Co., LLC)
0.125 mmol/L potassium ferrocyanide (manufactured by Kokusan Chemical Co., Ltd.)
625 U/mL catalase (manufactured by Nagase ChemteX Corp.)
50 kU/mL protease for glycated albumin (manufactured by Asahi Kasei Pharma Corp.; obtained in accordance with the method described in Japanese Patent No. 4565807)
1.25 mmol/L trisodium citrate (manufactured by FUJIFILM Wako Pure Chemical Corp.)
37.5 wt/vol% propylene glycol (manufactured by FUJIFILM Wako Pure Chemical Corp.)

(Sample)

The following samples were provided.

Control serum L (manufactured by Asahi Kasei Pharma Corp.)
Control serum H (manufactured by Asahi Kasei Pharma Corp.)

(Reagent preservation)

The prepared Trinder reagent-containing liquid partial composition was preserved at 5°C for 14 days or 28 days. Also, the 4-aminoantipyrine-containing liquid partial composition and the ferrocyanide-containing liquid partial compositions were preserved at 30°C for 14 days or 28 days.

(Assay preparation)

The 4-aminoantipyrine-containing liquid partial composition (1) and the ferrocyanide-containing liquid partial composition (1) of the three-reagent system were mixed at a volume ratio of 4:1 to prepare a 4-aminoantipyrine- and ferrocyanide-containing liquid partial composition (1) for use in assay.
The 4-aminoantipyrine-containing liquid partial composition (2) and the ferrocyanide-containing liquid partial composition (2) of the three-reagent system were mixed at a volume ratio of 1:4 to prepare a 4-aminoantipyrine- and ferrocyanide-containing liquid partial composition (2) for use in assay.

(Assay procedure)

To 180 μL of the Trinder reagent-containing liquid partial composition, 4.5 μL of the sample was added, and the mixture was reacted at 37°C for 5 minutes. Then, 45 μL of the 4-aminoantipyrine- and ferrocyanide-containing liquid partial composition was added thereto, and the mixture was further reacted at 37°C for 5 minutes. Change in absorbance was measured from absorbance Abs1 5 minutes after the sample was added to the Trinder reagent-containing liquid partial composition to absorbance Abs2 5 minutes after the 4-aminoantipyrine- and ferrocyanide-containing liquid partial composition was added to the mixed solution of the Trinder reagent-containing liquid partial composition and the sample. The change in absorbance, $A_C$, was given according to the following expression (18).

$$A_C = (Abs2) - (Abs1) \times 180 / 225 \quad (18)$$

wherein 180 represents the liquid quantity of the Trinder reagent-containing liquid partial composition, and 225 represents the total liquid quantity of the Trinder reagent-containing liquid partial composition and the 4-aminoantipyrine- and ferrocyanide-containing liquid partial composition.

Change in absorbance measured using the sample was defined as Acs, and change in absorbance measured using purified water instead of the sample was defined as a reagent blank Acb. A value obtained by subtracting the change in absorbance, Acb, of the reagent blank from the change in absorbance, Acs, measured using the sample was calculated as sensitivity ΔAbs according to the following expression (19).

$$\Delta Abs = Acs - Acb \quad (19)$$

The sensitivity of the partial composition preserved for 14 days was defined as ΔAbs14, and the sensitivity of the partial composition preserved for 28 days was defined as ΔAbs28. The sensitivity preservation rate was calculated as the ratio of ΔAbs28 to ΔAbs14 according to the following expression (20). The sensitivity preservation rate is shown in Table 8, together with the results about the reagent blank Acb of the partial composition preserved for 14 days.

Sensitivity preservation rate (%) = ΔAbs28 / ΔAbs14 × 100 (20)　　　　　　　　(20)

[Table 8-1]

| Reagent type | 4-Aminoantipyrine-containing liquid partial composition | Ferrocyanide-containing liquid partial composition | Reagent blank after 14-d preservation (mAbs) | Sample | Sensitivity preservation rate (%) |
|---|---|---|---|---|---|
| Three-reagent system | Partial composition (1)<br><br>37.5 wt/vol% propylene glycol | Partial composition (1)<br><br>pH 7.0 | 1.2 | Control serum L | 96.5 |
| | | | | Control serum H | 95.5 |
| Three-reagent system | | Partial composition (1)<br><br>pH 8.0 | 1.3 | Control serum L | 96.9 |
| | | | | Control serum H | 95.3 |
| Three-reagent system | | Partial composition (1)<br><br>pH 8.0<br>5 mmol/L trisodium citrate | 1.2 | Control serum L | 95.9 |
| | | | | Control serum H | 95.1 |

(continued)

| Reagent type | 4-Aminoantipyrine-containing liquid partial composition | Ferrocyanide-containing liquid partial composition | Reagent blank after 14-d preservation (mAbs) | Sample | Sensitivity preservation rate (%) |
|---|---|---|---|---|---|
| Three-reagent system | Partial composition (1)<br><br>37.5 wt/vol% glycol propylene<br><br>1.25 mmol/L trisodium citrate | Partial composition (1)<br><br>pH 7.0 | 0.9 | Control serum L | 96.0 |
| | | | | Control serum H | 94.7 |
| Three-reagent system | | Partial composition (1)<br><br>pH 8.0 | 1.0 | Control serum L | 96.2 |
| | | | | Control serum H | 94.7 |
| Three-reagent system | | Partial composition (1)<br><br>pH 8.0<br><br>5 mmol/L trisodium citrate | 1.0 | Control serum L | 96.0 |
| | | | | Control serum H | 94.9 |

[Table 8-2]

| | | | | | |
|---|---|---|---|---|---|
| Three-reagent system | Partial composition (2) pH 7.0 | Partial composition (2) 37.5 wt/vol% propylene glycol | 1.6 | Control serum L | 95.3 |
| | | | | Control serum H | 94.7 |
| Three-reagent system | Partial composition (2) pH 8.0 | | 1.3 | Control serum L | 96.0 |
| | | | | Control serum H | 95.9 |
| Three-reagent system | Partial composition (2) pH 8.0 25 mmol/L trisodium citrate | | 1.1 | Control serum L | 96.5 |
| | | | | Control serum H | 95.4 |
| Three-reagent system | Partial composition (2) pH 7.0 | Partial composition (2) 37.5 wt/vol% propylene glycol 1.25 mmol/L trisodium citrate | 1.7 | Control serum L | 95.3 |
| | | | | Control serum | 95.1 |
| Three-reagent system | Partial composition (2) pH 8.0 | | 1.3 | Control serum L | 96.3 |
| | | | | Control serum H | 95.8 |
| Three-reagent system | Partial composition (2) pH 8.0 25 mmol/L trisodium citrate | | 1.3 | Control serum L | 96.3 |
| | | | | Control serum H | 95.6 |

[Table 8-3]

| Three-reagent system | Partial composition (1) 62.5 wt/vol% trimethylene glycol | Partial composition (1) pH 7.0 | 2.5 | Control serum L | 96.5 |
|---|---|---|---|---|---|
| | | | | Control serum H | 95.8 |
| Three-reagent system | | Partial composition (1) pH 8.0 | 2.3 | Control serum L | 96.3 |
| | | | | Control serum H | 96.1 |
| Three-reagent system | | Partial composition (1) pH 8.0  5 mmol/L trisodium citrate | 1.7 | Control serum L | 96.5 |
| | | | | Control serum H | 95.9 |
| Three-reagent system | Partial composition (1) 62.5 wt/vol% trimethylene glycol  1.25 mmol/L trisodium citrate | Partial composition (1) pH 7.0 | 1.7 | Control serum L | 96.7 |
| | | | | Control serum H | 95.5 |
| Three-reagent system | | Partial composition (1) pH 8.0 | 1.3 | Control serum L | 96.6 |
| | | | | Control serum H | 95.8 |
| Three-reagent system | | Partial composition (1) pH 8.0  5 mmol/L trisodium citrate | 1.5 | Control serum L | 97.0 |
| | | | | Control serum H | 95.8 |

[Table 8-4]

| Three-reagent system | Partial composition (1) 62.5 wt/vol% ethylene glycol | Partial composition (1) pH 7.0 | 1.8 | Control serum L | 92.4 |
|---|---|---|---|---|---|
| | | | | Control serum H | 91.3 |
| Three-reagent system | | Partial composition (1) pH 8.0 | 1.8 | Control serum L | 92.2 |
| | | | | Control serum H | 91.4 |
| Three-reagent system | | Partial composition (1) pH 8.0  5 mmol/L trisodium citrate | 1.7 | Control serum L | 93.0 |
| | | | | Control serum H | 91.7 |
| Three-reagent system | Partial composition (1) 62.5 wt/vol% ethylene glycol  1.25 mmol/L trisodium citrate | Partial composition (1) pH 7.0 | 1.4 | Control serum L | 93.0 |
| | | | | Control serum H | 90.8 |
| Three-reagent system | | Partial composition (1) pH 8.0 | 1.2 | Control serum L | 92.2 |
| | | | | Control serum H | 91.0 |
| Three-reagent system | | Partial composition (1) pH 8.0  5 mmol/L trisodium citrate | 1.4 | Control serum L | 92.9 |
| | | | | Control serum H | 91.8 |

[Table 8-5]

| Three-reagent system | Partial composition (1) 0.75 wt/vol% 2-carboxyphenylboronic acid | Partial composition (1) pH 7.0 | 1.8 | Control serum L | 96.1 |
|---|---|---|---|---|---|
| | | | | Control serum H | 95.1 |
| Three-reagent system | | Partial composition (1) pH 8.0 | 1.7 | Control serum L | 96.0 |
| | | | | Control serum H | 94.4 |
| Three-reagent system | | Partial composition (1) pH 8.0 5 mmol/L trisodium citrate | 1.8 | Control serum L | 95.4 |
| | | | | Control serum H | 95.1 |
| Three-reagent system | Partial composition (1) 0.75 wt/vol% 2-carboxyphenylboronic acid 1.25 mmol/L trisodium citrate | Partial composition (1) pH 7.0 | 2.1 | Control serum L | 96.0 |
| | | | | Control serum H | 94.7 |
| Three-reagent system | | Partial composition (1) pH 8.0 | 2.0 | Control serum L | 95.1 |
| | | | | Control serum H | 94.5 |
| Three-reagent system | | Partial composition (1) pH 8.0 5 mmol/L trisodium citrate | 2.2 | Control serum L | 96.4 |
| | | | | Control serum H | 94.9 |

As is evident from Table 8, use of a stabilizer that gave an oxidation-reduction potential of ferrocyanide higher than 0.058 V upon mixing the stabilizer with the ferrocyanide caused small change in reagent blank even if pH was larger than 7. Use of the stabilizer that gave an oxidation-reduction potential of ferrocyanide higher than 0.058 V upon mixing the stabilizer with the ferrocyanide caused small change in reagent blank even when the chelating agent was added.

[Example 6: Change of reagent configuration]

(Preparation of 4-aminoantipyrine-containing liquid partial composition)

The following reagent components were dissolved in purified water so as to achieve the described concentrations. The solution was adjusted to pH 7.0 with sodium hydroxide to prepare a 4-aminoantipyrine-containing liquid partial composition.

50 mmol/L tris(hydroxymethyl)aminomethane (manufactured by FUJIFILM Wako Pure Chemical Corp.)
0.05 wt/vol% sodium azide (manufactured by Merck KGaA)
8 mmol/L 4-aminoantipyrine (manufactured by ACTEC Laboratories Inc.)
10 kU/mL protease for glycated albumin (manufactured by Asahi Kasei Pharma Corp.; obtained in accordance with the method described in Japanese Patent No. 4565807)
10 wt/vol% propylene glycol (manufactured by FUJIFILM Wako Pure Chemical Corp.)

(Preparation of Trinder reagent- and ferrocyanide-containing liquid partial composition)

The following reagent components were dissolved in purified water so as to achieve the described concentrations. The solution was adjusted to pH 7.5 with sodium hydroxide to prepare a Trinder reagent- and ferrocyanide-containing liquid partial composition.

50 mmol/L tris(hydroxymethyl)aminomethane (manufactured by FUJIFILM Wako Pure Chemical Corp.)
5 wt/vol% D(-)-sorbitol (manufactured by FUJIFILM Wako Pure Chemical Corp.)
0.05 wt/vol% sodium azide (manufactured by Merck KGaA)
10 mmol/L TOOS (manufactured by Dojindo Laboratories Co., Ltd.)
5 U/mL peroxidase (manufactured by Amano Enzyme Inc.)
20 U/mL ketoamine oxidase (manufactured by Asahi Kasei Pharma Corp.)
0.08 mmol/L potassium ferrocyanide (manufactured by Kokusan Chemical Co., Ltd.)

(Sample)

The following sample was provided.
Control serum H (manufactured by Asahi Kasei Pharma Corp.)

(Reagent preservation)

The prepared Trinder reagent- and ferrocyanide-containing liquid partial composition was preserved at 30°C for 14 days or 28 days, and the 4-aminoantipyrine-containing liquid partial composition was preserved at 5°C for 14 days or 28 days.

(Assay procedure)

To 180 µL of the 4-aminoantipyrine-containing liquid partial composition, 6 µL of the sample was added, and the mixture was reacted at 37°C for 5 minutes. Then, 60 µL of the Trinder reagent- and ferrocyanide-containing liquid partial composition was added thereto, and the mixture was further reacted at 37°C for 5 minutes. Change in absorbance was measured from absorbance Abs1 5 minutes after the sample to the 4-aminoantipyrine-containing liquid partial composition was added to absorbance Abs2 5 minutes after the Trinder reagent- and ferrocyanide-containing liquid partial composition was added to the mixed solution of the 4-aminoantipyrine-containing liquid partial composition and the sample. The change in absorbance, $A_C$, was given according to the following expression (21).

$$A_C = (Abs2) - (Abs1) \times 180 / 240 \quad (21)$$

wherein 180 represents the liquid quantity of the 4-aminoantipyrine-containing liquid partial composition, and 240 represents the total liquid quantity of the 4-aminoantipyrine-containing liquid partial composition and the Trinder reagent- and ferrocyanide-containing liquid partial composition.

Change in absorbance measured using the sample was defined as Acs, and change in absorbance measured using purified water instead of the sample was defined as a reagent blank Acb. A value obtained by subtracting the change in absorbance, Acb, of the reagent blank from the change in absorbance, Acs, measured using the sample was calculated as sensitivity ΔAbs according to the following expression (22).

$$\Delta Abs = Acs - Acb \quad (22)$$

The sensitivity of the partial composition preserved for 14 days was defined as ΔAbs14, and the sensitivity of the partial composition preserved for 28 days was defined as ΔAbs28. The sensitivity preservation rate was calculated as the ratio of ΔAbs28 to ΔAbs14 according to the following expression (23). The sensitivity preservation rate is shown in Table 9, together with the results about the reagent blank Acb of the partial composition preserved for 14 days.

Sensitivity preservation rate (%) = ΔAbs28 / ΔAbs14 × 100 (23)     (23)

[Table 9]

| Reagent type | Stabilizer contained in 4-aminoantipyrine-containing liquid partial composition | Reagent blank after 14-d preservation (mAbs) | Sample | Sensitivity preservation rate (%) |
|---|---|---|---|---|
| Two-reagent system | 10 wt/vol% propylene glycol | 0.2 | Control serum H | 107.7 |

As is evident from Table 9, it was found that when a stabilizer having a concentration that gives an oxidation-reduction potential of ferrocyanide higher than 0.058 V upon mixing the stabilizer with the ferrocyanide is used, the reagent blank is low.

[Example 7: Change in reagent blank depending on type of protease stabilizer and verification of long-term specimen sensitivity stability]

(Preparation of Trinder reagent-containing liquid partial composition)

The following reagent components were dissolved in purified water so as to achieve the described concentrations. The solution was adjusted to pH 7.6 with sodium hydroxide to prepare a Trinder reagent-containing liquid partial composition.

100 mmol/L N-tris(hydroxymethyl)methyl-2-aminoethanesulfonic acid (manufactured by Dojindo Laboratories Co., Ltd.)
0.05 wt/vol% sodium azide (manufactured by Merck KGaA)
1 mmol/L sodium ethylenediaminetetraacetate (manufactured by Kokusan Chemical Co., Ltd.)
6 wt/vol% D(-)-sorbitol (manufactured by FUJIFILM Wako Pure Chemical Corp.)
2 mmol/L N,N-bis(4-sulfobutyl)-3-methylaniline, disodium salt (manufactured by Dojindo Laboratories Co., Ltd.)
10 U/mL peroxidase (manufactured by Amano Enzyme Inc.)
30 U/mL ketoamine oxidase (manufactured by Asahi Kasei Pharma Corp.)

(Two-reagent system: Preparation of 4-aminoantipyrine- and ferrocyanide-containing liquid partial composition)

The following reagent components were dissolved in purified water so as to achieve the described concentrations. The solution was adjusted to pH 7.5 with sodium hydroxide to prepare a 4-aminoantipyrine- and ferrocyanide-containing liquid partial composition containing a stabilizer.

200 mmol/L 3,3-dimethylglutaric acid (manufactured by Tokyo Chemical Industry Co., Ltd.)
0.05 wt/vol% ProClin 300 (manufactured by Sigma-Aldrich Co., LLC)
5 mmol/L 4-aminoantipyrine (manufactured by ACTEC Laboratories Inc.)
0.1 mmol/L potassium ferrocyanide (manufactured by Kokusan Chemical Co., Ltd.)
500 U/mL catalase (manufactured by Nagase ChemteX Corp.)
40 kU/mL protease for glycated albumin (manufactured by Asahi Kasei Pharma Corp.; obtained in accordance with the method described in Japanese Patent No. 4565807)
Stabilizer having a concentration described later

(Two-reagent system: Stabilizer contained in 4-aminoantipyrine- and ferrocyanide-containing liquid partial composition)

30 wt/vol% propylene glycol (manufactured by FUJIFILM Wako Pure Chemical Corp.)
37.5 wt/vol% propylene glycol (manufactured by FUJIFILM Wako Pure Chemical Corp.)
50 wt/vol% trimethylene glycol (manufactured by FUJIFILM Wako Pure Chemical Corp.)
62.5 wt/vol% trimethylene glycol (manufactured by FUJIFILM Wako Pure Chemical Corp.)
50 wt/vol% ethylene glycol (manufactured by FUJIFILM Wako Pure Chemical Corp.)
62.5 wt/vol% ethylene glycol (manufactured by FUJIFILM Wako Pure Chemical Corp.)
0.6 wt/vol% 2-carboxyphenylboronic acid (manufactured by Tokyo Chemical Industry Co., Ltd.)
0.75 wt/vol% 2-carboxyphenylboronic acid (manufactured by Tokyo Chemical Industry Co., Ltd.)
30 wt/vol% dimethyl sulfoxide (manufactured by Nacalai Tesque, Inc.)
37.5 wt/vol% dimethyl sulfoxide (manufactured by Nacalai Tesque, Inc.)

(Three-reagent system: Preparation of 4-aminoantipyrine-containing liquid partial composition (1))

The following reagent components were dissolved in purified water so as to achieve the described concentrations. The solution was adjusted to pH 7.5 with sodium hydroxide to prepare a 4-aminoantipyrine-containing liquid partial composition (1) containing a stabilizer.

250 mmol/L 3,3-dimethylglutaric acid (manufactured by Tokyo Chemical Industry Co., Ltd.)
0.05 wt/vol% ProClin 300 (manufactured by Sigma-Aldrich Co., LLC)
6.25 mmol/L 4-aminoantipyrine (manufactured by ACTEC Laboratories Inc.)
625 U/mL catalase (manufactured by Nagase ChemteX Corp.)
50 kU/mL protease for glycated albumin (manufactured by Asahi Kasei Pharma Corp.; obtained in accordance with the method described in Japanese Patent No. 4565807)

Stabilizer having a concentration described later

(Three-reagent system: Stabilizer contained in 4-aminoantipyrine-containing liquid partial composition (1))

37.5 wt/vol% propylene glycol (manufactured by FUJIFILM Wako Pure Chemical Corp.)
50 wt/vol% propylene glycol (manufactured by FUJIFILM Wako Pure Chemical Corp.)
62.5 wt/vol% trimethylene glycol (manufactured by FUJIFILM Wako Pure Chemical Corp.)
62.5 wt/vol% ethylene glycol (manufactured by FUJIFILM Wako Pure Chemical Corp.)
0.75 wt/vol% 2-carboxyphenylboronic acid (manufactured by Tokyo Chemical Industry Co., Ltd.)
37.5 wt/vol% dimethyl sulfoxide (manufactured by Nacalai Tesque, Inc.)

(Three-reagent system: Preparation of ferrocyanide-containing liquid partial composition (1))

The following reagent components were dissolved in purified water so as to achieve the described concentrations. The solution was adjusted to pH 7.0 with sodium hydroxide to prepare a ferrocyanide-containing liquid partial composition (1).

100 mmol/L 3,3-dimethylglutaric acid (manufactured by Tokyo Chemical Industry Co., Ltd.)
0.05 wt/vol% ProClin 300 (manufactured by Sigma-Aldrich Co., LLC)
0.5 mmol/L potassium ferrocyanide (manufactured by Kokusan Chemical Co., Ltd.)

(Three-reagent system: Preparation of 4-aminoantipyrine-containing liquid partial composition (2))

The following reagent components were dissolved in purified water so as to achieve the described concentrations. The solution was adjusted to pH 7.0 with sodium hydroxide to prepare a 4-aminoantipyrine-containing liquid partial composition (2).

100 mmol/L 3,3-dimethylglutaric acid (manufactured by Tokyo Chemical Industry Co., Ltd.)
0.05 wt/vol% ProClin 300 (manufactured by Sigma-Aldrich Co., LLC)
25 mmol/L 4-aminoantipyrine (manufactured by ACTEC Laboratories Inc.)

(Three-reagent system: Preparation of ferrocyanide-containing liquid partial composition (2))

The following reagent components were dissolved in purified water so as to achieve the described concentrations. The solution was adjusted to pH 7.5 with sodium hydroxide to prepare a ferrocyanide-containing liquid partial composition (2) containing a stabilizer.

250 mmol/L 3,3-dimethylglutaric acid (manufactured by Tokyo Chemical Industry Co., Ltd.)
0.05 wt/vol% ProClin 300 (manufactured by Sigma-Aldrich Co., LLC)
0.125 mmol/L potassium ferrocyanide (manufactured by Kokusan Chemical Co., Ltd.)
625 U/mL catalase (manufactured by Nagase ChemteX Corp.)
50 kU/mL protease for glycated albumin (manufactured by Asahi Kasei Pharma Corp.; obtained in accordance with the method described in Japanese Patent No. 4565807)
Stabilizer having a concentration described later

(Three-reagent system: Stabilizer contained in ferrocyanide-containing liquid partial composition (2))

37.5 wt/vol% propylene glycol (manufactured by FUJIFILM Wako Pure Chemical Corp.)
37.5 wt/vol% dimethyl sulfoxide (manufactured by Nacalai Tesque, Inc.)

(Sample)

The following samples were provided.

Control serum L (manufactured by Asahi Kasei Pharma Corp.)
Control serum H (manufactured by Asahi Kasei Pharma Corp.)

(Reagent preservation)

The prepared Trinder reagent-containing liquid partial composition, 4-aminoantipyrine- and ferrocyanide-containing partial composition, 4-aminoantipyrine-containing liquid partial composition, and ferrocyanide-containing liquid partial composition were preserved at 5°C for 28 days or 1 year.

(Assay preparation)

The 4-aminoantipyrine-containing liquid partial composition (1) and the ferrocyanide-containing liquid partial composition (1) of the three-reagent system were mixed at a volume ratio of 4:1 to prepare a 4-aminoantipyrine- and ferrocyanide-containing liquid partial composition (1) for use in assay.

The 4-aminoantipyrine-containing liquid partial composition (2) and the ferrocyanide-containing liquid partial composition (2) of the three-reagent system were mixed at a volume ratio of 1:4 to prepare a 4-aminoantipyrine- and ferrocyanide-containing liquid partial composition (2) for use in assay.

(Assay procedure)

To 180 μL of the Trinder reagent-containing liquid partial composition, 4.5 μL of the sample was added, and the mixture was reacted at 37°C for 5 minutes. Then, 45 μL of the 4-aminoantipyrine- and ferrocyanide-containing liquid partial composition was added thereto, and the mixture was further reacted at 37°C for 5 minutes. Change in absorbance was measured from absorbance Abs1 5 minutes after the sample was added to the Trinder reagent-containing liquid partial composition to absorbance Abs2 5 minutes after the 4-aminoantipyrine- and ferrocyanide-containing liquid partial composition was added to the mixed solution of the Trinder reagent-containing liquid partial composition and the sample. The change in absorbance, $A_C$, was given according to the following expression (24).

$$A_C = (Abs2) - (Abs1) \times 180 / 225 \quad (24)$$

wherein 180 represents the liquid quantity of the Trinder reagent-containing liquid partial composition, and 225 represents the total liquid quantity of the Trinder reagent-containing liquid partial composition and the 4-aminoantipyrine- and ferrocyanide-containing liquid partial composition.

Change in absorbance measured using the sample was defined as Acs, and change in absorbance measured using purified water instead of the sample was defined as a reagent blank Acb. A value obtained by subtracting the change in absorbance, Acb, of the reagent blank from the change in absorbance, Acs, measured using the sample was calculated as sensitivity ΔAbs according to the following expression (25).

$$\Delta Abs = Acs - Acb \quad (25)$$

The sensitivity of the partial composition preserved for 28 days was defined as ΔAbs28, and the sensitivity of the partial composition preserved for 1 year was defined as ΔAbs365. The long-term sensitivity preservation rate was calculated as the ratio of ΔAbs365 to ΔAbs28 according to the following expression (26). The long-term sensitivity preservation rate is shown in Table 10, together with the results about the reagent blank of the partial composition preserved for 1 year.

Sensitivity preservation rate (%) = ΔAbs365 / ΔAbs28 × 100 (26)                                                    (26)

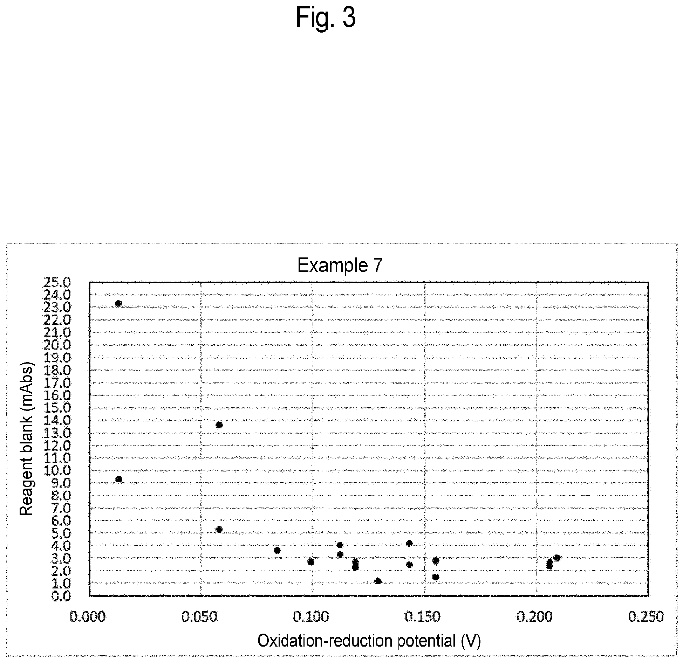

Table 11 and Figure 3 show the relationship between the reagent blank and the oxidation-reduction potential of ferrocyanide after preservation for 1 year of the partial composition. The oxidation-reduction potential value at the concentration when the stabilizer and the ferrocyanide were mixed was used as the oxidation-reduction potential of ferrocyanid. For example, when the stabilizer and the ferrocyanide were contained in the same partial composition (the 4-aminoantipyrine- and ferrocyanide-containing liquid partial composition of the two-reagent system, and the ferrocyanide-containing liquid partial composition (2) of the three-reagent system), the oxidation-reduction potential value at the stabilizer concentration in the partial composition was used. When the stabilizer and the ferrocyanide were contained in separate partial compositions (the 4-aminoantipyrine-containing liquid partial composition (1) and the ferrocyanide-containing liquid partial composition (1) of the three-reagent system), the oxidation-reduction potential value at a stabilizer concentration in a mixture of the 4-aminoantipyrine-containing liquid partial composition (1) and the ferrocyanide-containing liquid partial composition (1) was used.

[Table 10-1]

| Reagent type | 4-Aminoantipyrine- and ferrocyanide-containing liquid partial composition | Reagent blank after 1-year preservation (mAbs) | Sample | Long-term sensitivity preservation rate (%) |
|---|---|---|---|---|
| Two-reagent system | 30 wt/vol% propylene glycol | 2.8 | Control serum L | 85.7 |
| | | | Control serum H | 87.2 |
| Two-reagent system | 37.5 wt/vol% propylene glycol | 2.5 | Control serum L | 86.8 |
| | | | Control serum H | 89.2 |
| Three-reagent system | 4-Aminoantipyrine-containing liquid partial composition (1) 37.5 wt/vol% propylene glycol | 1.5 | Control serum L | 102.0 |
| | Ferrocyanide-containing liquid partial composition (1) | | Control serum H | 99.3 |
| Three-reagent system | 4-Aminoantipyrine-containing liquid partial composition (1) 50 wt/vol% propylene glycol | 1.2 | Control serum L | 101.7 |
| | Ferrocyanide-containing liquid partial composition (1) | | Control serum H | 99.4 |

[Table 10-2]

| Three-reagent system | 4-Aminoantipyrine-containing liquid partial composition (2) | 4.2 | Control serum L | 89.6 |
|---|---|---|---|---|
| | Ferrocyanide-containing liquid partial composition (2) 37.5 wt/vol% propylene glycol | | Control serum H | 91.4 |
| Two-reagent system | 50 wt/vol% trimethylene glycol | 3.3 | Control serum L | 85.8 |
| | | | Control serum H | 90.0 |
| Two-reagent system | 62.5 wt/vol% trimethylene glycol | 3.6 | Control serum L | 88.0 |
| | | | Control serum H | 91.3 |
| Three-reagent system | 4-Aminoantipyrine-containing liquid partial composition (1) 62.5 wt/vol% trimethylene glycol | 4.0 | Control serum L | 99.3 |
| | Ferrocyanide-containing liquid partial composition (1) | | Control serum H | 98.6 |

[Table 10-3]

| Two-reagent system | 50 wt/vol% ethylene glycol | 2.3 | Control serum L | 81.9 |
|---|---|---|---|---|
| | | | Control serum H | 86.4 |
| Two-reagent system | 62.5 wt/vol% ethylene glycol | 2.7 | Control serum L | 77.8 |
| | | | Control serum H | 85.4 |
| Three-reagent system | 4-Aminoantipyrine-containing liquid partial composition (1) 62.5 wt/vol% ethylene glycol | 2.7 | Control serum L | 96.8 |
| | Ferrocyanide-containing liquid partial composition (1) | | Control serum H | 97.2 |

(continued)

| Two-reagent system | 0.6 wt/vol% 2-carboxyphenylboronic acid | 2.7 | Control serum L | 86.8 |
|---|---|---|---|---|
| | | | Control serum H | 90.5 |

[Table 10-4]

| Two-reagent system | 0.75 wt/vol% 2-carboxyphenylboronic acid | 3.0 | Control serum L | 86.4 |
|---|---|---|---|---|
| | | | Control serum H | 89.9 |
| Three-reagent system | 4-Aminoantipyrine-containing liquid partial composition (1) 0.75 wt/vol% 2-carboxyphenylboronic acid | 2.4 | Control serum L | 88.5 |
| | Ferrocyanide-containing liquid partial composition (1) | | Control serum H | 91.2 |
| Two-reagent system | 30 wt/vol% dimethyl sulfoxide | 5.3 | Control serum L | 74.4 |
| | | | Control serum H | 79.5 |
| Two-reagent system | 37.5 wt/vol% dimethyl sulfoxide | 9,3 | Control serum L | 73.7 |
| | | | Control serum H | 78.0 |

[Table 10-5]

| Three-reagent system | 4-Aminoantipyrine-containing liquid partial composition (1) 37.5 wt/vol% dimethyl sulfoxide | 13.6 | Control serum L | 94.0 |
|---|---|---|---|---|
| | Ferrocyanide-containing liquid partial composition (1) | | Control serum H | 95.3 |
| Three-reagent system | 4-Aminoantipyrine-containing liquid partial composition (2) | 23.3 | Control serum L | 90.8 |
| | Ferrocyanide-containing liquid partial composition (2) 37.5 wt/vol% dimethyl sulfoxide | | Control serum H | 92.0 |

[Table 11-1]

| Reagent type | 4-Aminoantipyrine- and ferrocyanide-containing liquid partial composition | Reagent blank after 1-year preservation (mAbs) | Oxidation-reduction potential (V) |
|---|---|---|---|
| Two-reagent system | 30 wt/vol% propylene glycol | 2.8 | 0.155 |
| Two-reagent system | 37.5 wt/vol% propylene glycol | 2.5 | 0.143 |
| Three-reagent system | 4-Aminoantipyrine-containing liquid partial composition (1) 37.5 wt/vol% propylene glycol | 1.5 | 0.155 |
| | Ferrocyanide-containing liquid partial composition (1) | | |
| Three-reagent system | 4-Aminoantipyrine-containing liquid partial composition (1) 50 wt/vol% propylene glycol | 1.2 | 0.129 |
| | Ferrocyanide-containing liquid partial composition (1) | | |
| Three-reagent system | 4-Aminoantipyrine-containing liquid partial composition (2) | 4.2 | 0.143 |
| | Ferrocyanide-containing liquid partial composition (2) 37.5 wt/vol% propylene glycol | | |
| Two-reagent system | 50 wt/vol% trimethylene glycol | 3.3 | 0.112 |
| Two-reagent system | 62.5 wt/vol% trimethylene glycol | 3.6 | 0.084 |

[Table 11-2]

| Three-reagent system | 4-Aminoantipyrine-containing liquid partial composition (1) 62.5 wt/vol% trimethylene glycol | 4.0 | 0.112 |
|---|---|---|---|
| | Ferrocyanide-containing liquid partial composition (1) | | |
| Two-reagent system | 50 wt/vol% ethylene glycol | 2.3 | 0.119 |
| Two-reagent system | 62.5 wt/vol% ethylene glycol | 2.7 | 0.099 |
| Three-reagent system | 4-Aminoantipyrine-containing liquid partial composition (1) 62.5 wt/vol% ethylene glycol | 2.7 | 0.119 |
| | Ferrocyanide-containing liquid partial composition (1) | | |
| Two-reagent system | 0.6 wt/vol% 2-carboxyphenylboronic acid | 2.7 | 0.206 |
| Two-reagent system | 0.75 wt/vol% 2-carboxyphenylboronic acid | 3.0 | 0.209 |
| Three-reagent system | 4-Aminoantipyrine-containing liquid partial composition (1) 0.75 wt/vol% 2-carboxyphenylboronic acid | 2.4 | 0.206 |
| | Ferrocyanide-containing liquid partial composition (1) | | |
| Two-reagent system | 30 wt/vol% dimethyl sulfoxide | 5.3 | 0.058 |

[Table 11-3]

| Two-reagent system | 37.5 wt/vol% dimethyl sulfoxide | 9.3 | 0.013 |
|---|---|---|---|
| Three-reagent system | 4-Aminoantipyrine-containing liquid partial composition (1) 37.5 wt/vol% dimethyl sulfoxide | 13.6 | 0.058 |
| | Ferrocyanide-containing liquid partial composition (1) | | |
| Three-reagent system | 4-Aminoantipyrine-containing liquid partial composition (2) | 23.3 | 0.013 |
| | Ferrocyanide-containing liquid partial composition (2) 37.5 wt/vol% dimethyl sulfoxide | | |

The results about the reagent preserved for a long period are also similar to those in Example 2. Specifically, as is evident from Table 10, Table 11, and Figure 3, it was found that both in the two-reagent system and in the three-reagent system, the reagent blank-suppressing effect is exhibited by a stabilizer that increases the oxidation-reduction potential of the ferrocyanide above 0.058 V upon mixing the stabilizer with the ferrocyanide. It was also found that for all the stabilizers, the change of the two-reagent system to the three-reagent system improves a specimen sensitivity preservation rate. A two-reagent system using dimethyl sulfoxide as the stabilizer decreased the sensitivity preservation rate to 80% or less. Accordingly, it was found that use of a stabilizer other than dimethyl sulfoxide is also more preferred in terms of sensitivity preservation rates.

[Example 8: Verification of long-term stability of effect of chelating agent addition]

(Preparation of Trinder reagent-containing liquid partial composition)

The following reagent components were dissolved in purified water so as to achieve the described concentrations. The solution was adjusted to pH 7.6 with sodium hydroxide to prepare a Trinder reagent-containing liquid partial composition.

100 mmol/L N-tris(hydroxymethyl)methyl-2-aminoethanesulfonic acid (manufactured by Dojindo Laboratories Co., Ltd.)
0.05 wt/vol% sodium azide (manufactured by Merck KGaA)
1 mmol/L sodium ethylenediaminetetraacetate (manufactured by Kokusan Chemical Co., Ltd.)
6 wt/vol% D(-)-sorbitol (manufactured by FUJIFILM Wako Pure Chemical Corp.)
2 mmol/L N,N-bis(4-sulfobutyl)-3-methylaniline, disodium salt (manufactured by Dojindo Laboratories Co., Ltd.)
10 U/mL peroxidase (manufactured by Amano Enzyme Inc.)
30 U/mL ketoamine oxidase (manufactured by Asahi Kasei Pharma Corp.)

(Two-reagent system: Preparation of 4-aminoantipyrine- and ferrocyanide-containing liquid partial composition)

The following reagent components were dissolved in purified water so as to achieve the described concentrations. The solution was adjusted to pH 7.5 with sodium hydroxide to prepare a 4-aminoantipyrine- and ferrocyanide-containing liquid partial composition containing propylene glycol as a stabilizer, the partial composition containing trisodium citrate or containing no trisodium citrate.

200 mmol/L 3,3-dimethylglutaric acid (manufactured by Tokyo Chemical Industry Co., Ltd.)
0.05 wt/vol% ProClin 300 (manufactured by Sigma-Aldrich Co., LLC)
5 mmol/L 4-aminoantipyrine (manufactured by ACTEC Laboratories Inc.)
0.1 mmol/L potassium ferrocyanide (manufactured by Kokusan Chemical Co., Ltd.)
500 U/mL catalase (manufactured by Nagase ChemteX Corp.)
40 kU/mL protease for glycated albumin (manufactured by Asahi Kasei Pharma Corp.; obtained in accordance with the method described in Japanese Patent No. 4565807)
1 mmol/L trisodium citrate (manufactured by FUJIFILM Wako Pure Chemical Corp.)
Stabilizer having a concentration described later

(Two-reagent system: Stabilizer contained in 4-aminoantipyrine- and ferrocyanide-containing liquid partial composition)

30 wt/vol% propylene glycol (manufactured by FUJIFILM Wako Pure Chemical Corp.)
37.5 wt/vol% propylene glycol (manufactured by FUJIFILM Wako Pure Chemical Corp.)

(Three-reagent system: Preparation of 4-aminoantipyrine-containing liquid partial composition (1))

The following reagent components were dissolved in purified water so as to achieve the described concentrations. The solution was adjusted to pH 7.5 with sodium hydroxide to prepare a 4-aminoantipyrine-containing liquid partial composition (1) containing a stabilizer, the partial composition containing trisodium citrate or containing no trisodium citrate.

250 mmol/L 3,3-dimethylglutaric acid (manufactured by Tokyo Chemical Industry Co., Ltd.)
0.05 wt/vol% ProClin 300 (manufactured by Sigma-Aldrich Co., LLC)
6.25 mmol/L 4-aminoantipyrine (manufactured by ACTEC Laboratories Inc.)
625 U/mL catalase (manufactured by Nagase ChemteX Corp.)
50 kU/mL protease for glycated albumin (manufactured by Asahi Kasei Pharma Corp.; obtained in accordance with the method described in Japanese Patent No. 4565807)
1.25 mmol/L trisodium citrate (manufactured by FUJIFILM Wako Pure Chemical Corp.)
Stabilizer having a concentration described later

(Three-reagent system: Stabilizer contained in 4-aminoantipyrine-containing liquid partial composition (1))

37.5 wt/vol% propylene glycol (manufactured by FUJIFILM Wako Pure Chemical Corp.)
62.5 wt/vol% trimethylene glycol (manufactured by FUJIFILM Wako Pure Chemical Corp.)
62.5 wt/vol% ethylene glycol (manufactured by FUJIFILM Wako Pure Chemical Corp.)
0.75 wt/vol% 2-carboxyphenylboronic acid (manufactured by Tokyo Chemical Industry Co., Ltd.)

(Three-reagent system: Preparation of ferrocyanide-containing liquid partial composition (1))

The following reagent components were dissolved in purified water so as to achieve the described concentrations. The solution was adjusted to pH 7.0 with sodium hydroxide to prepare a ferrocyanide-containing liquid partial composition (1), the partial composition containing trisodium citrate or containing no trisodium citrate.

100 mmol/L 3,3-dimethylglutaric acid (manufactured by Tokyo Chemical Industry Co., Ltd.)
0.05 wt/vol% ProClin 300 (manufactured by Sigma-Aldrich Co., LLC)

0.5 mmol/L potassium ferrocyanide (manufactured by Kokusan Chemical Co., Ltd.)
5 mmol/L trisodium citrate (manufactured by FUJIFILM Wako Pure Chemical Corp.)

(Three-reagent system: Preparation of 4-aminoantipyrine-containing liquid partial composition (2))

The following reagent components were dissolved in purified water so as to achieve the described concentrations. The solution was adjusted to pH 7.0 with sodium hydroxide to prepare a partial composition containing 4-aminoantipyrine-containing liquid partial composition (2).

100 mmol/L 3,3-dimethylglutaric acid (manufactured by Tokyo Chemical Industry Co., Ltd.)
0.05 wt/vol% ProClin 300 (manufactured by Sigma-Aldrich Co., LLC)
25 mmol/L 4-aminoantipyrine (manufactured by ACTEC Laboratories Inc.)

(Three-reagent system: Preparation of ferrocyanide-containing liquid partial composition (2))

The following reagent components were dissolved in purified water so as to achieve the described concentrations. The solution was adjusted to pH 7.5 with sodium hydroxide to prepare a ferrocyanide-containing liquid partial composition (2) containing propylene glycol as a stabilizer, the partial composition containing trisodium citrate or containing no trisodium citrate.

250 mmol/L 3,3-dimethylglutaric acid (manufactured by Tokyo Chemical Industry Co., Ltd.)
0.05 wt/vol% ProClin 300 (manufactured by Sigma-Aldrich Co., LLC)
0.125 mmol/L potassium ferrocyanide (manufactured by Kokusan Chemical Co., Ltd.)
625 U/mL catalase (manufactured by Nagase ChemteX Corp.)
50 kU/mL protease for glycated albumin (manufactured by Asahi Kasei Pharma Corp.; obtained in accordance with the method described in Japanese Patent No. 4565807)
1.25 mmol/L trisodium citrate (manufactured by FUJIFILM Wako Pure Chemical Corp.)
37.5 wt/vol% propylene glycol (manufactured by FUJIFILM Wako Pure Chemical Corp.)

(Sample)

The following samples were provided.

Control serum L (manufactured by Asahi Kasei Pharma Corp.)
Control serum H (manufactured by Asahi Kasei Pharma Corp.)

(Reagent preservation)

The prepared Trinder reagent-containing liquid partial composition, 4-aminoantipyrine- and ferrocyanide-containing liquid partial composition, 4-aminoantipyrine-containing liquid partial composition, and ferrocyanide-containing liquid partial composition were preserved at 5°C for 28 days or 1 year.

(Assay preparation)

The 4-aminoantipyrine-containing liquid partial composition (1) and the ferrocyanide-containing liquid partial composition (1) of the three-reagent system were mixed at a volume ratio of 4:1 to prepare a 4-aminoantipyrine- and ferrocyanide-containing liquid partial composition (1) for use in assay.
The 4-aminoantipyrine-containing liquid partial composition (2) and the ferrocyanide-containing liquid partial composition (2) of the three-reagent system were mixed at a volume ratio of 1:4 to prepare a 4-aminoantipyrine- and ferrocyanide-containing liquid partial composition (2) for use in assay.

(Assay procedure)

To 180 μL of the Trinder reagent-containing liquid partial composition, 4.5 μL of the sample was added, and the mixture was reacted at 37°C for 5 minutes. Then, 45 μL of the 4-aminoantipyrine- and ferrocyanide-containing liquid partial composition was added thereto, and the mixture was further reacted at 37°C for 5 minutes. Change in absorbance was measured from absorbance Abs1 5 minutes after the sample was added to the Trinder reagent-containing liquid partial composition to absorbance Abs2 5 minutes after the 4-aminoantipyrine- and ferrocyanide-containing liquid partial composition was added to the mixed solution of the Trinder reagent-containing liquid partial composition and the sample. The change in absorbance, $A_C$, was given according to the following expression (27).

$$A_C = (Abs2) - (Abs1) \times 180 / 225 \quad (27)$$

wherein 180 represents the liquid quantity of the Trinder reagent-containing liquid partial composition, and 225 represents the total liquid quantity of the Trinder reagent-containing liquid partial composition and the 4-aminoantipyrine- and ferrocyanide-containing liquid partial composition.

Change in absorbance measured using the sample was defined as Acs, and change in absorbance measured using purified water instead of the sample was defined as a reagent blank Acb. A value obtained by subtracting the change in absorbance, Acb, of the reagent blank from the change in absorbance, Acs, measured using the sample was calculated as sensitivity $\Delta$Abs according to the following expression (28).

$$\Delta Abs = Acs - Acb \quad (28)$$

The sensitivity of the partial composition preserved for 28 days was defined as $\Delta$Abs28, and the sensitivity of the partial composition preserved for 1 year was defined as $\Delta$Abs365. The long-term sensitivity preservation rate was calculated as the ratio of $\Delta$Abs365 to $\Delta$Abs28 according to the following expression (29). The long-term sensitivity preservation rate is shown in Table 12, together with the results about the reagent blank of the partial composition preserved for 1 year.

Long-term sensitivity preservation rate (%) = $\Delta$Abs365 / $\Delta$Abs28 $\times$ 100 (29)      (29)

[Table 12-1]

| Reagent type | 4-Aminoantipyrine-containing liquid partial composition | Ferrocyanide-containing liquid partial composition | Reagent blank after 1-year preservation (mAbs) | Sample | Long-term sensitivity preservation rate (%) |
|---|---|---|---|---|---|
| Two-reagent system | 30 wt/vol% propylene glycol | | 2.8 | Control serum L | 85.7 |
| | | | | Control serum H | 87.2 |
| Two-reagent system | 30 wt/vol% propylene glycol 1 mmol/L trisodium citrate | | 2.0 | Control serum L | 93.9 |
| | | | | Control serum H | 93.4 |
| Two-reagent system | 37.5 wt/vol% propylene glycol | | 2.5 | Control serum L | 86.8 |
| | | | | Control serum H | 89.2 |
| Two-reagent system | 37.5 wt/vol% propylene glycol 1 mmol/L trisodium citrate | | 1.9 | Control serum L | 97.3 |
| | | | | Control serum H | 96.7 |
| Three-reagent system | Partial composition (1) 37.5 wt/vol% propylene glycol | Partial composition (1) | 1.5 | Control serum L | 102.0 |
| | | | | Control serum H | 99.3 |
| Three-reagent system | | Partial composition (1) 5 mmol/L trisodium citrate | 1.7 | Control serum L | 97.0 |
| | | | | Control serum H | 96.6 |

[Table 12-2]

| Three-reagent system | Partial composition (1) 37.5 wt/vol% propylene glycol 1.25 mmol/L trisodium citrate | Partial composition (1) | 1.5 | Control serum L | 101.9 |
| | | | | Control serum H | 100.2 |
| Three-reagent system | | Partial composition (1) 5 mmol/L trisodium citrate | 1.4 | Control serum L | 105.7 |
| | | | | Control serum H | 101.9 |
| Three-reagent system | Partial composition (2) | Partial composition (2) 37.5 wt/vol% propylene glycol | 4.2 | Control serum L | 89.6 |
| | | | | Control serum H | 91.4 |
| Three-reagent system | | Partial composition (2) 37.5 wt/vol% propylene glycol 1.25 mmol/L trisodium citrate | 4.1 | Control serum L | 89.6 |
| | | | | Control serum H | 91.3 |
| Three-reagent system | Partial composition (1) 62.5 wt/vol% trimethylene glycol | Partial composition (1) | 4.0 | Control serum L | 99.3 |
| | | | | Control serum H | 98.6 |
| Three-reagent system | | Partial composition (1) 5 mmol/L trisodium citrate | 3.2 | Control serum L | 102.0 |
| | | | | Control serum H | 99.8 |

[Table 12-3]

| Three-reagent system | Partial composition (1) 62.5 wt/vol% trimethylene glycol 1.25 mmol/L trisodium citrate | Partial composition (1) | 3.2 | ' Control serum L | 102.3 |
| | | | | Control serum H | 101.0 |
| Three-reagent system | | Partial composition (1) 5 mmol/L trisodium citrate | 2.6 | Control serum L | 106.9 |
| | | | | Control serum H | 102.8 |
| Three-reagent system | Partial composition (1) 62.5 wt/vol% ethylene glycol | Partial composition (1) | 2.7 | Control serum L | 96.8 |
| | | | | Control serum H | 97.2 |
| Three-reagent system | | Partial composition (1) 5 mmol/L trisodium citrate | 2.3 | Control serum L | 99.1 |
| | | | | Control serum H | 98.1 |
| Three-reagent system | Partial composition (1) 62.5 wt/vol% glycol ethylene 1.25 mmol/L trisodium citrate | Partial composition (1) | 2.2 | Control serum L | 104.6 |
| | | | | Control serum H | 101.7 |
| Three-reagent system | | Partial composition (1) 5 mmol/L trisodium citrate | 2.0 | Control serum L | 107.1 |
| | | | | Control serum H | 103.2 |

[Table 12-4]

| Three-reagent system | Partial composition (1) 0.75 wt/vol% 2-carboxyphenylboronic acid | Partial composition (1) | 2.4 | Control serum L | 88.5 |
|---|---|---|---|---|---|
| | | | | Control serum H | 91.2 |
| Three-reagent system | | Partial composition (1) 5 mmol/L trisodium citrate | 2.2 | Control serum L | 90.1 |
| | | | | Control serum H | 92.0 |
| Three-reagent system | Partial composition (1) 0.75 wt/vol% 2-carboxyphenylboronic acid 1.25 mmol/L trisodium citrate | Partial composition (1) | 2.6 | Control serum L | 90.5 |
| | | | | Control serum H | 92.8 |
| Three-reagent system | | Partial composition (1) 5 mmol/L trisodium citrate | 2.4 | Control serum L | 93.2 |
| | | | | Control serum H | 95.1 |

As is evident from Table 12, when a stabilizer that gave an oxidation-reduction potential of ferrocyanide higher than 0.058 V upon mixing the stabilizer with the ferrocyanide was used, the reagent blank without the addition of the chelating agent (trisodium citrate) was almost the same as the reagent blank in the case of adding the chelating agent (trisodium citrate).

[Example 9: Verification of long-term stability of effect of pH and chelating agent type]

(Preparation of Trinder reagent-containing liquid partial composition)

The following reagent components were dissolved in purified water so as to achieve the described concentrations. The solution was adjusted to pH 7.6 with sodium hydroxide to prepare a Trinder reagent-containing liquid partial composition.

100 mmol/L N-tris(hydroxymethyl)methyl-2-aminoethanesulfonic acid (manufactured by Dojindo Laboratories Co., Ltd.)
0.05 wt/vol% sodium azide (manufactured by Merck KGaA)
1 mmol/L sodium ethylenediaminetetraacetate (manufactured by Kokusan Chemical Co., Ltd.)
6 wt/vol% D(-)-sorbitol (manufactured by FUJIFILM Wako Pure Chemical Corp.)
2 mmol/L N,N-bis(4-sulfobutyl)-3-methylaniline, disodium salt (manufactured by Dojindo Laboratories Co., Ltd.)
10 U/mL peroxidase (manufactured by Amano Enzyme Inc.)
30 U/mL ketoamine oxidase (manufactured by Asahi Kasei Pharma Corp.)

(Three-reagent system: Preparation of 4-aminoantipyrine-containing liquid partial composition)

The following reagent components were dissolved in purified water so as to achieve the described concentrations. The solution was adjusted to pH 7.5 with sodium hydroxide to prepare a 4-aminoantipyrine-containing liquid partial composition containing propylene glycol as a stabilizer.

250 mmol/L 3,3-dimethylglutaric acid (manufactured by Tokyo Chemical Industry Co., Ltd.)
0.05 wt/vol% ProClin 300 (manufactured by Sigma-Aldrich Co., LLC)
6.25 mmol/L 4-aminoantipyrine (manufactured by ACTEC Laboratories Inc.)
625 U/mL catalase (manufactured by Nagase ChemteX Corp.)
50 kU/mL protease for glycated albumin (manufactured by Asahi Kasei Pharma Corp.; obtained in accordance with the method described in Japanese Patent No. 4565807)
50 wt/vol% propylene glycol (manufactured by FUJIFILM Wako Pure Chemical Corp.)

(Three-reagent system: Preparation of ferrocyanide-containing liquid partial composition (1))

The following reagent components were dissolved in purified water so as to achieve the described concentrations. The solution was pH-adjusted with sodium hydroxide to prepare a ferrocyanide-containing liquid partial composition (1) having distinctive pH.

> 100 mmol/L 3,3-dimethylglutaric acid (manufactured by Tokyo Chemical Industry Co., Ltd.)
> 0.05 wt/vol% ProClin 300 (manufactured by Sigma-Aldrich Co., LLC)
> 0.5 mmol/L potassium ferrocyanide (manufactured by Kokusan Chemical Co., Ltd.)
> Adjusted to pH of a concentration described later

(pH of ferrocyanide-containing liquid partial composition (1))

> pH 6.5
> pH 7.0
> pH 7.5
> pH 8.0

(Three-reagent system: Preparation of ferrocyanide-containing liquid partial composition (2))

The following reagent components were dissolved in purified water so as to achieve the described concentrations. The solution was adjusted to pH 7.0 with sodium hydroxide to prepare a ferrocyanide-containing liquid partial composition (2) containing a chelating agent.

> 100 mmol/L 3,3-dimethylglutaric acid (manufactured by Tokyo Chemical Industry Co., Ltd.)
> 0.05 wt/vol% ProClin 300 (manufactured by Sigma-Aldrich Co., LLC)
> 0.5 mmol/L potassium ferrocyanide (manufactured by Kokusan Chemical Co., Ltd.)
> Chelating agent having a concentration described later

(Chelating agent contained in ferrocyanide-containing liquid partial composition (2))

> 5 mmol/L sodium ethylenediaminetetraacetate (manufactured by Kokusan Chemical Co., Ltd., abbreviation: EDTA)
> 5 mmol/L glycol ether diaminetetraacetic acid (manufactured by Dojindo Laboratories Co., Ltd., abbreviation: EGTA)
> 5 mmol/L N-(2-hydroxyethyl)iminodiacetic acid (manufactured by Dojindo Laboratories Co., Ltd., abbreviation: HIDA)
> 5 mmol/L nitrilotris(methylphosphonic acid), trisodium salt (manufactured by Dojindo Laboratories Co., Ltd., abbreviation: NTPO)
> 5 mmol/L nitrilotriacetic acid (manufactured by Dojindo Laboratories Co., Ltd., abbreviation: NTA)
> 5 mmol/L trans-1,2-diaminocyclohexane-N,N,N,N-tetraacetic acid, monohydrate (manufactured by Dojindo Laboratories Co., Ltd., abbreviation: CyDTA)
> 5 mmol/L iminodiacetic acid (manufactured by Dojindo Laboratories Co., Ltd., abbreviation: IDA)
> 5 mmol/L diethylenetriamine-N,N,N',N",N"-pentaacetic acid (manufactured by Dojindo Laboratories Co., Ltd., abbreviation: DTPA)
> 5 mmol/L 1,3-diamino-2-propanol-N,N,N',N'-tetraacetic acid (manufactured by Dojindo Laboratories Co., Ltd., abbreviation: DPTA-OH)
> 5 mmol/L sodium gluconate (manufactured by FUJIFILM Wako Pure Chemical Corp.)
> 5 mmol/L sodium L(-)-malate (manufactured by FUJIFILM Wako Pure Chemical Corp.)
> 5 mmol/L succinic acid (manufactured by Kanto Chemical Co., Inc.)
> 5 mmol/L trisodium citrate (manufactured by FUJIFILM Wako Pure Chemical Corp.)
> 5 mmol/L salicylic acid (manufactured by Nacalai Tesque, Inc.)
> 5 mmol/L potassium (+)-tartrate (manufactured by FUJIFILM Wako Pure Chemical Corp.)
> 5 mmol/L N-[tris(hydroxymethyl)methyl]glycine (manufactured by Dojindo Laboratories Co., Ltd., abbreviation: Tricine)
> 5 mmol/L N,N-bis(2-hydroxyethyl)glycine (manufactured by Dojindo Laboratories Co., Ltd., abbreviation: Bicine)

(Sample)

The following samples were provided.

Control serum L (manufactured by Asahi Kasei Pharma Corp.)
Control serum H (manufactured by Asahi Kasei Pharma Corp.)

(Reagent preservation)

The prepared Trinder reagent-containing liquid partial composition, 4-aminoantipyrine-containing liquid partial composition, and ferrocyanide-containing liquid partial compositions (1) and (2) were preserved at 5°C for 28 days or 1 year.

(Assay preparation)

The 4-aminoantipyrine-containing liquid partial composition and the ferrocyanide-containing liquid partial composition (1) or (2) of the three-reagent system were mixed at a volume ratio of 4:1 to prepare a 4-aminoantipyrine- and ferrocyanide-containing liquid partial composition for use in assay.

(Assay procedure)

To 180 μL of the Trinder reagent-containing liquid partial composition, 4.5 μL of the sample was added, and the mixture was reacted at 37°C for 5 minutes. Then, 45 μL of the 4-aminoantipyrine- and ferrocyanide-containing liquid partial composition was added thereto, and the mixture was further reacted at 37°C for 5 minutes. Change in absorbance was measured from absorbance Abs1 5 minutes after the sample was added to the Trinder reagent-containing liquid partial composition to absorbance Abs2 5 minutes after the 4-aminoantipyrine- and ferrocyanide-containing liquid partial composition was added to the mixed solution of the Trinder reagent-containing liquid partial composition and the sample. The change in absorbance, $A_C$, was given according to the following expression (30).

$$A_C = (Abs2) - (Abs1) \times 180 / 225 \quad (30)$$

wherein 180 represents the liquid quantity of the Trinder reagent-containing liquid partial composition, and 225 represents the total liquid quantity of the Trinder reagent-containing liquid partial composition and the 4-aminoantipyrine- and ferrocyanide-containing liquid partial composition.

Change in absorbance measured using the sample was defined as Acs, and change in absorbance measured using purified water instead of the sample was defined as a reagent blank Acb. A value obtained by subtracting the change in absorbance, Acb, of the reagent blank from the change in absorbance, Acs, measured using the sample was calculated as sensitivity ΔAbs according to the following expression (31).

$$\Delta Abs = Acs - Acb \quad (31)$$

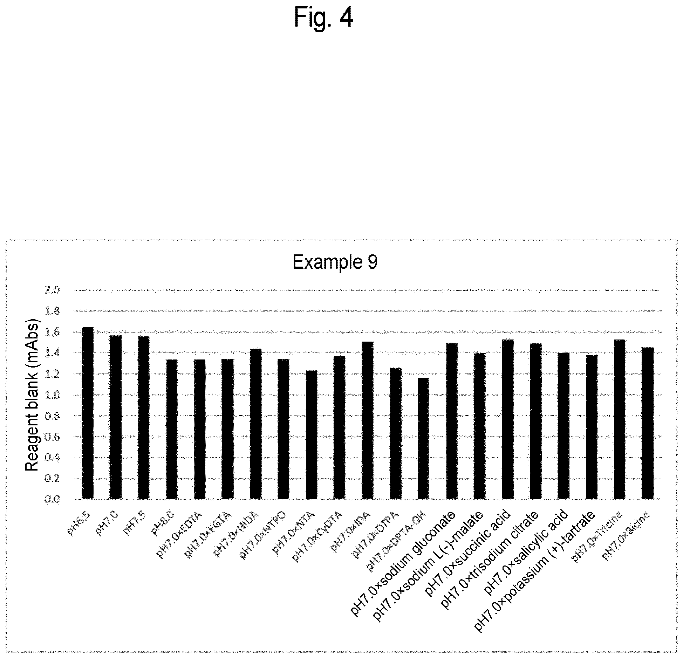

The sensitivity of the partial composition preserved for 28 days was defined as ΔAbs28, and the sensitivity of the partial composition preserved for 1 year was defined as ΔAbs365. The long-term sensitivity preservation rate was calculated as the ratio of ΔAbs365 to ΔAbs28 according to the following expression (32). The long-term sensitivity preservation rate is shown in Table 13 and Figure 4, together with the results about the reagent blank of the partial composition preserved for 1 year.

Long-term sensitivity preservation rate (%) = ΔAbs365 / ΔAbs28 × 100 (32)  (32)

[Table 13-1]

| Reagent type | Ferrocyanide-containing liquid partial composition | | Reagent blank after 1-year preservation (mAbs) | Sample | Long-term sensitivity preservation rate (%) |
| | pH | Chelating agent type | | | |
|---|---|---|---|---|---|
| Three-reagent system | 6.5 | None | 1.6 | Control serum L | 97.9 |
| | | | | Control serum H | 98.1 |

(continued)

| Reagent type | Ferrocyanide-containing liquid partial composition | | Reagent blank after 1-year preservation (mAbs) | Sample | Long-term sensitivity preservation rate (%) |
|---|---|---|---|---|---|
| | pH | Chelating agent type | | | |
| Three-re-agent system | 7.0 | None | 1.6 | Control serum L | 98.9 |
| | | | | Control serum H | 99.2 |
| Three-reagent system | 7.5 | None | 1.6 | Control serum L | 101.6 |
| | | | | Control serum H | 100.5 |
| Three-reagent system | 8.0 | None | 1.3 | Control serum L | 104.5 |
| | | | | Control serum H | 101.6 |
| Three-reagent system | 7.0 | EDTA | 1.3 | Control serum L | 103.5 |
| | | | | Control serum H | 101.4 |
| Three-reagent system | 7.0 | EGTA | 1.3 | Control serum L | 102.1 |
| | | | | Control serum H | 100.5 |
| Three-reagent system | 7.0 | HIDA | 1.4 | Control serum L | 102.1 |
| | | | | Control serum H | 100.4 |
| Three-reagent system | 7.0 | NTPO | 1.3 | Control serum L | 103.8 |
| | | | | Control serum H | 100.9 |
| Three-reagent system | 7.0 | NTA | 1.2 | Control serum L | 102.9 |
| | | | | Control serum H | 100.2 |
| Three-reagent system | 7.0 | CyDTA | 1.4 | Control serum L | 104.0 |
| | | | | Control serum H | 100.9 |
| Three-reagent system | 7.0 | IDA | 1.5 | Control serum L | 99.3 |
| | | | | Control serum H | 99.6 |

[Table 13-2]

| | | | | | |
|---|---|---|---|---|---|
| Three-reagent system | 7.0 | DTPA | 1.3 | Control serum L | 102.5 |
| | | | | Control serum H | 101.1 |
| Three-reagent system | 7.0 | DPTA-OH | 1.2 | Control serum L | 102.5 |
| | | | | Control serum H | 101.0 |
| Three-reagent system | 7.0 | Sodium gluconate | 1.5 | Control serum L | 102.3 |
| | | | | Control serum H | 101.2 |
| Three-reagent system | 7.0 | Sodium L(-)-malate | 1.4 | Control serum L | 101.7 |
| | | | | Control serum H | 100.8 |
| Three-reagent system | 7.0 | Succinic acid | 1.5 | Control serum L | 99.7 |
| | | | | Control serum H | 99.2 |

(continued)

| Three-reagent system | 7.0 | Trisodium citrate | 1.5 | Control serum L | 102.6 |
|---|---|---|---|---|---|
| | | | | Control serum H | 101.2 |
| Three-reagent system | 7.0 | Salicylic acid | 1.4 | Control serum L | 103.0 |
| | | | | Control serum H | 100.9 |
| Three-reagent system | 7.0 | Potassium (+)-tartrate | 1.4 | Control serum L | 103.1 |
| | | | | Control serum H | 101.3 |
| Three-reagent system | 7.0 | Tricine | 1.5 | Control serum L | 102.0 |
| | | | | Control serum H | 101.0 |
| Three-reagent system | 7.0 | Bicine | 1.5 | Control serum L | 102.7 |
| | | | | Control serum H | 100.8 |

As is evident from Table 13 and Figure 4, varying values of pH of the ferrocyanide-containing liquid partial composition caused small change in reagent blank. Also, varying types of the chelating agent added to the ferrocyanide-containing liquid partial composition caused small change in reagent blank.

[Example 10: Verification of long-term stability of effect of pH]

(Preparation of Trinder reagent-containing liquid partial composition)

The following reagent components were dissolved in purified water so as to achieve the described concentrations. The solution was adjusted to pH 7.6 with sodium hydroxide to prepare a Trinder reagent-containing liquid partial composition.

100 mmol/L N-tris(hydroxymethyl)methyl-2-aminoethanesulfonic acid (manufactured by Dojindo Laboratories Co., Ltd.)
0.05 wt/vol% sodium azide (manufactured by Merck KGaA)
1 mmol/L sodium ethylenediaminetetraacetate (manufactured by Kokusan Chemical Co., Ltd.)
6 wt/vol% D(-)-sorbitol (manufactured by FUJIFILM Wako Pure Chemical Corp.)
2 mmol/L N,N-bis(4-sulfobutyl)-3-methylaniline, disodium salt (manufactured by Dojindo Laboratories Co., Ltd.)
10 U/mL peroxidase (manufactured by Amano Enzyme Inc.)
30 U/mL ketoamine oxidase (manufactured by Asahi Kasei Pharma Corp.)

(Three-reagent system: Preparation of 4-aminoantipyrine-containing liquid partial composition (1))

The following reagent components were dissolved in purified water so as to achieve the described concentrations. The solution was adjusted to pH 7.5 with sodium hydroxide to prepare a 4-aminoantipyrine-containing liquid partial composition (1) containing a stabilizer, the partial composition containing trisodium citrate or containing no trisodium citrate.

250 mmol/L 3,3-dimethylglutaric acid (manufactured by Tokyo Chemical Industry Co., Ltd.)
0.05 wt/vol% ProClin 300 (manufactured by Sigma-Aldrich Co., LLC)
6.25 mmol/L 4-aminoantipyrine (manufactured by ACTEC Laboratories Inc.)
625 U/mL catalase (manufactured by Nagase ChemteX Corp.)
50 kU/mL protease for glycated albumin (manufactured by Asahi Kasei Pharma Corp.; obtained in accordance with the method described in Japanese Patent No. 4565807)
1.25 mmol/L trisodium citrate (manufactured by FUJIFILM Wako Pure Chemical Corp.)
Stabilizer having a concentration described later

(Three-reagent system: Stabilizer contained in 4-aminoantipyrine-containing liquid partial composition (1))

EP 3 995 586 B1

37.5 wt/vol% propylene glycol (manufactured by FUJIFILM Wako Pure Chemical Corp.)
62.5 wt/vol% trimethylene glycol (manufactured by FUJIFILM Wako Pure Chemical Corp.)
62.5 wt/vol% ethylene glycol (manufactured by FUJIFILM Wako Pure Chemical Corp.)
0.75 wt/vol% 2-carboxyphenylboronic acid (manufactured by Tokyo Chemical Industry Co., Ltd.)

(Three-reagent system: Preparation of ferrocyanide-containing liquid partial composition (1))

The following reagent components were dissolved in purified water so as to achieve the described concentrations. The solution was adjusted to pH 7.0 or pH 8.0 to prepare a ferrocyanide-containing liquid partial composition. The ferrocyanide-containing liquid partial composition of pH 8.0 was prepared as a partial composition containing trisodium citrate and a partial composition containing no trisodium citrate.

100 mmol/L 3,3-dimethylglutaric acid (manufactured by Tokyo Chemical Industry Co., Ltd.)
0.05 wt/vol% ProClin 300 (manufactured by Sigma-Aldrich Co., LLC)
0.5 mmol/L potassium ferrocyanide (manufactured by Kokusan Chemical Co., Ltd.)
5 mmol/L trisodium citrate (manufactured by FUJIFILM Wako Pure Chemical Corp.)

(Three-reagent system: Preparation of 4-aminoantipyrine-containing liquid partial composition (2))

The following reagent components were dissolved in purified water so as to achieve the described concentrations. The solution was adjusted to pH 7.0 or pH 8.0 with sodium hydroxide to prepare a 4-aminoantipyrine-containing liquid partial composition. The 4-aminoantipyrine-containing liquid partial composition adjusted to pH 8.0 was prepared as a partial composition containing trisodium citrate and a partial composition containing no trisodium citrate.

100 mmol/L 3,3-dimethylglutaric acid (manufactured by Tokyo Chemical Industry Co., Ltd.)
0.05 wt/vol% ProClin 300 (manufactured by Sigma-Aldrich Co., LLC)
25 mmol/L 4-aminoantipyrine (manufactured by ACTEC Laboratories Inc.)
25 mmol/L trisodium citrate (manufactured by FUJIFILM Wako Pure Chemical Corp.) (added only to the partial composition adjusted to pH 8.0)

(Three-reagent system: Preparation of ferrocyanide-containing liquid partial composition (2))

The following reagent components were dissolved in purified water so as to achieve the described concentrations. The solution was adjusted to pH 7.5 with sodium hydroxide to prepare a ferrocyanide-containing liquid partial composition containing propylene glycol as a stabilizer, the partial composition containing trisodium citrate or containing no trisodium citrate.

250 mmol/L 3,3-dimethylglutaric acid (manufactured by Tokyo Chemical Industry Co., Ltd.)
0.05 wt/vol% ProClin 300 (manufactured by Sigma-Aldrich Co., LLC)
0.125 mmol/L potassium ferrocyanide (manufactured by Kokusan Chemical Co., Ltd.)
625 U/mL catalase (manufactured by Nagase ChemteX Corp.)
50 kU/mL protease for glycated albumin (manufactured by Asahi Kasei Pharma Corp.; obtained in accordance with the method described in Japanese Patent No. 4565807)
1.25 mmol/L trisodium citrate (manufactured by FUJIFILM Wako Pure Chemical Corp.)
37.5 wt/vol% propylene glycol (manufactured by FUJIFILM Wako Pure Chemical Corp.)

(Sample)

The following samples were provided.

Control serum L (manufactured by Asahi Kasei Pharma Corp.)
Control serum H (manufactured by Asahi Kasei Pharma Corp.)

(Reagent preservation)

The prepared Trinder reagent-containing liquid partial composition, 4-aminoantipyrine-containing liquid partial composition, and ferrocyanide-containing liquid partial compositions were preserved at 5°C for 28 days or 1 year.

(Assay preparation)

The 4-aminoantipyrine-containing liquid partial composition (1) and the ferrocyanide-containing liquid partial composition (1) of the three-reagent system were mixed at a volume ratio of 4:1 to prepare a 4-aminoantipyrine- and ferrocyanide-containing liquid partial composition (1) for use in assay.

The 4-aminoantipyrine-containing liquid partial composition (2) and the ferrocyanide-containing liquid partial composition (2) of the three-reagent system were mixed at a volume ratio of 1:4 to prepare a 4-aminoantipyrine- and ferrocyanide-containing liquid partial composition (2) for use in assay.

(Assay procedure)

To 180 μL of the Trinder reagent-containing liquid partial composition, 4.5 μL of the sample was added, and the mixture was reacted at 37°C for 5 minutes. Then, 45 μL of the 4-aminoantipyrine- and ferrocyanide-containing liquid partial composition was added thereto, and the mixture was further reacted at 37°C for 5 minutes. Change in absorbance was measured from absorbance Abs1 5 minutes after the sample was added to the Trinder reagent-containing liquid partial composition to absorbance Abs2 5 minutes after the 4-aminoantipyrine- and ferrocyanide-containing liquid partial composition was added to the mixed solution of the Trinder reagent-containing liquid partial composition and the sample. The change in absorbance, $A_C$, was given according to the following expression (33).

$$A_C = (Abs2) - (Abs1) \times 180 / 225 \quad (33)$$

wherein 180 represents the liquid quantity of the Trinder reagent-containing liquid partial composition, and 225 represents the total liquid quantity of the Trinder reagent-containing liquid partial composition and the 4-aminoantipyrine- and ferrocyanide-containing liquid partial composition.

Change in absorbance measured using the sample was defined as Acs, and change in absorbance measured using purified water instead of the sample was defined as a reagent blank Acb. A value obtained by subtracting the change in absorbance, Acb, of the reagent blank from the change in absorbance, Acs, measured using the sample was calculated as sensitivity ΔAbs according to the following expression (34).

$$\Delta Abs = Acs - Acb \quad (34)$$

The sensitivity of the partial composition preserved for 28 days was defined as ΔAbs28, and the sensitivity of the partial composition preserved for 1 year was defined as ΔAbs365. The long-term sensitivity preservation rate was calculated as the ratio of ΔAbs365 to ΔAbs28 according to the following expression (35). The long-term sensitivity preservation rate is shown in Table 14, together with the results about the reagent blank of the partial composition preserved for 1 year.

Long-term sensitivity preservation rate (%) = ΔAbs365 / ΔAbs28 × 100 (35)                    (35)

[Table 14-1]

| Reagent type | 4-Aminoantipyrine-containing liquid partial composition | Ferrocyanide-containing liquid partial composition | Reagent blank after 1-year preservation (mAbs) | Sample | Long-term sensitivity preservation rate (%) |
|---|---|---|---|---|---|
| Three-reagent system | Partial composition (1)  37.5 wt/vol% propylene glycol | Partial composition (1) pH 7.0 | 1.5 | Control serum L | 102.0 |
| | | | | Control serum H | 99.3 |
| Three-reagent system | | Partial composition (1) pH 8.0 | 1.8 | Control serum L | 97.2 |
| | | | | Control serum H | 96.8 |
| Three-reagent system | | Partial composition (1) pH 8.0  5 mmol/L trisodium citrate | 1.8 | Control serum L | 97.0 |
| | | | | Control serum H | 96.5 |
| Three-reagent system | Partial composition (1)  37.5 wt/vol% propylene glycol  1.25 mmol/L trisodium citrate | Partial composition (1) pH 7.0 | 1.5 | Control serum L | 101.9 |
| | | | | Control serum H | 100.2 |
| Three-reagent system | | Partial composition (1) pH 8.0 | 1.6 | Control serum L | 105.1 |
| | | | | Control serum H | 101.8 |
| Three-reagent system | | Partial composition (1) pH 8.0  5 mmol/L trisodium citrate | 1.4 | Control serum L | 105.6 |
| | | | | Control serum H | 102.4 |

[Table 14-2]

| | | | | | |
|---|---|---|---|---|---|
| Three-reagent system | Partial composition (2) pH 7.0 | Partial composition (2) 37.5 wt/vol% propylene glycol | 4.2 | Control serum L | 89.6 |
| | | | | Control serum H | 91.4 |
| Three-reagent system | Partial composition (2) pH 8.0 | | 1.5 | Control serum L | 93.8 |
| | | | | Control serum H | 94.8 |
| Three-reagent system | Partial composition (2) pH 8.0  25 mmol/L trisodium citrate | | 1.4 | Control serum L | 97.4 |
| | | | | Control serum H | 97.2 |

(continued)

| Three-reagent system | Partial composition (2) pH 7.0 | Partial composition (2) 37.5 wt/vol% propylene glycol 1.25 mmol/L trisodium citrate | 4.1 | Control serum L | 89.6 |
|---|---|---|---|---|---|
| | | | | Control serum | 91.3 |
| Three-reagent system | Partial composition (2) pH 8.0 | | 1.4 | Control serum L | 94.0 |
| | | | | Control serum H | 95.1 |
| Three-reagent system | Partial composition (2) pH 8.0 25 mmol/L trisodium citrate | | 1.4 | Control serum L | 98.1 |
| | | | | Control serum H | 97.6 |

[Table 14-3]

| Three-reagent system | Partial composition (1) 62.5 wt/vol% trimethylene glycol | Partial composition (1) pH 7.0 | 4.0 | Control serum L | 99.3 |
|---|---|---|---|---|---|
| | | | | Control serum H | 98.6 |
| Three-reagent system | | Partial composition (1) pH 8.0 | 3.6 | Control serum L | 102.1 |
| | | | | Control serum H | 100.0 |
| Three-reagent system | | Partial composition (1) pH 8.0 5 mmol/L trisodium citrate | 3.6 | Control serum L | 102.8 |
| | | | | Control serum H | 100.5 |
| Three-reagent system | Partial composition (1) 62.5 wt/vol% trimethylene glycol 1.25 mmol/L trisodium citrate | Partial composition (1) pH 7.0 | 3.2 | Control serum L | 102.3 |
| | | | | Control serum H | 101.0 |
| Three-reagent system | | Partial composition (1) pH 8.0 | 2.4 | Control serum L | 106.1 |
| | | | | Control serum H | 103.0 |
| Three-reagent system | | Partial composition (1) pH 8.0 5 mmol/L trisodium citrate | 2.6 | Control serum L | 106.2 |
| | | | | Control serum H | 103.5 |

[Table 14-4]

| Three-reagent system | Partial composition (1) 62.5 wt/vol% ethylene glycol | Partial composition (1) pH 7.0 | 2.7 | Control serum L | 96.8 |
|---|---|---|---|---|---|
| | | | | Control serum H | 97.2 |
| Three-reagent system | | Partial composition (1) pH 8.0 | 2.4 | Control serum L | 98.6 |
| | | | | Control serum H | 98.4 |
| Three-reagent system | | Partial composition (1) pH 8.0 5 mmol/L trisodium citrate | 2.2 | Control serum L | 98.7 |
| | | | | Control serum H | 98.2 |

(continued)

| Three-re-agent sys-tem | Partial composition (1) 62.5 wt/vol% ethylene glycol 1.25 mmol/L trisodium citrate | Partial composition (1) pH 7.0 | 2.2 | Control serum L | 104.6 |
| --- | --- | --- | --- | --- | --- |
| | | | | Control serum H | 101.7 |
| Three-re-agent sys-tem | | Partial composition (1) pH 8.0 | 2.0 | Control serum L | 107.3 |
| | | | | Control serum H | 104.2 |
| Three-re-agent sys-tem | | Partial composition (1) pH 8.0 5 mmol/L trisodium citrate | 1.9 | Control serum L | 107.4 |
| | | | | Control serum H | 103.7 |

[Table 14-5]

| Three-reagent system | Partial composition (1) 0.75 wt/vol% 2-carboxyphenylboronic acid | Partial composition (1) pH 7.0 | 2.4 | Control serum L | 88.5 |
| --- | --- | --- | --- | --- | --- |
| | | | | Control serum H | 91.2 |
| Three-reagent system | | Partial composition (1) pH 8.0 | 2.2 | Control serum L | 90.1 |
| | | | | Control serum H | 92.6 |
| Three-reagent system | | Partial composition (1) pH 8.0 5 mmol/L trisodium citrate | 2.4 | Control serum L | 89.1 |
| | | | | Control serum H | 92.6 |
| Three-reagent system | Partial composition (1) 0.75 wt/vol% 2-carboxyphenylboronic acid 1.25 mmol/L trisodium citrate | Partial composition (1) pH 7.0 | 2.6 | Control serum L | 90.5 |
| | | | | Control serum H | 92.8 |
| Three-reagent system | | Partial composition (1) pH 8.0 | 2.3 | Control serum L | 94.5 |
| | | | | Control serum H | 95.1 |
| Three-reagent system | | Partial composition (1) pH 8.0 5 mmol/L trisodium citrate | 2.2 | Control serum L | 93.6 |
| | | | | Control serum H | 94.8 |

As is evident from Table 14, use of a stabilizer that gave an oxidation-reduction potential of ferrocyanide higher than 0.058 V upon mixing the stabilizer with the ferrocyanide caused small change in reagent blank even if pH was larger than 7. Use of the stabilizer that gave an oxidation-reduction potential of ferrocyanide higher than 0.058 V upon mixing the stabilizer with the ferrocyanide caused small change in reagent blank even when the chelating agent was added.

## Claims

1. A glycated protein assay reagent containing at least a Trinder reagent, 4-aminoantipyrine, protease, a stabilizer of the protease, and ferrocyanide, wherein

   at least the Trinder reagent is contained in a Trinder reagent-containing partial composition,
   at least the 4-aminoantipyrine is contained in a 4-aminoantipyrine-containing partial composition, and
   the stabilizer of the protease is selected from the group consisting of propylene glycol, trimethylene glycol, ethylene glycol and carboxyphenylboronic acid.

2. The glycated protein assay reagent according to claim 1, wherein one of the Trinder reagent-containing partial composition and the 4-aminoantipyrine-containing partial composition further contains the protease and the stabilizer of the protease.

3. The glycated protein assay reagent according to claim 2, wherein the Trinder reagent-containing partial composition further contains the protease and the stabilizer of the protease.

4. The glycated protein assay reagent according to any one of claims 1 to 3, wherein the 4-aminoantipyrine-containing partial composition further contains fructosyl amino acid oxidase.

5. The glycated protein assay reagent according to claim 2, wherein the 4-aminoantipyrine-containing partial composition further contains the protease and the stabilizer of the protease.

6. The glycated protein assay reagent according to any one of claims 1 to 3 and 5, wherein the Trinder reagent-containing partial composition further contains fructosyl amino acid oxidase.

7. The glycated protein assay reagent according to any one of claims 1 to 6, wherein at least one of the Trinder reagent-containing partial composition and the 4-aminoantipyrine-containing partial composition contains the ferrocyanide.

8. The glycated protein assay reagent according to claim 1, wherein one of the Trinder reagent-containing partial composition and the 4-aminoantipyrine-containing partial composition further contains the protease, the stabilizer of the protease and the ferrocyanide;
especially wherein the 4-aminoantipyrine-containing partial composition further contains the protease, the stabilizer of the protease, and the ferrocyanide.

9. The glycated protein assay reagent according to any one of claims 1 to 8, wherein the concentration of the stabilizer of the protease is a concentration that increases the oxidation-reduction potential of the ferrocyanide above 0.058 V when the stabilizer of the protease and the ferrocyanide are mixed, and
the oxidation-reduction potential is an oxidation-reduction potential in a reaction system containing the stabilizer of the protease and the ferrocyanide and not containing glycated protein.

10. The glycated protein assay reagent according to claim 9, wherein the oxidation-reduction potential is higher than 0.058 V and less than or equal to 0.400 V.

11. The glycated protein assay reagent according to any one of claims 1 to 9, wherein the 4-aminoantipyrine-containing partial composition or the Trinder reagent-containing partial composition contains the stabilizer of the protease, the stabilizer of the protease is propylene glycol, and the concentration of the propylene glycol in the 4-aminoantipyrine-containing partial composition or the Trinder reagent-containing partial composition is 7.5 wt/vol% or higher and 80 wt/vol% or lower; or

the stabilizer of the protease is trimethylene glycol, and the concentration of the trimethylene glycol in the 4-aminoantipyrine-containing partial composition or the Trinder reagent-containing partial composition is 40 wt/vol% or higher and 80 wt/vol% or lower; or
the stabilizer of the protease is ethylene glycol, and the concentration of the ethylene glycol in the 4-aminoantipyrine-containing partial composition or the Trinder reagent-containing partial composition is 40 wt/vol% or higher and 80 wt/vol% or lower; or
the stabilizer of the protease is 2-carboxyphenylboronic acid, and the concentration of the 2-carboxyphenylboronic acid in the 4-aminoantipyrine-containing partial composition or the Trinder reagent-containing partial composition is 0.2 wt/vol% or higher and 10 wt/vol% or lower.

12. The glycated protein assay reagent according to any one of claims 1 to 11, wherein the glycated protein is glycated albumin; and/or

wherein the glycated protein assay reagent is preserved at 2°C or higher and 10°C or lower; and/or
wherein the stabilizer of the protease excludes dimethyl sulfoxide; and/or
wherein one of the Trinder reagent-containing partial composition and the 4-aminoantipyrine-containing partial composition further contains peroxidase.

13. A method for assaying a glycated protein, containing:

contacting a Trinder reagent, 4-aminoantipyrine, protease, a stabilizer of the protease, and ferrocyanide with a specimen; and
detecting color development depending on the glycated protein present in the specimen, wherein
at least before the contact, the Trinder reagent is contained in a Trinder reagent-containing partial composition,
at least before the contact, the 4-aminoantipyrine is contained in a 4-aminoantipyrine-containing partial composition,
one of the Trinder reagent-containing partial composition and the 4-aminoantipyrine-containing partial composition contains the protease and the stabilizer of the protease,

one of the Trinder reagent-containing partial composition and the 4-aminoantipyrine-containing partial composition contains the ferrocyanide, and
the stabilizer of the protease is selected from the group consisting of propylene glycol, trimethylene glycol, ethylene glycol and carboxyphenylboronic acid.

14. A method for preserving a glycated protein assay reagent, containing:

preparing a Trinder reagent-containing partial composition containing at least a Trinder reagent;
preserving the Trinder reagent-containing partial composition;
preparing a 4-aminoantipyrine-containing partial composition containing at least 4-aminoantipyrine; and
preserving the 4-aminoantipyrine-containing partial composition, wherein
one of the Trinder reagent-containing partial composition and the 4-aminoantipyrine-containing partial composition further contains protease and a stabilizer of the protease,
one of the Trinder reagent-containing partial composition and the 4-aminoantipyrine-containing partial composition further contains ferrocyanide, and
the stabilizer of the protease is selected from the group consisting of propylene glycol, trimethylene glycol, ethylene glycol and carboxyphenylboronic acid.

15. A method for stabilizing a glycated protein assay reagent, containing:

preparing a Trinder reagent-containing partial composition containing at least a Trinder reagent; and
preparing a 4-aminoantipyrine-containing partial composition containing at least 4-aminoantipyrine, wherein
one of the Trinder reagent-containing partial composition and the 4-aminoantipyrine-containing partial composition further comprises protease and a stabilizer of the protease,
one of the Trinder reagent-containing partial composition and the 4-aminoantipyrine-containing partial composition further comprises ferrocyanide, and
the stabilizer of the protease is selected from the group consisting of propylene glycol, trimethylene glycol, ethylene glycol and carboxyphenylboronic acid.

## Patentansprüche

1. Glykosyliertes Protein-Assay-Reagenz, das mindestens ein Trinder-Reagenz, 4-Aminoantipyrin, Protease, einen Stabilisator der Protease und Ferrocyanid enthält, wobei

mindestens das Trinder-Reagenz in einer Trinder-Reagenz-haltigen Teilzusammensetzung enthalten ist,
mindestens das 4-Aminoantipyrin in einer 4-Aminoantipyrin-haltigen Teilzusammensetzung enthalten ist und
der Stabilisator der Protease aus der Gruppe ausgewählt ist, die aus Propylenglykol, Trimethylenglykol, Ethylenglykol und Carboxyphenylboronsäure besteht.

2. Glykosyliertes Protein-Assay-Reagenz gemäß Anspruch 1, wobei eine der Trinder-Reagenz-haltigen Teilzusammensetzung und der 4-Aminoantipyrin-haltigen Teilzusammensetzung ferner die Protease und den Stabilisator der Protease enthält.

3. Glykosyliertes Protein-Assay-Reagenz gemäß Anspruch 2, wobei die Trinder-Reagenz-haltige Teilzusammensetzung ferner die Protease und den Stabilisator der Protease enthält.

4. Glykosyliertes Protein-Assay-Reagenz gemäß einem der Ansprüche 1 bis 3, wobei die 4-Aminoantipyrin-haltige Teilzusammensetzung ferner Fructosylaminosäureoxidase enthält.

5. Glykosyliertes Protein-Assay-Reagenz gemäß Anspruch 2, wobei die 4-Aminoantipyrin-haltige Teilzusammensetzung ferner die Protease und den Stabilisator der Protease enthält.

6. Glykosyliertes Protein-Assay-Reagenz gemäß einem der Ansprüche 1 bis 3 und 5, wobei die Trinder-Reagenz-haltige Teilzusammensetzung ferner Fructosylaminosäureoxidase enthält.

7. Glykosyliertes Protein-Assay-Reagenz gemäß einem der Ansprüche 1 bis 6, wobei mindestens eine der Trinder-Reagenz-haltigen Teilzusammensetzung und der 4-Aminoantipyrin-haltigen Teilzusammensetzung das Ferrocyanid enthält.

8. Glykosyliertes Protein-Assay-Reagenz gemäß Anspruch 1, wobei eine der Trinder-Reagenz-haltigen Teilzusammensetzung und der 4-Aminoantipyrin-haltigen Teilzusammensetzung ferner die Protease, den Stabilisator der Protease und das Ferrocyanid enthält; insbesondere wobei die 4-Aminoantipyrin haltige Teilzusammensetzung ferner die Protease, den Stabilisator der Protease und das Ferrocyanid enthält.

9. Glykosyliertes Protein-Assay-Reagenz gemäß einem der Ansprüche 1 bis 8, wobei die Konzentration des Protease-Stabilisators eine Konzentration ist, die das Oxidations-Reduktions-Potential des Ferrocyanids über 0,058 V erhöht, wenn der Stabilisator der Protease und das Ferrocyanid gemischt werden, und das Oxidations-Reduktions-Potential ein Oxidations-Reduktions-Potential in einem Reaktionssystem ist, das den Stabilisator der Protease und das Ferrocyanid enthält und kein glykosyliertes Protein enthält.

10. Glykosyliertes Protein-Assay-Reagenz Reagenz gemäß Anspruch 9, wobei das Oxidations-Reduktions-Potential höher als 0,058 V und kleiner oder gleich 0,400 V ist.

11. Glykosyliertes Protein-Assay-Reagenz gemäß einem der Ansprüche 1 bis 9, wobei die 4-Aminoantipyrin-haltige Teilzusammensetzung oder die Trinder-Reagenz-haltige Teilzusammensetzung den Stabilisator der Protease enthält, der Stabilisator der Protease Propylenglykol ist und die Konzentration des Propylenglykols in der 4-Aminoantipyrin-haltigen Teilzusammensetzung oder der Trinder-Reagenz-haltigen Teilzusammensetzung 7,5 Gew./Vol.-% oder höher und 80 Gew./Vol.-% oder niedriger ist; oder

der Stabilisator der Protease Trimethylenglykol ist und die Konzentration des Trimethylenglykols in der 4-Aminoantipyrin-haltigen Teilzusammensetzung oder der Trinder-Reagenz-haltigen Teilzusammensetzung 40 Gew./Vol.-% oder höher und 80 Gew./Vol.-% oder niedriger ist; oder der Stabilisator der Protease Ethylenglykol ist und die Konzentration des Ethylenglykols in der 4-Aminoantipyrin-haltigen Teilzusammensetzung oder der Trinder-Reagenz-haltigen Teilzusammensetzung 40 Gew./Vol.-% oder höher und 80 Gew./Vol.-% oder niedriger ist; oder der Stabilisator der Protease 2-Carboxyphenylboronsäure ist und die Konzentration der 2-Carboxyphenylboronsäure in der 4-Aminoantipyrin-haltigen Teilzusammensetzung oder der Trinder-Reagenz-haltigen Teilzusammensetzung 0,2 Gew./Vol.-% oder höher und 10 Gew./Vol.-% oder niedriger ist.

12. Glykosyliertes Protein-Assay-Reagenz gemäß einem der Ansprüche 1 bis 11, wobei das glykosylierte Protein glykosyliertes Albumin ist; und/oder

wobei das glykosylierte Protein-Assay-Reagenz bei 2° C oder höher und bei 10° C oder niedriger aufbewahrt wird; und/oder wobei der Stabilisator der Protease Dimethylsulfoxid ausschließt; und/oder wobei eine der Trinder-Reagenz-haltigen Teilzusammensetzung und der 4-Aminoantipyrin-haltigen Teilzusammensetzung ferner Peroxidase enthält.

13. Verfahren zum Nachweis eines glykosylierten Proteins, umfassend:

Inkontaktbringen von einem Trinder-Reagenz, 4-Aminoantipyrin, Protease, einem Stabilisator der Protease und Ferrocyanid mit einer Probe; und Nachweisen der Farbentwicklung in Abhängigkeit von dem in der Probe vorhandenen glykosylierten Protein, wobei mindestens vor dem Kontakt das Trinder-Reagenz in einer Trinder-Reagenz-haltigen Teilzusammensetzung enthalten ist, mindestens vor dem Kontakt das 4-Aminoantipyrin in einer 4-Aminoantipyrin haltigen Teilzusammensetzung enthalten ist, eine der Trinder-Reagenz-haltigen Teilzusammensetzung und der 4-Aminoantipyrin-haltigen Teilzusammensetzung die Protease und den Stabilisator der Protease enthält, eine der Trinder-Reagenz-haltigen Teilzusammensetzung und der 4-Aminoantipyrin-haltigen Teilzusammensetzung das Ferrocyanid enthält und der Stabilisator der Protease aus der Gruppe ausgewählt ist, die aus Propylenglykol, Trimethylenglykol, Ethylenglykol und Carboxyphenylboronsäure besteht.

**14.** Verfahren zur Konservierung eines glykosylierten Protein-Assay-Reagenzes, umfassend:

Herstellen einer Trinder-Reagenz-haltigen Teilzusammensetzung, die mindestens ein Trinder-Reagenz enthält;
Konservieren der Trinder-Reagenz-haltigen Teilzusammensetzung;
Herstellen einer 4-Aminoantipyrin-haltigen Teilzusammensetzung, die mindestens 4-Aminoantipyrin enthält; und
Konservieren der 4-Aminoantipyrin-haltigen Teilzusammensetzung, wobei
eine der Trinder-Reagenz-haltigen Teilzusammensetzung und der 4-Aminoantipyrin-haltigen Teilzusammensetzung ferner Protease und einen Stabilisator der Protease enthält,
eine der Trinder-Reagenz-haltigen Teilzusammensetzungen und der 4-Aminoantipyrin-haltigen Teilzusammensetzung ferner Ferrocyanid enthält, und
der Stabilisator der Protease aus der Gruppe ausgewählt ist, die aus Propylenglykol, Trimethylenglykol, Ethylenglykol und Carboxyphenylboronsäure besteht.

**15.** Verfahren zur Stabilisierung eines glykosylierten Protein-Assay-Reagenzes, umfassend:

Herstellen einer Trinder-Reagenz-haltigen Teilzusammensetzung, die mindestens ein Trinder-Reagenz enthält; und
Herstellen einer 4-Aminoantipyrin-haltigen Teilzusammensetzung, die mindestens 4-Aminoantipyrin enthält, wobei
eine der Trinder-Reagenz-haltigen Teilzusammensetzung und der 4-Aminoantipyrin-haltigen Teilzusammensetzung ferner Protease und einen Stabilisator der Protease enthält,
eine der Trinder-Reagenz-haltigen Teilzusammensetzung und der 4-Aminoantipyrin-haltigen Teilzusammensetzung ferner Ferrocyanid enthält, und
der Stabilisator der Protease aus der Gruppe ausgewählt ist, die aus Propylenglykol, Trimethylenglykol, Ethylenglykol und Carboxyphenylboronsäure besteht.

## Revendications

**1.** Réactif d'essai des protéines glyquées contenant au moins un réactif de Trinder, 4-aminoantipyrine, une protéase, un stabilisateur de la protéase et du ferrocyanure, dans lequel

au moins le réactif de Trinder est contenu dans une composition partielle contenant le réactif de Trinder,
au moins la 4-aminoantipyrine est contenue dans une composition partielle contenant de la 4-aminoantipyrine, et
le stabilisateur de la protéase est choisi parmi le groupe constitué par le propylène glycol, le triméthylène glycol, l'éthylène glycol et l'acide carboxyphénylboronique.

**2.** Réactif d'essai des protéines glyquées selon la revendication 1, dans lequel l'une de la composition partielle contenant le réactif de Trinder et de la composition partielle contenant la 4-aminoantipyrine contient en outre la protéase et le stabilisateur de la protéase.

**3.** Réactif d'essai des protéines glyquées selon la revendication 2, dans lequel la composition partielle contenant le réactif de Trinder contient en outre la protéase et le stabilisateur de la protéase.

**4.** Réactif d'essai des protéines glyquées selon l'une des des revendications 1 à 3, dans lequel la composition partielle contenant de la 4-aminoantipyrine contient en outre de la fructosyl amino acide oxydase.

**5.** Réactif d'essai des protéines glyquées selon la revendication 2, dans lequel la composition partielle contenant de la 4-aminoantipyrine contient en outre la protéase et le stabilisateur de la protéase.

**6.** Réactif d'essai des protéines glyquées selon l'une des revendications 1 à 3 et 5, dans lequel la composition partielle contenant le réactif de Trinder contient en outre de la fructosyl amino acide oxidase.

**7.** Réactif d'essai des protéines glyquées selon l'une des revendications 1 à 6, dans lequel au moins l'une de la composition partielle contenant le réactif de Trinder et de la composition partielle contenant de la 4-aminoantipyrine contient le ferrocyanure.

8. Réactif d'essai des protéines glyquées selon la revendication 1, dans lequel l'une de la composition partielle contenant le réactif de Trinder et de la composition partielle contenant de la 4-aminoantipyrine contient en outre la protéase, le stabilisateur de la protéase et le ferrocyanure;
en particulier dans lequel la composition partielle contenant de la 4-aminoantipyrine contient en outre la protéase, le stabilisateur de la protéase et le ferrocyanure.

9. Réactif d'essai des protéines glyquées selon l'une des revendications 1 à 8, dans lequel la concentration du stabilisateur de la protéase est une concentration qui augmente le potentiel d'oxydoréduction du ferrocyanure au-dessus de 0,058 V lorsque le stabilisateur de la protéase et le ferrocyanure sont mélangés, et
le potentiel d'oxydoréduction est un potentiel d'oxydoréduction dans un système réactionnel contenant le stabilisateur de la protéase et le ferrocyanure et ne contenant pas de protéine glyquée.

10. Réactif d'essai des protéines glyquées selon la revendication 9, dans lequel le potentiel d'oxydoréduction est supérieur à 0,058 V et inférieur ou égal à 0,400 V.

11. Réactif d'essai des protéines glyquées selon l'une des revendications 1 à 9, dans lequel la composition partielle contenant de la 4-aminoantipyrine ou la composition partielle contenant le réactif de Trinder contient le stabilisateur de la protéase, le stabilisateur de la protéase est le propylène glycol, et la concentration du propylène glycol dans la composition partielle contenant de la 4-aminoantipyrine ou la composition partielle contenant le réactif de Trinder est 7,5 % en poids/volume ou supérieur et 80 % en poids/volume ou inférieur; ou

le stabilisateur de la protéase est le triméthylène glycol, et la concentration du triméthylène glycol dans la composition partielle contenant de la 4-aminoantipyrine ou dans la composition partielle contenant le réactif de Trinder est 40 % en poids/volume ou supérieur et 80 % en poids/volume ou inférieur; ou
le stabilisateur de la protéase est l'éthylène glycol, et la concentration de l'éthylène glycol dans la composition partielle contenant de la 4-aminoantipyrine ou dans la composition partielle contenant le réactif de Trinder est 40 % en poids/volume ou supérieur et 80 % en poids/volume ou inférieur; ou
le stabilisateur de la protéase est l'acide 2-carboxyphénylboronique, et la concentration de l'acide 2-carboxyphénylboronique dans la composition partielle contenant de la 4-aminoantipyrine ou dans la composition partielle contenant le réactif de Trinder est 0,2 % en poids/volume ou supérieur et 10 % en poids/volume ou inférieur.

12. Réactif d'essai des protéines glyquées selon l'une des revendications 1 à 11, dans lequel la protéine glyquée est l'albumine glyquée; et/ou

dans lequel le réactif d'essai des protéines glyquées est conservé à une température de 2° C ou supérieur et 10 °C ou inférieur; et/ou
dans lequel le stabilisateur de la protéase exclut le diméthylsulfoxyde; et/ou
dans lequel l'une de la composition partielle contenant le réactif de Trinder et de la composition partielle contenant la 4-aminoantipyrine contient en outre de la peroxydase.

13. Procédé d'essai d'une protéine glyquée, comprenant:

la mise en contact d'un réactif de Trinder, de 4-aminoantipyrine, de protéase, d'un stabilisateur de la protéase et de ferrocyanure avec un échantillon ; et
détecter le développement de la couleur en fonction de la protéine glyquée présente dans l'échantillon, dans lequel
au moins avant la mise en contact, le réactif de Trinder est contenu dans une composition partielle contenant du réactif de Trinder,
au moins avant le contact, la 4-aminoantipyrine est contenue dans une composition partielle contenant de la 4-aminoantipyrine,
l'une de la composition partielle contenant le réactif de Trinder et de la composition partielle contenant la 4-aminoantipyrine contient la protéase et le stabilisateur de la protéase,
l'une de la composition partielle contenant le réactif de Trinder et de la composition partielle contenant la 4-aminoantipyrine contient le ferrocyanure, et
le stabilisateur de la protéase est choisi parmi le groupe constitué du propylène glycol, du triméthylène glycol, de l'éthylène glycol et de l'acide carboxyphénylboronique.

14. Procédé de conservation d'un réactif d'essai des protéines glyquées, comprenant:

la préparation d'une composition partielle contenant un réactif de Trinder contenant au moins un réactif de Trinder;

la conservation de la composition partielle contenant le réactif de Trinder;

la préparation d'une composition partielle contenant de la 4-aminoantipyrine contenant au moins de la 4-aminoantipyrine; et

la conservation de la composition partielle contenant de la 4-aminoantipyrine, dans lequel

l'une parmi la composition partielle contenant le réactif de Trinder et la composition partielle contenant la 4-aminoantipyrine contient en outre une protéase et un stabilisateur de la protéase,

l'une de la composition partielle contenant le réactif de Trinder et de la composition partielle contenant la 4-aminoantipyrine contient en outre du ferrocyanure, et

le stabilisateur de la protéase est choisi dans le groupe constitué par le propylène glycol, le triméthylène glycol, l'éthylène glycol et l'acide carboxyphénylboronique.

15. Procédé pour stabiliser un réactif d'essai de protéines glyquées, comprenant :

la préparation d'une composition partielle contenant un réactif de Trinder contenant au moins un réactif de Trinder; et

la préparation d'une composition partielle contenant de la 4-aminoantipyrine contenant au moins de la 4-aminoantipyrine, dans lequel

l'une de la composition partielle contenant le réactif de Trinder et de la composition partielle contenant la 4-aminoantipyrine comprend en outre une protéase et un stabilisateur de la protéase,

l'une de la composition partielle contenant le réactif Trinder et de la composition partielle contenant la 4-aminoantipyrine comprend en outre du ferrocyanure, et

le stabilisateur de la protéase est choisi parmi le groupe comprenant le propylène glycol, le triméthylène glycol, l'éthylène glycol et l'acide carboxyphénylboronique.

# Fig. 1

# Fig. 2

# Fig. 3

# Fig. 4

## REFERENCES CITED IN THE DESCRIPTION

### Patent documents cited in the description

- JP 6046846 A
- JP 5192193 A
- WO 9634977 A
- JP 2195900 A
- WO 199713872 A
- WO 2002061119 A
- WO 1996041859 A
- WO 1995028092 A
- WO 9813462 A

- WO 9813459 A
- JP 2004049063 A
- JP 2006010711 A
- CN 109239059 A
- WO 2001094618 A
- JP 2005261383 A
- JP 4565807 B

### Non-patent literature cited in the description

- *Clinica Chimica Acta*, 2002, vol. 324, 61-71